(19) Europäisches Patentamt
European Patent Office
Office européen des brevets

(11) EP 4 353 260 A1

(12) EUROPEAN PATENT APPLICATION
published in accordance with Art. 153(4) EPC

(43) Date of publication:
**17.04.2024 Bulletin 2024/16**

(21) Application number: **22816156.8**

(22) Date of filing: **01.06.2022**

(51) International Patent Classification (IPC):
***A61K 45/00*** *(2006.01)* ***A61K 48/00*** *(2006.01)*
***A61K 31/41*** *(2006.01)* ***A61K 31/425*** *(2006.01)*
***A61K 31/433*** *(2006.01)* ***A61K 31/437*** *(2006.01)*

(52) Cooperative Patent Classification (CPC):
**A61K 31/41; A61K 31/425; A61K 31/433; A61K 31/437; A61K 45/00; A61K 48/00**

(86) International application number:
**PCT/JP2022/022350**

(87) International publication number:
**WO 2022/255411 (08.12.2022 Gazette 2022/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **01.06.2021 US 202163195318 P**

(71) Applicant: **Kyoto University**
**Kyoto-shi, Kyoto 606-8501 (JP)**

(72) Inventors:
- **HAGIWARA, Masatoshi**
  **Kyoto 606-8501 (JP)**
- **IIDA, Kei**
  **Kyoto 606-8501 (JP)**
- **NODA, Takeshi**
  **Kyoto 606-8501 (JP)**
- **AJIRO, Masahiko**
  **Kyoto 606-8501 (JP)**

(74) Representative: **Dehns**
**St. Bride's House**
**10 Salisbury Square**
**London EC4Y 8JD (GB)**

Remarks:
The complete document including Reference Table(s) and the Sequence Listing(s) can be downloaded from the EPO website

# (54) METHOD FOR ENHANCING INNATE IMMUNE RESPONSE

(57) A method for enhancement innate immunity is provided. In one aspect, the present disclosure relates to a method for enhancing an innate immune response of a subject or a cell, the subject or the cell being a subject or a cell having an innate immune response that a gene to be subjected to splicing participates in, and this method includes administering a compound that affects alternative splicing to a subject or a cell, or bringing a compound that affects alternative splicing into contact with the subject or the cell.

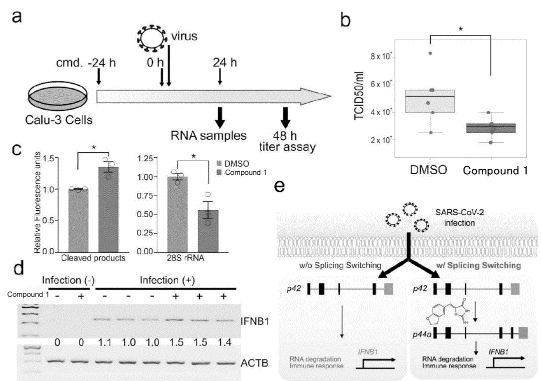


FIG. 3

EP 4 353 260 A1

## Description

### Technical Field

The present disclosure relates to a method for enhancing an innate immune response.

### Background Art

As of July 2021, more than 180 million people have been infected with SARS-CoV-2, and more than 4.0 million people have died of COVID-19 (https://covid19.who.int). Despite extensive efforts towards prophylactic vaccination against SARS-CoV-2, the incidence of COVID-19 continues to increase, with rapid emergence of mutant strains (https7/www.who.int/). In order to understand the mechanism of SARS-CoV-2 pathogenesis and develop novel therapeutic approaches, it is essential to analyze genetic factors responsible for susceptibility and severity. A previous genome-wide association study (GWAS) in over 2,000 COVID-19 patients uncovered several susceptibility-associated genes, including LZTFL1, CCHCR1, OAS1, OAS2, OAS3, DPP9, TYK2, and IFNAR2 (Non-Patent Document 1: Pairo-Castineira et al., 2021). The COVID-19 host genetics initiative also gathered genetic information for more than 30,000 COVID-19 patients, including over 5,000 patients with very severe respiratory symptoms, and showed similar results (Non-Patent Document 2: The COVID-19 Host Genetics Initiative, 2021). It revealed 131 SNPs associated with severe respiratory conditions ($p < 5 \times 10^{-8}$) in the gene cluster of OAS1, OAS2, and OAS3 (FIG. 1a). They consist of 5 SNPs within the OAS1 locus, 72 SNPs within intergenic regions across OAS1, OAS2, and OAS3, 46 SNPs within the OAS3 locus, and 8 SNPs within the OAS2 locus. Importance of the OAS family of genes was further indicated in a transcriptome study in an in vitro model of SARS-CoV-2 infection (Non-Patent Document 3: Blanco-Melo et al., 2020), in which normal human bronchial epithelial (NHBE) cells exhibited an increased expression of OAS1, OAS2, and OAS3 (2.3, 2.1, and 2.2-fold increase, respectively). Of the aforementioned SNPs, this genomic region includes SNPs associated with hospitalized COVID-19 patients (32 SNPs) and all COVID-19 patients (125 SNPs) with low p-values ($p < 5 \times 10^{-8}$) (FIGS. 1b and 1c), which are concentrated around the terminal exon of OAS1 and intron 2 of OAS3. In particular, the SNP with the lowest p-value was the G>A SNP rs10774671, located at the last base of intron 5 (Table 1), and its association with infectious viruses, including SARS coronavirus and Dengue virus, has been reported (Non-Patent Document 4: Hamano et al., 2005; Non-Patent Document 5: He et al., 2006; Non-Patent Document 6, Lin et al., 2009).

### Citation List

#### Non-Patent Document

[Non-Patent Document 1] Pairo-Castineira et al., 2021, Nature 591:92-98
[Non-Patent Document 2] The COVID-19 Host Genetics Initiative,2021
[Non-Patent Document 3] Blanco-Melo et al., 2020, Cell 181:1036-1045
[Non-Patent Document 4] Hamano et al., 2005, Biochem Biophys Res Commun 329:1234-9
[Non-Patent Document 5] He et al., 2006, BMC Infect Dis 6:106
[Non-Patent Document 6] Lin et al., 2009, J Immunol 183:8035-43


### Summary

#### Problem to be Solved by the Invention

The present disclosure provides a method for enhancing innate immunity, a composition capable of enhancing innate immunity, and a method for preventing a viral infectious disease, alleviating a symptom, or treating a viral infectious disease.

In one aspect, the present disclosure relates to a method for enhancing an innate immune response of a subject or a cell,

the subject or the cell being a subject or a cell having an innate immune response that a gene to be subjected to splicing participates in,

the method including administering a compound that affects alternative splicing to the subject or the cell, or bringing a compound that affects alternative splicing into contact with the subject or the cell.

In another aspect, the present disclosure relates to a composition for enhancing an innate immune response of a subject or a cell,

the subject or the cell being a subject or a cell having an innate immune response that a gene to be subjected to splicing participates in,
the composition containing a compound that affects alternative splicing as an active ingredient.

In another aspect, the present disclosure relates to a method for preventing a viral infectious disease, alleviating a symptom of a viral infectious disease, or treating a viral infectious disease, the method including:

(1) confirming whether at least one condition selected from the group consisting of

a condition in which a subject has an innate immune response that a gene to be subjected to splicing participates in,
a condition in which the subject has an innate immune response attenuated by alternative splicing of a gene for the innate immune response,
a condition in which the subject has a mutation or polymorphism (including an SNP) that causes alternative splicing that attenuates the innate immune response in a gene on an innate immune response pathway, and
a condition in which the subject has the A-allele of SNP rs10774671, is satisfied; and

(2) administering a compound that affects alternative splicing to the subject who satisfies the condition in the (1) above, or bringing a compound that affects alternative splicing into contact with the subject.

The present disclosure provides a method for enhancing innate immunity, a composition capable of enhancing innate immunity, and a method for preventing a viral infectious disease, alleviating a symptom, or treating a viral infectious disease.

Description of Embodiments

The rapidly accumulating disease susceptibility information collected from coronavirus disease (COVID-19) patient genomes must be urgently utilized to develop therapeutic interventions for SARS-CoV-2 infectious diseases. Chromosome 12q24.13 that encodes the 2'-5'-oligoadenylate synthase (OAS) family that senses viral genomic RNAs and triggers an antiviral response has been identified as one of the genomic regions containing SNPs associated with COVID-19 severity. Ahigh-risk SNPs identified as the splice acceptor site of OAS1 exon 6 is known to alter proportions of various splicing isoforms and enzyme activities.
The inventors of the present invention employed in silico motif searches and RNA pull-down assays in order to define factors responsible for the OAS1 splicing. Then, the inventors of the present invention rationally selected candidates for splicing modulators for modulating this splicing.
As a result, the inventors of the present invention found that inhibition of CDC-like kinase using a small chemical compound induces switching of OAS1 splice isoforms in human lung cells. Furthermore, in this condition, an increase in resistance to SARS-CoV-2 infection, enhanced RNA degradation, and activation of the transcription of interferon $\beta 1$ were observed.
The inventors of the present invention found that these results indicate the possibility of use of a chemical splicing modifier to boost an innate immune response against SAR,S-CoV 2 infection, and conceived of the present invention based on these findings.
Table 1 below shows an association ($p<5\times10^{-8}$) between SNPs on the OAS1 loci and COVID-19, which has been confirmed severe respiratory symptoms.

[Table 1]

| (Table 1) SNPs on OAS1 locus associated with very severe respiratory confirmed COVID-19 ($p < 5 \times 10^{-8}$). | | | | | | | |
|---|---|---|---|---|---|---|---|
| **Chr** | **Pos** | **ID** | **p-value*** | **log (Odds Ratio)*** | **Ref** | **Alt** | **Location to OAS1 transcript variant 1 (p46)** |
| 12 | 112,912,991 | rs2057778 | $6.86\times10^{-10}$ | 0.17 | G | T | Intron 3 (1756th base of 5273nt.) |
| 12 | 112,914,354 | rs4767023 | $1.70\times10^{-10}$ | 0.17 | T | C | Intron 3 (3119th base of 5273nt.) |

(continued)

| (Table 1) SNPs on OAS1 locus associated with very severe respiratory confirmed COVID-19 ($p < 5 \times 10^{-8}$). | | | | | | | |
|---|---|---|---|---|---|---|---|
| **Chr** | **Pos** | **ID** | **p-value*** | **log (Odds Ratio)*** | **Ref** | **Alt** | **Location to OAS1 transcript variant 1 (p46)** |
| 12 | 112,919,388 | rs10774671 | $1.10 \times 10^{-12}$ | 0.18 | G | A | Intron 5 (1688th base of 1688nt.) |
| 12 | 112,919,404 | rs1131476 | $4.54 \times 10^{-12}$ | 0.18 | G | A | Exon 6 (16th base of 515nt., CDS, Ala>Thr) |
| 12 | 112,919,637 | rs2660 | $2.68 \times 10^{-12}$ | 0.19 | G | A | Exon 6 (249th base of 515nt., 3' UTR) |
| * From GWAS anslysis for very severe respiratory confirmed COVID-19 | | | | | | | |

[Method for enhancing innate immune response]

In one aspect, the present disclosure relates to a method for enhancing an innate immune response of a subject or a cell. In the method for enhancing an innate immune response according to the present disclosure, the subject or the cell is a subject or a cell having an innate immune response that a gene to be subjected to splicing participates in, and this method includes administering a compound that affects alternative splicing to a subject or a cell, or bringing a compound that affects alternative splicing into contact with the subject or the cell.

Immunity is broadly divided into natural immunity, which is innate immunity, and acquired immunity, which is adaptive immunity. The term "innate immune response" used in the present disclosure refers to a nonspecific immune response, which is comprehensively performed against a pathogen (antigen) that has entered an organism, without using an antibody, for example. In one or more embodiments, in the innate immune response, pathogens (antigens), which have entered an organism, are recognized and taken up by antigen-presenting cells such as dendritic cells and macrophages, and thus the antigen-presenting cells are activated and secrete interferons, cytokines, and the like.

In one or more embodiments, the wording "the subject or the cell is a subject or a cell having an innate immune response that a gene to be subjected to splicing participates in" in the present disclosure indicates that the subject or the cell is a subject or the cell in which alternative splicing may occur in an innate immune-related gene in the subject or the cell, or an individual, immune cells and other cell types that exhibits an innate immune response due to such alternative splicing, and the immune cells are such as myeloid cell-line innate immune response cells (e.g., monocytes, granulocytes, red blood cells, and platelets) that are differentiated from common myeloid progenitors (CMPs) and innate immune response cells (e.g., cytotoxic natural killer cells) that are differentiated from common lymphoid progenitors (CLPs), and the other cell types may be involved in innate immune response regulation, and host cell types for viral infection.

In one or more embodiments, the subject or the cell having an innate immune response that a gene to be subjected to splicing participates in indicates that an innate immune-related gene, which expresses a protein having antiviral activity, in the subject and/or the cell has a mutation or polymorphism that causes alternative splicing such that the expression of the protein is controlled or suppressed.

In one or more embodiments, examples of the innate immune response that a gene to be subjected to splicing participates in include innate immune responses involving genes having a mutation or polymorphism (may include SNPs) that causes alternative splicing, which attenuates the innate immune responses. In one or more embodiments, examples of SNPs that cause alternative splicing, which attenuates an innate immune response, include SNP on OAS1 (2',5'-oligoadenylate synthase 1) such as A-allele at rs10774671, A-allele at rs1131476, andA-allele at rs2660.

In one or more embodiments, enhancement of the innate immune response of a subject or a cell can be confirmed using, as an indication, an increase in the expression level of interferons (e.g., IFN-$\alpha$, IFN-$\beta$, etc.) and cytokines (e.g., TNF-$\alpha$ etc.) in the subject or the cell to which the above compound is administered or with which the compound is brought into contact. In one or more embodiments, the expression level of the interferons and the cytokines can be found by measuring the amount of mRNA through RT-PCR or the like.

In one or more embodiments, examples of the innate immune response include innate immune responses to viruses. In one or more embodiments, examples of viruses include RNA viruses such as coronaviruses. In one or more embodiments, examples of the innate immune response include innate immune responses to infection with SARS-CoV, MARS-CoV, or SARS-CoV-2.

In one or more embodiments, examples of innate immune responses include innate immune responses realized by a pathway selected from TP53 (tumor protein p53), TLR3 (toll-like receptor 3), TLR7 (toll-like receptor 7), RARRES3

(retinoic acid receptor response factor (tazarotene induced)3), IFNA1 (interferon α1), IFNA2 (interferon α2), IFNA4 (interferon α4), IFNA5 (interferon α5), IFNA6 (interferon α6), IFNA7 (interferon α7), IFNA8 (interferon α8), IFNA10 (interferon α10), IFNA13 (interferon α13), IFNA14 (interferon α14), IFNA16 (interferon α16), IFNA17 (interferon α17), IFNA21 (interferon α21), IFNK (interferon κ), IFNB1 (interferon β1), IL6 (interleukin 6 (interferon β2)), TICAM1 (toll-like receptor adapter molecule 1), TICAM2 (toll-like receptor adapter molecule 2), MAVS (mitochondrial antiviral signaling protein), STAT1 (signal transducer and activator of transcription 1, 91 kDa), STAT2 (signal transducer and activator of transcription 2, 113 kDa), EIF2AK2 (eukaryotic translation initiation factor 2-α kinase 2), IRF3 (interferon regulatory factor 3), TBK1 (TANK-binding kinase 1), CDKN1A (cyclin-dependent kinase inhibitor 1A (p21, Cip 1)), CDKN2A (cyclin-dependent kinase inhibitor 2A (inhibit melanoma, p16, CDK4)), RNASEL (ribonuclease L (2',5'-oligoisoadenylate synthase dependent)), IFNAR1 (interferon (α, β, and ω) receptor 1), IFNAR2 (interferon (α, β, and ω) receptor 2), OAS1 (2',5'-oligoadenylate synthase 1, 40/46 kDa), OAS2 (2',5'-oligoadenylate synthase 2, 69/71 kDa), OAS3 (2',5'-oligoadenylate synthase 3, 100 kDa), OASL (2',5'-oligoadenylate synthase-like), RB1 (retinoblastoma 1), ISG15 (ISG15 ubiquitin-like modifier), ISG20 (interferon stimulated exonuclease gene, 20 kDa), IFIT1 (interferon induced protein 1 with tetratricopeptide repeats), IFIT2 (interferon induced protein 2 with tetratricopeptide repeats), IFIT3 (interferon induced protein 3 with tetratricopeptide repeats), and IFIT5 (interferon induced protein 5 with tetratricopeptide repeats). In one or more embodiments, examples of the innate immune response include OAS1, OAS2, and OAS3. In one or more embodiments, the innate immune response may be an innate immune response caused by a biologically active fragment, analog, or variant thereof.

In one or more embodiments, the subject or the cell has the A-allele of SNP rs10774671. In one or more embodiments, examples of the subject or the cell include a subject or a cell having an innate immune response attenuated by alternative splicing of a gene involved in the innate immune response. In one or more embodiments, the subject or the cell may have a mutation or polymorphism (may include SNPs), which causes alternative splicing that attenuates the innate immune response, in a gene involved in an innate immune response pathway.

In one or more embodiments, examples of the subject in the present disclosure include humans and animals other than humans. In one or more embodiments, examples of the animals other than humans include mammals such as mice, rats, guinea pigs, hamsters, rabbits, cats, dogs, sheep, pigs, cows, horses, goats, and monkeys, but the animals other than humans are not limited thereto. In one or more embodiments, examples of the cells in the present disclosure include cells derived from humans and animals other than humans, but the cells are not limited thereto.

In one or more embodiments, the method according to the present disclosure may include administering a compound that affects alternative splicing to a subject having an innate immune response that a gene to be subjected to splicing participates in and requiring enhancement of an innate immune response.

The compound that is administered to or brought into contact with the subject or the cell is a compound that affects alternative splicing, and examples thereof include, in one or more embodiments, compounds having an ability to inhibit protein kinases CLKs (CDC-like kinases) or an ability to protein kinases CLKs. Whether a compound has the ability to inhibit or activate CLKs can be confirmed using the method described in Examples.

In one or more embodiments, examples of the compound having an ability to inhibit or activate protein kinases CLKs include compounds having the ability to inhibit at least one kinase belonging to the CLK family, compounds having the activity of inhibiting the phosphorylation activity of at least one of the kinases belonging to the CLK family, and compounds having the ability to inhibit at least one selected from the group consisting of CLK1, CLK2, CLK3, and CLK4.

In one or more embodiments, examples of the compound that is administered to or brought into contact with the subject or the cell include compounds represented by Formula (II) to (VIII) below, prodrugs thereof, and pharmaceutically acceptable salts thereof.

Specific compounds will be described later.

[Composition for enhancing innate immune response]

In another aspect, the present disclosure relates to a composition for enhancing an innate immune response of a subject or a cell. In the composition for enhancing an innate immune response according to the present disclosure, the subject or the cell is a subject or a cell having an innate immune response that a gene to be subjected to splicing participates in, and the composition contains a compound that affects alternative splicing as an active ingredient.

In one or more embodiments, the composition according to the present disclosure may be a pharmaceutical composition.

In one or more embodiments, the composition according to the present disclosure contains, as an active ingredient, a compound that affects alternative splicing, and may further contain a pharmaceutically acceptable carrier, antiseptic, diluent, excipient, or other medicinally acceptable components. In one or more embodiments, examples of the compound contained in the composition according to the present disclosure serving as an active ingredient include compounds having an ability to inhibit or activate protein kinases CLKs. In one or more embodiments, examples of the compound contained in the composition according to the present disclosure serving as an active ingredient include

compounds represented by Formulae (II) to (VIII) below, prodrugs thereof, and pharmaceutically acceptable salts thereof.

In one or more embodiments, the content of the compound, which affects alternative splicing and is an active ingredient, with the composition of the present disclosure can be determined as appropriate depending on a dosage form, an administration method, a carrier, and the like. In one or more embodiments, the composition according to the present disclosure can be produced using an ordinary method by adding the compound according to the present disclosure at a percentage of 0.01% to 100% (w/w) or 0.1% to 95% (w/w) to the total amount of a preparation.

In one or more embodiments, a known drug preparation technique may be applied to the composition according to the present disclosure to have a dosage form that is suitable for an administration form. In one or more embodiments, examples of the administration form include oral administration and parenteral administration. In one or more embodiments, examples of dosage forms of a preparation for oral administration include tablets, capsules, granules, powders, pills, troches, syrups, and liquid formulations (e.g., solutions and suspensions). In one or more embodiments, examples of a preparation for parenteral administration include injections and aerosols. In one or more embodiments, these preparations may be produced using a known method using additives such as excipients, lubricants, binders, disintegrants, stabilizing agents, corrigents, and diluents.

In one or more embodiments, examples of the excipient include starches such as starch, potato starch, and corn starch, lactose, crystalline cellulose, and calcium hydrogen phosphate. In one or more embodiments, examples of the lubricant include ethyl cellulose, hydroxypropyl cellulose, hydroxypropyl methylcellulose, shellac, talc, carnauba wax, and paraffin. In one or more embodiments, examples of the binder include polyvinylpyrrolidone, macrogol, and compounds that are similar to those given as examples of the excipient. In one or more embodiments, examples of the disintegrant include compounds that are similar to those given as examples of the excipient, chemically-modified starches and celluloses such as croscarmellose sodium, sodium carboxymethyl starch, and crosslinked polyvinylpyrrolidone. In one or more embodiments, examples of the stabilizing agent include para-hydroxybenzoic acid esters such as methylparaben and propylparaben; alcohols such as chlorobutanol, benzyl alcohol, and phenylethyl alcohol; benzalkonium chloride; phenols such as phenol and cresol; thimerosal; dehydroacetic acid; and sorbic acid. In one or more embodiments, examples of the corrigent include sweeteners, acidulants, and flavors that are often used.

In one or more embodiments, ethanol, phenol, chlorocresol, purified water, distilled water, or the like can be used as a solvent to produce liquid formulations for oral administration, and a surfactant, a preservative, a tonicity agent, a pH adjusting agent, an emulsifying agent, or the like can also be used as needed. In one or more embodiments, liquid formulations for oral administration may further contain solubilizers, moistening agents, suspending agents, sweetening agents, corrigents, flavoring agents, or antiseptics.

Injections for parenteral administration may be sterile aqueous or non-aqueous liquid formulations, suspensions, or emulsions. In one or more embodiments, an aqueous solvent for injections may be distilled water or physiological saline. In one or more embodiments, a non-aqueous solvent for injections may be vegetable oil, alcohols, or polysorbate 80 (pharmacopeia name). Examples of the vegetable oil include propylene glycol, polyethylene glycol, and olive oil. Examples of the alcohols include ethanol. In one or more embodiments, the injection agent may further contain a tonicity agent, antiseptic agent, a moistening agent, an emulsifier, a dispersant, a stabilizing agent, or a dissolution adjuvant. In one or more embodiments, these preparations may be sterilized through filtration through a bacteria retaining filter, mixed with a germicide, or through irradiation. Further, a composition obtained by dissolving or suspending a sterile solid composition in sterile water or an injection solvent prior to use can also be used as preparations thereof.

A method for using a composition according to the present disclosure may change depending on the symptoms, age, administration method, and the like. In one or more embodiments, with the usage method, the composition can be intermittently or continuously administered orally, percutaneously, submucosally, subcutaneously, intramuscularly, intravascularly, intracerebrally, or intraperitoneally such that the concentration of the compound in the body that is an active ingredient is in the range of 100 pM to 1 mM. In a non-limiting embodiment, in the case of oral administration, the composition may be administered to a subject (an adult human if the subject is a human) in a dosage of 0.01 mg/kg body weight to 2000 mg/kg body weight, 0.1 mg/kg body weight to 500 mg/kg body weight, or 0.1 mg/kg body weight to 100 mg/kg body weight, which is expressed in terms of the compound contained, once or over several times in a day according to a symptom. In a non-limiting embodiment, in the case of intravenous administration, the composition may be administered to a subject (an adult human if the subject is a human) in a dosage of 0.001 mg/kg body weight to 50 mg/kg body weight, or 0.01 mg/kg body weight to 50 mg/kg body weight, once or over several times in a day according to a symptom.

[Method for preventing viral infectious disease, alleviating symptom, or treating viral infectious disease]

In another aspect, the present disclosure relates to a method for preventing a viral infectious disease, alleviating a symptom of a viral infectious disease, or treating a viral infectious disease. The method includes (1) confirming whether the subject satisfies at least one condition selected from the group consisting of a) to d) below and (2) administering a compound that affects alternative splicing to a subject or a cell thereof that satisfies the condition in (1) above or bringing

the compound into contact with the subject or the cell thereof.

a) The subject has an innate immune response that a gene to be subjected to splicing participates in,
b) the subject has an innate immune response attenuated by alternative splicing of a gene for the innate immune response,
c) the subject has a mutation or polymorphism (including an SNP) that causes alternative splicing that attenuates the innate immune response in a gene on an innate immune response pathway, and
d) the subject has the A-allele of SNP rs10774671.

In one or more embodiments, the method according to the present disclosure may include administering a compound that affects alternative splicing to the subject that satisfies at least one condition selected from the group consisting of a) to d) above and requires enhancement of an innate immune response or prevention of a viral infectious disease, alleviation of a symptom of a viral infectious disease, or treatment of a viral infectious disease.

In this aspect, the subject, the cell, the active ingredient, the pharmaceutical composition, the usage method, and the like may be the same as described above.

[Compound that affects alternative splicing (compound having ability to inhibit or activate protein kinases CLKs)]

In the present disclosure, in one or more embodiments, examples of the compound that affects alternative splicing (the compound having the ability to inhibit or activate protein kinases CLKs) include compounds represented by Formulae (II) to (VIII) below, prodrugs thereof, and pharmaceutically acceptable salts thereof.

[Chemical Formula 1]

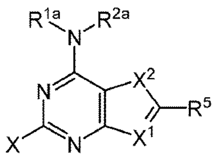 (II) 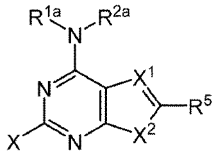 (II') 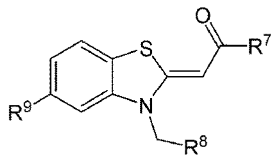 (III)

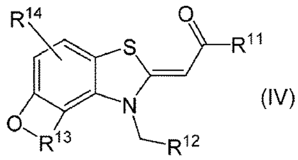 (IV) 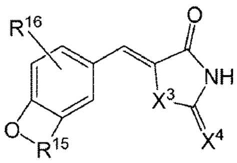 (V)

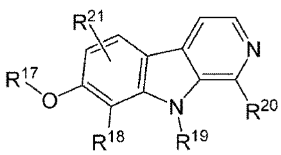 (VI) 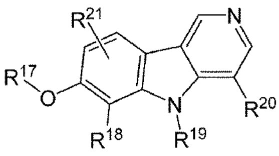 (VII) 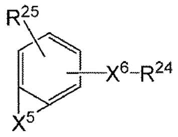 (VIII)

In Formulae (II) and (II'),

$R^{1a}$ and $R^{2a}$ each independently represent a hydrogen atom, a substituted or unsubstituted $C_1$-$C_6$ alkyl group, a substituted or unsubstituted benzyl group, a substituted or unsubstituted heteroarylmethyl group, a substituted or unsubstituted heteroarylethyl group, a substituted or unsubstituted aryloxy group, a substituted or unsubstituted heteroaryloxy group, a substituted or unsubstituted alkoxyamidoalkyl group, a substituted or unsubstituted aryl group, or a substituted or unsubstituted heteroaryl group, or alternatively, $R^{1a}$ and $R^{2a}$ bind to each other to form a ring together with N, and the ring is a substituted or unsubstituted monocyclic heterocyclic ring, or a substituted or unsubstituted bicyclic heterocyclic ring,
$R^5$ represents a hydrogen atom, a halogen atom, or a substituted or unsubstituted $C_1$-$C_6$ alkoxy group;
$X^1$ represents N or CH;
$X^2$ represents $-N(R^3)-$, S, or O;
$R^3$ represents a hydrogen atom, a $C_1$-$C_6$ alkyl group, a benzyl group, a heteroarylmethyl group, a substituted or unsubstituted aryl group, a substituted or unsubstituted heteroaryl group, or $CH_2OC(O)R^4$-:

$R^4$ represents a $C_1$-$C_6$ alkyl group, a benzyl group, a heteroarylmethyl group, a substituted or unsubstituted aryl group, or a substituted or unsubstituted heteroaryl group; and
X represents a hydrogen atom, a halogen atom, an amino group, an $R^{1a}$- and $R^{2a}$-substituted amino group, an azido group, a cyano group, a nitro group, a hydroxy group, a $C_1$-$C_6$ alkyloxy group, a substituted or unsubstituted aryloxy group, a substituted or unsubstituted heteroaryloxy group, a mercapto group, a $C_1$-$C_6$ alkylthio group, a substituted or unsubstituted arylthio group, a substituted or unsubstituted heteroarylthio group, a benzyl group, a heteroarylmethyl group, a substituted or unsubstituted aryl group, or a substituted or unsubstituted heteroaryl group.

In one or more embodiments, examples of the compound represented by Formula (II) or (II') include compounds below,

[Chemical Formula 2]

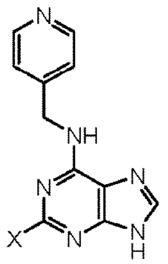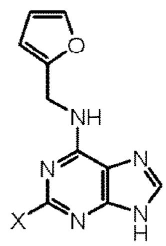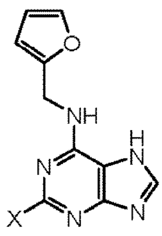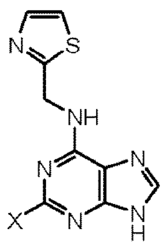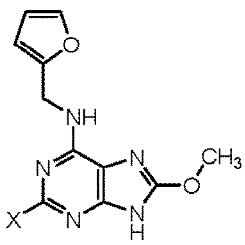

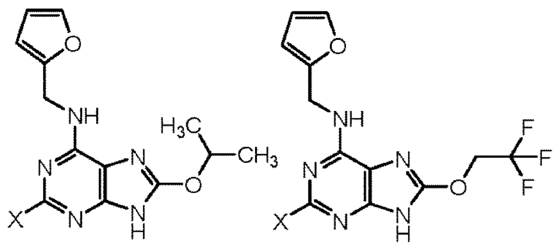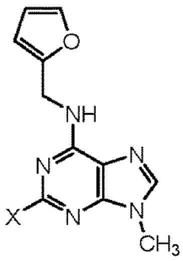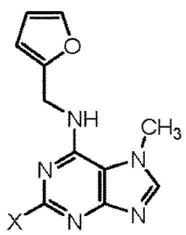

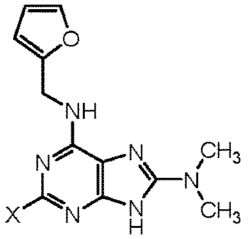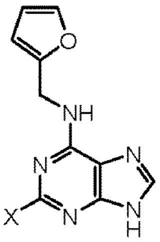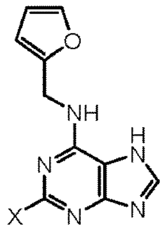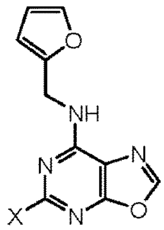

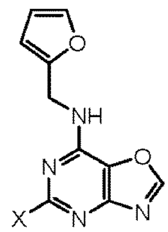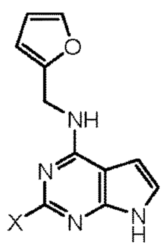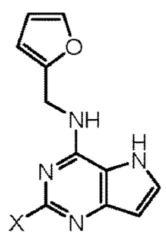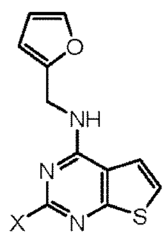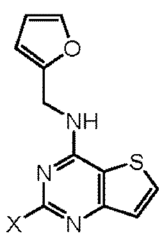

where X represents a halogen atom. In one or more embodiments, X represents a chlorine atom, a fluorine atom, or an iodine atom, and preferably a chlorine atom or a fluorine atom.

In Formula (III),

$R^7$ and $R^8$ each independently represent a hydrogen atom, a halogen-substituted or unsubstituted $C_1$-$C_{10}$ alkyl group, or a $C_2$-$C_6$ alkenyl group;
$R^9$ represents a hydrogen atom, a halogen atom, or a halogen-substituted or unsubstituted $C_1$-$C_{10}$ alkyl group, $-OR^{10}$, $-NHR^{10}$, or $-N(R^{10})_2$; and
$R^{10}$ represents a hydrogen atom or a $C_1$-$C_{10}$ alkyl group.

In one or more embodiments, in Formula (III), $R^7$ represents a hydrogen atom, a methyl group, a halogenated

methyl group, an ethyl group, a halogenated ethyl group, a propyl group, or a halogenated propyl group, $R^8$ represents a hydrogen atom, a methyl group, an ethyl group, a propyl group, a butyl group, a pentyl group, a hexyl group, or $-CH_2CH=CH_2$, and $R^9$ represents a halogen atom, a hydroxy group, $-OCH_3$, or $-OCH_2CH_3$.

In one or more embodiments, in Formula (III), $R^7$ represents a hydrogen atom, a methyl group, a trifluoromethyl group, an ethyl group, a halogenated ethyl group, a propyl group, or a halogenated propyl group, $R^8$ represents a hydrogen atom, a methyl group, an ethyl group, a propyl group, a butyl group, a pentyl group, a hexyl group, or $-CH_2CH=CH_2$, and $R^9$ represents a fluorine atom, a chlorine atom, a bromine atom, a hydroxy group, $-OCH_3$, or $-OCH_2CH_3$.

In one or more embodiments, examples of the compound represented by Formula (III) include the compounds below.

[Chemical Formula 3]

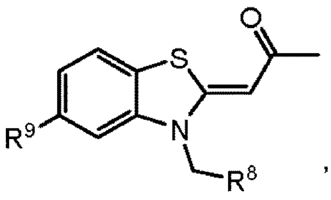 , 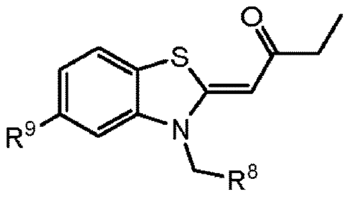 , 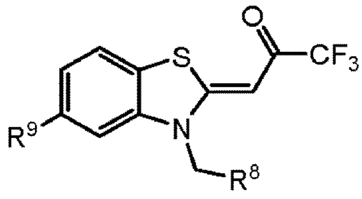

In one or more embodiments, in the above compound, $R^8$ represents a hydrogen atom, a methyl group, an ethyl group, a propyl group, a butyl group, a pentyl group, a hexyl group, or $-CH_2CH=CH_2$, and $R^9$ represents a fluorine atom, a chlorine atom, a bromine atom, $-OCH_3$, or $-OCH_2CH_3$.

In one or more embodiments, examples of the compound represented by Formula (III) include the compounds below.

[Chemical Formula 4]

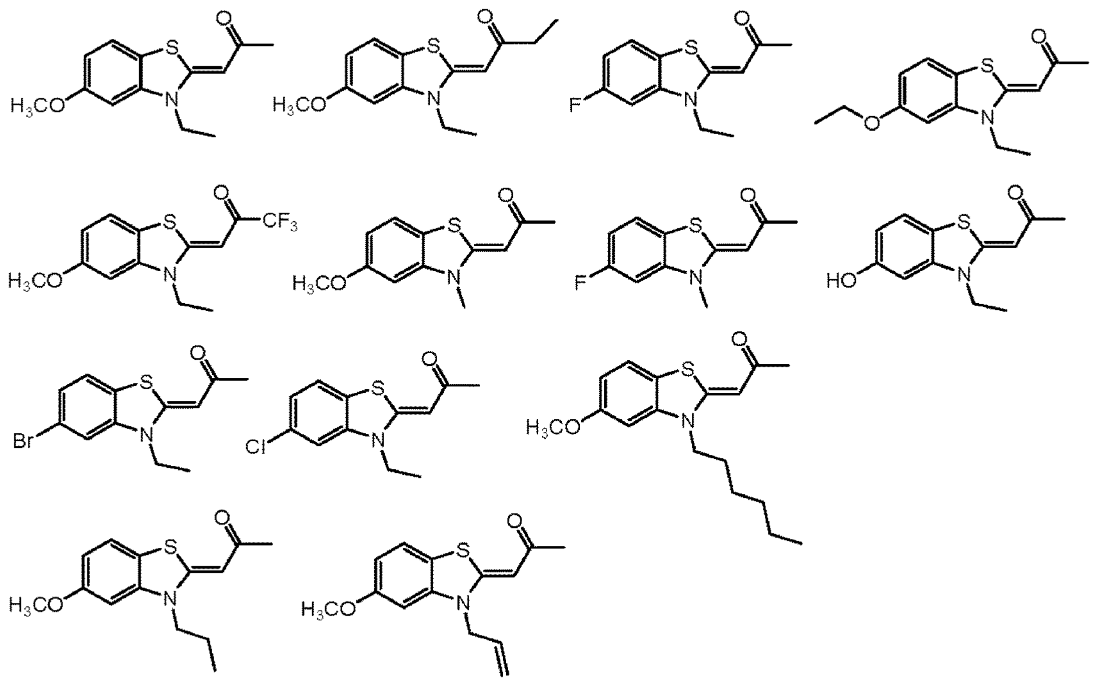

In Formula (IV),

$R^{11}$ and $R^{12}$ each independently represent a hydrogen atom or a $C_1$-$C_6$ alkyl group;
$R^{13}$ represents

[Chemical Formula 5]

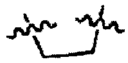, 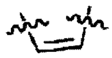, or 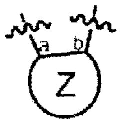

where Z forms, together with atoms marked with a and b, a ring selected from the group consisting of one benzene ring, one heteroaromatic ring, an aromatic ring fused with one or more benzene rings, a heteroaromatic ring fused with one or more heteroaromatic rings, a mixed fused polycyclic ring in which one or more benzene rings and one or more heteroaromatic rings are fused, and cycloaliphatic groups, and the ring may include one or more substituents, the substituents being a hydrogen atom, a halogen atom, or a $C_1$-$C_6$ alkyl group; and

$R^{14}$ represents a hydrogen atom, a halogen atom, or a $C_1$-$C_6$ alkyl group.

In one or more embodiments, examples of the compound represented by Formula (IV) include the compounds below,

[Chemical Formula 6]

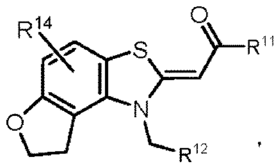, 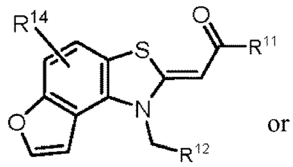 or 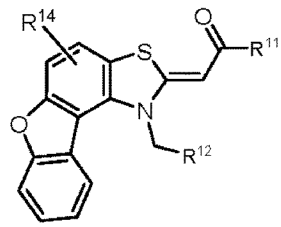

where $R^{11}$ and $R^{12}$ each independently represent a hydrogen atom or a $C_1$-$C_6$ alkyl group, and $R^{14}$ represents a hydrogen atom, a halogen atom, or $C_1$-$C_6$ alkyl group. In one or more embodiments, $R^{11}$ and $R^{12}$ each independently represent a hydrogen atom, a methyl group, an ethyl group, a propyl group, an isopropyl group, a butyl group, or a tert-butyl group, $R^{14}$ represents a hydrogen atom, a halogen atom, a methyl group, an ethyl group, a propyl group, or an isopropyl group, preferably $R^{11}$ and $R^{12}$ each independently represent a hydrogen atom, a methyl group, an ethyl group, a propyl group, an isopropyl group, a butyl group, or a tert-butyl group, $R^{14}$ represents a hydrogen atom, and more preferably $R^{11}$ and $R^{12}$ each independently represent a hydrogen atom, a methyl group, an ethyl group, a propyl group, or an isopropyl group, and $R^{14}$ represents a hydrogen atom.

In one or more embodiments, examples of the compound represented by Formula (IV) include the compounds below.

[Chemical Formula 7]

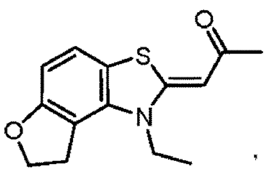, 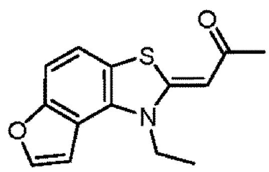 or 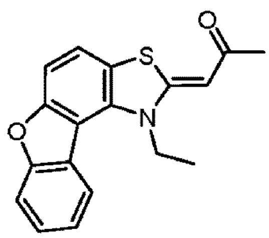

In Formula (V),

$X^3$ and $X^4$ each independently represent S or NH;

$R^{15}$ represents

[Chemical Formula 8]

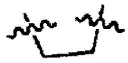, 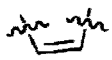, or 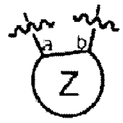

where Z forms, together with atoms marked with a and b, a ring selected from the group consisting of one benzene ring, one heteroaromatic ring, an aromatic ring fused with one or more benzene rings, a heteroaromatic ring fused with one or more heteroaromatic rings, a mixed fused polycyclic ring in which one or more benzene rings and one or more heteroaromatic rings are fused, and cycloaliphatic groups, and the ring may include one or more substituents, the substituents being a hydrogen atom, a halogen atom, or a $C_1$-$C_6$ alkyl group; and

$R^{16}$ represents a hydrogen atom, a halogen atom, or a $C_1$-$C_6$ alkyl group.

In one or more embodiments, examples of the compound represented by Formula (V) include the compounds below,

[Chemical Formula 9]

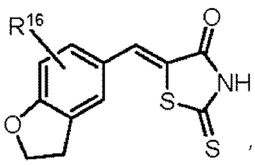, 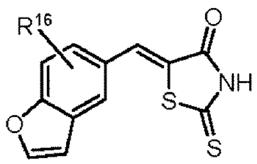, 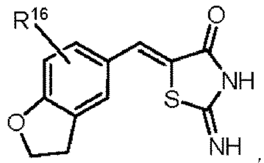, 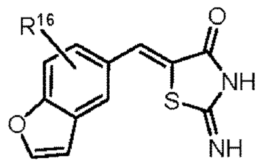,

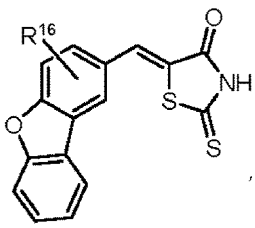, 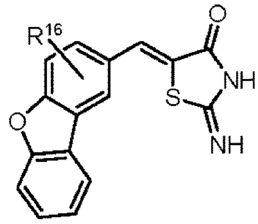 or 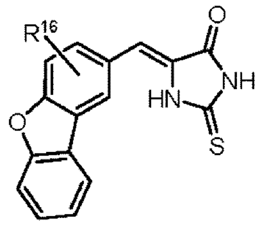

where $R^{16}$ represents a hydrogen atom, a halogen atom, or a $C_1$-$C_6$ alkyl group, preferably a hydrogen atom, a halogen atom, a methyl group, or an ethyl group, more preferably a hydrogen atom, a methyl group, or an ethyl group.

In one or more embodiments, examples of the compound represented by Formula (V) include the compounds below.

[Chemical Formula 10]

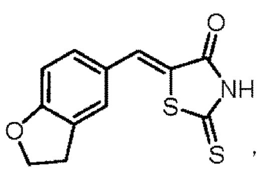, 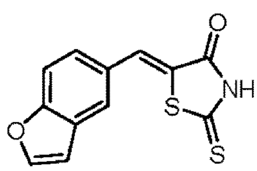, 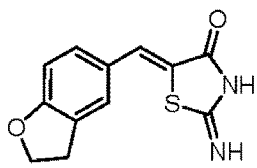, 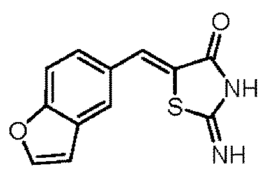,

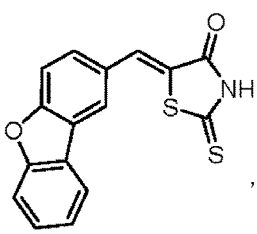, 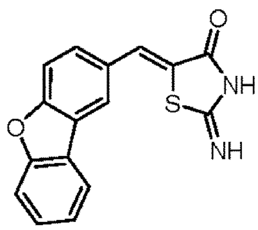 or 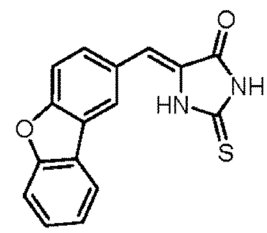

In Formulae (VI) and (VII),

$R^{17}$ and $R^{19}$ each independently represent a hydrogen atom, a $C_1$-$C_6$ alkyl group, a benzyl group, a heteroarylmethyl group, a substituted or unsubstituted aryl group, or a substituted or unsubstituted heteroaryl group;

$R^{18}$ represents -$R^{22}$, -C≡C-$R^{22}$, -CH=CH-$R^{22}$, or -O-($CH_2$)n-$R^{22}$, n is 1 to 6, $R^{22}$ represents a hydrogen atom, a hydroxy group, a $C_1$-$C_8$ alkyl group, -Si($R^{23}$)$_3$, a substituted or unsubstituted phenyl group, a monocyclic heteroaromatic group, or a cycloaliphatic group, or alternatively, $R^{17}$ and $R^{18}$ bind to each other to form a ring, and -$R^{17}$-$R^{18}$- is substituted by -($CH_2$)m-$CH_2$-, -CH=CH-, -$(CH_2)_m$-O-, or a halogen atom, m is 1 to 6, $R^{23}$ represents a hydrogen atom, a $C_1$-$C_6$ alkyl group, a trihalomethyl group, or a hydroxy group, and three $R^{23}$ of -Si($R^{23}$)$_3$ may be different from each other; and

$R^{20}$ and $R^{21}$ represent a hydrogen atom or a $C_1$-$C_6$ alkyl group.

In one or more embodiments, examples of the compound represented by Formula (VI) or (VII) include the compounds below,

[Chemical Formula 11]

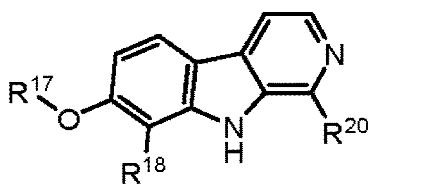

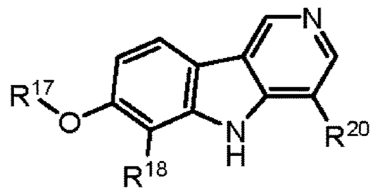

where $R^{17}$ represents, in one or more embodiments, a hydrogen atom, a methyl group, an ethyl group, a propyl group, a butyl group, a pentyl group, a hexyl group, a benzyl group, or a phenyl group, and preferably a methyl group, an ethyl group, a propyl group, a butyl group, or a tert-butyl group;

where $R^{18}$ represents, in one or more embodiments, a hydrogen atom, a hydroxy group, a methyl group, an ethyl group, a propyl group, a butyl group, a pentyl group, a hexyl group, a benzyl group, or a phenyl group, and preferably a hydrogen atom, a methyl group, or an ethyl group; or

in one or more embodiments, $R^{17}$ and $R^{18}$ bind to each other to form a ring, and -$R^{17}$-$R^{18}$- represents -$CH_2$-$CH_2$-, -$(CH_2)_2$-$CH_2$-, -$(CH_2)_3$-$CH_2$-, -CH=CH-, -($CH_2$)-O-, -$(CH_2)_2$-O-, or -$(CH_2)_3$-O-, or these may be substituted by a halogen atom; and

$R^{20}$ represents, in one or more embodiments, a hydrogen atom, a methyl group, an ethyl group, a propyl group, or a butyl group.

In one or more embodiments, examples of the compound represented by Formula (VI) or (VII) include the compounds below.

[Chemical Formula 12]

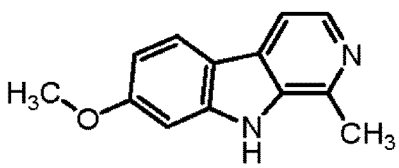

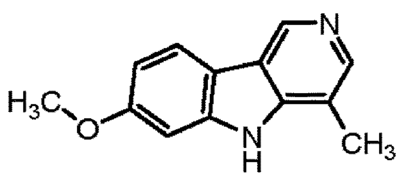

In Formula (VIII),

$X^5$ represents

[Chemical Formula 13]

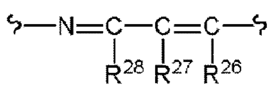
,
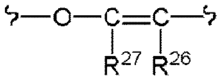
, or
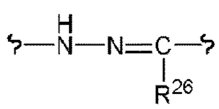

where $R^{26}$, $R^{27}$, and $R^{28}$ each independently represent a hydrogen atom, a halogen atom, a carboxyl group, an amino group, a hydroxy group, a $C_1$-$C_4$ alkyl group, or a halogen-substituted $C_1$-$C_4$ alkyl group;
$X^6$ represents -(atomic bonding) or -NH-;
$R^{24}$ represents

[Chemical Formula 14]

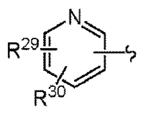,
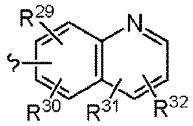,
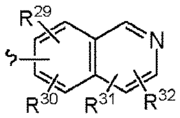,
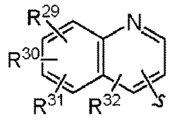, or
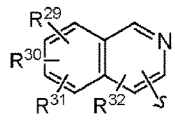

where $R^{29}$, $R^{30}$, $R^{31}$, and $R^{32}$ each independently represent a hydrogen atom, a halogen atom, a carboxyl group, an amino group, a hydroxy group, a $C_1$-$C_4$ alkyl group, or a halogen-substituted $C_1$-$C_4$ alkyl group; and
$R^{25}$ represents a hydrogen atom, a halogen atom, a carboxyl group, an amino group, a hydroxy group, or a halogen-substituted or unsubstituted $C_1$-$C_4$ alkyl group.

In one or more embodiments, examples of the compound represented by Formula (VIII) include the compounds below,

[Chemical Formula 15]

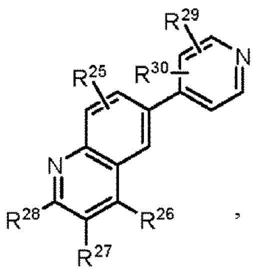,
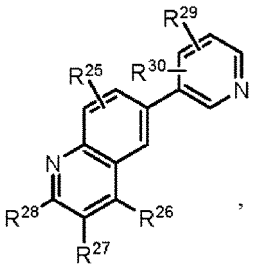,
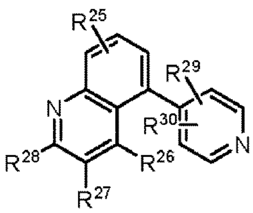 or
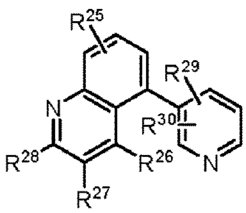

where $R^{25}$, $R^{26}$, $R^{27}$, $R^{28}$, $R^{29}$, and $R^{30}$ each independently represent a hydrogen atom, a halogen atom, a carboxyl group, an amino group, a hydroxy group, a $C_1$-$C_4$ alkyl group, or a halogen-substituted $C_1$-$C_4$ alkyl group. In one or more embodiments, $R^{25}$, $R^{26}$, $R^{27}$, $R^{28}$, $R^{29}$, and $R^{30}$ each independently represent a hydrogen atom, a halogen atom, a methyl group, an ethyl group, a propyl group, an isopropyl group, a halogenated methyl group, a halogenated ethyl group, or a halogenated propyl group, preferably one, two, three, four, or five of $R^{25}$, $R^{26}$, $R^{27}$, $R^{28}$, $R^{29}$, and $R^{30}$ represent hydrogen atoms, and the others of $R^{25}$, $R^{26}$, $R^{27}$, $R^{28}$, $R^{29}$, and $R^{30}$ each independently represent a hydrogen atom, a halogen atom, a methyl group, an ethyl group, a propyl group, an isopropyl group, a halogenated methyl group, a halogenated ethyl group, or a halogenated propyl group, or all of $R^{25}$, $R^{26}$, $R^{27}$, $R^{28}$, $R^{29}$, and $R^{30}$ represent hydrogen atoms.

In one or more embodiments, examples of the compound represented by Formula (VIII) include the compounds below,

[Chemical Formula 16]

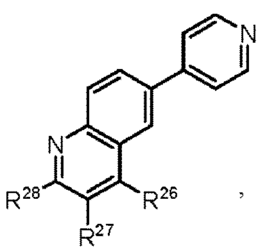,
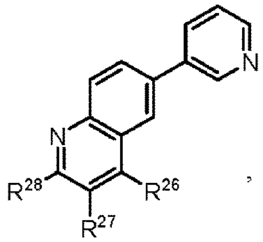,
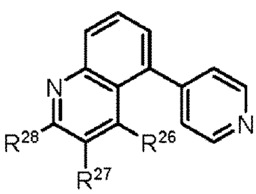 or
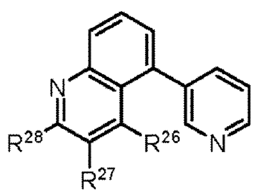

where $R^{26}$, $R^{27}$, and $R^{28}$ each independently represent a hydrogen atom, a halogen atom, a carboxyl group, an amino group

group, a hydroxy group, a $C_1$-$C_4$ alkyl group, or a halogen-substituted $C_1$-$C_4$ alkyl group. In one or more embodiments, $R^{26}$, $R^{27}$, and $R^{28}$ each independently represent a hydrogen atom, a halogen atom, a methyl group, an ethyl group, a propyl group, an isopropyl group, a halogenated methyl group, a halogenated ethyl group, or a halogenated propyl group, preferably one or two of $R^{26}$, $R^{27}$, and $R^{28}$ represent hydrogen atoms, and the others of $R^{26}$, $R^{27}$, and $R^{28}$ represent hydrogen atoms, halogen atoms, methyl groups, ethyl groups, propyl groups, isopropyl groups, halogenated methyl groups, halogenated ethyl groups, or halogenated propyl groups, or all of $R^{26}$, $R^{27}$, and $R^{28}$ are hydrogen atoms.

In one or more embodiments, examples of the compound represented by Formula (VIII) include the compounds below,

[Chemical Formula 17]

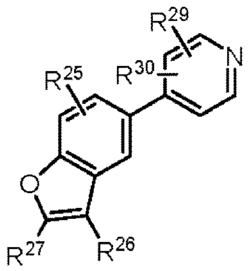

or

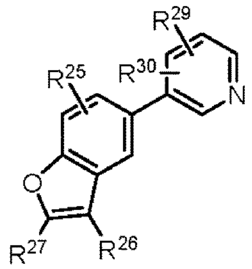

where $R^{25}$, $R^{26}$, $R^{27}$, $R^{29}$, and $R^{30}$ each independently represent a hydrogen atom, a halogen atom, a carboxyl group, an amino group, a hydroxy group, a $C_1$-$C_4$ alkyl group, or a halogen-substituted $C_1$-$C_4$ alkyl group. In one or more embodiments, $R^{25}$, $R^{26}$, $R^{27}$, $R^{29}$, and $R^{30}$ each independently represent a hydrogen atom, a halogen atom, a methyl group, an ethyl group, a propyl group, an isopropyl group, a halogenated methyl group, a halogenated ethyl group, or a halogenated propyl group, preferably one, two, three, or four of $R^{25}$, $R^{26}$, $R^{27}$, $R^{29}$, and $R^{30}$ represent hydrogen atoms, and the others of $R^{25}$, $R^{26}$, $R^{27}$, $R^{29}$, and $R^{30}$ represent hydrogen atoms, halogen atoms, methyl groups, ethyl groups, propyl groups, isopropyl groups, halogenated methyl groups, halogenated ethyl groups, or halogenated propyl groups, or all of $R^{25}$, $R^{26}$, $R^{27}$, $R^{29}$, and $R^{30}$ represent hydrogen atoms.

In one or more embodiments, examples of the compound represented by Formula (VIII) include the compounds below,

[Chemical Formula 18]

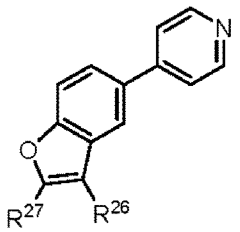

or

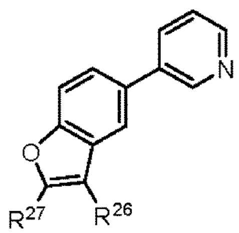

where $R^{26}$ and $R^{27}$ each independently represent a hydrogen atom, a halogen atom, a carboxyl group, an amino group, a hydroxy group, a $C_1$-$C_4$ alkyl group, or a halogen-substituted $C_1$-$C_4$ alkyl group. In one or more embodiments, $R^{26}$ and $R^{27}$ each independently represent a hydrogen atom, a halogen atom, a methyl group, an ethyl group, a propyl group, an isopropyl group, a halogenated methyl group, a halogenated ethyl group, or a halogenated propyl group, preferably one of $R^{26}$ and $R^{27}$ represent a hydrogen atom, and the other of $R^{26}$ and $R^{27}$ represent a hydrogen atom, a halogen atom, a methyl group, an ethyl group, a propyl group, an isopropyl group, a halogenated methyl group, a halogenated ethyl group, or a halogenated propyl group, or both of $R^{26}$ and $R^{27}$ are hydrogen atoms.

In one or more embodiments, examples of the compound represented by Formula (VIII) include the compounds below,

[Chemical Formula 19]

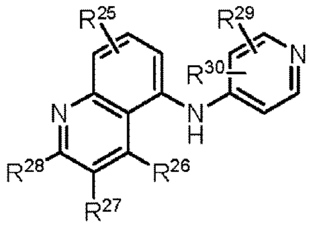

,

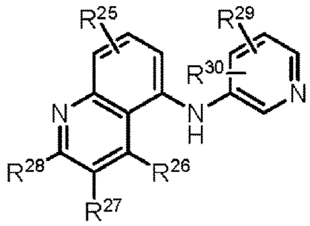

or

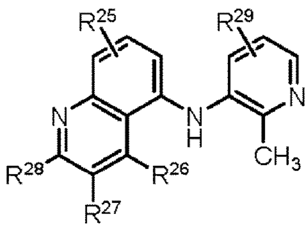

where $R^{25}$, $R^{26}$, $R^{27}$, $R^{28}$, $R^{29}$, and $R^{30}$ each independently represent a hydrogen atom, a halogen atom, a carboxyl group, an amino group, a hydroxy group, a $C_1$-$C_4$ alkyl group, or a halogen-substituted $C_1$-$C_4$ alkyl group. In one or more embodiments, $R^{25}$, $R^{26}$, $R^{27}$, $R^{28}$, $R^{29}$, and $R^{30}$ each independently represent a hydrogen atom, a halogen atom, a methyl group, an ethyl group, a propyl group, an isopropyl group, a halogenated methyl group, a halogenated ethyl group, or a halogenated propyl group, preferably one, two, three, four, or five of $R^{25}$, $R^{26}$, $R^{27}$, $R^{28}$, $R^{29}$, and $R^{30}$ represent hydrogen atoms, and the others of $R^{25}$, $R^{26}$, $R^{27}$, $R^{28}$, $R^{29}$, and $R^{30}$ represent hydrogen atoms, halogen atoms, methyl groups, ethyl groups, propyl groups, isopropyl groups, halogenated methyl groups, halogenated ethyl groups, or halogenated propyl groups, or all of $R^{25}$, $R^{26}$, $R^{27}$, $R^{28}$, $R^{29}$, and $R^{30}$ represent hydrogen atoms.

In one or more embodiments, examples of the compound represented by Formula (VIII) include the compounds below,

[Chemical Formula 20]

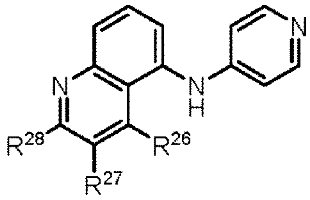

,

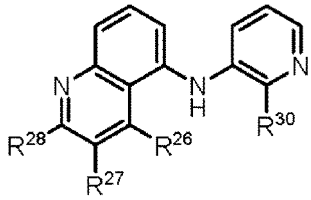

or

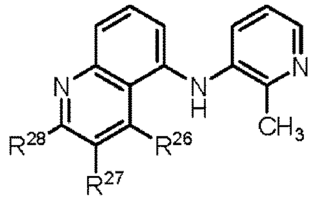

where $R^{26}$, $R^{27}$, and $R^{28}$ each independently represent a hydrogen atom, a halogen atom, a carboxyl group, an amino group, a hydroxy group, a $C_1$-$C_4$ alkyl group, or a halogen-substituted $C_1$-$C_4$ alkyl group, and $R^{30}$ represents a hydrogen atom, a halogen atom, a carboxyl group, an amino group, a hydroxy group, a $C_1$-$C_4$ alkyl group, or a halogen-substituted $C_1$-$C_4$ alkyl group. In one or more embodiments, $R^{26}$, $R^{27}$, and $R^{28}$ each independently represent a hydrogen atom, a halogen atom, a methyl group, an ethyl group, a propyl group, an isopropyl group, a halogenated methyl group, a halogenated ethyl group, or a halogenated propyl group, $R^{30}$ represents a hydrogen atom, a methyl group, an ethyl group, a halogenated methyl group, or a halogenated ethyl group, preferably one or two of $R^{26}$, $R^{27}$, and $R^{28}$ represent hydrogen atoms, and the others of $R^{26}$, $R^{27}$, and $R^{28}$ represent hydrogen atoms, halogen atoms, methyl groups, ethyl groups, propyl groups, isopropyl groups, halogenated methyl groups, halogenated ethyl groups, or halogenated propyl groups, and $R^{30}$ represents a hydrogen atom or a methyl group, or all of $R^{26}$, $R^{27}$, and $R^{28}$ represent hydrogen atoms.

In one or more embodiments, examples of the compound represented by Formula (VIII) include the compounds below,

[Chemical Formula 21]

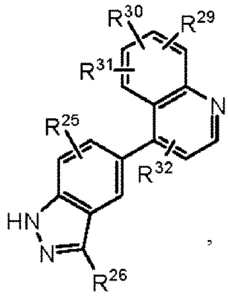 , 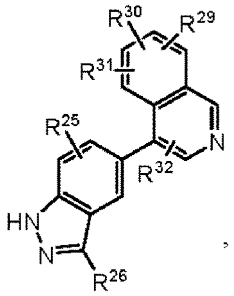 , 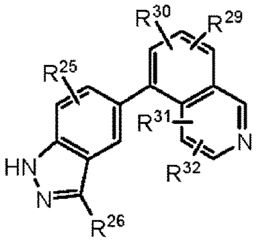 , 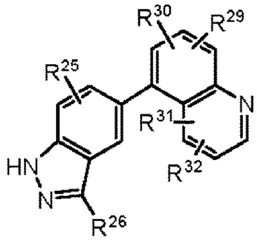 ,

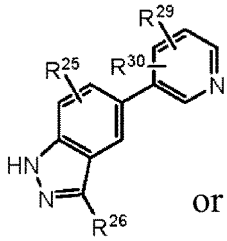 or 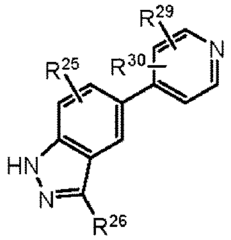

where $R^{25}$, $R^{26}$, $R^{29}$, $R^{30}$, $R^{31}$, and $R^{32}$ each independently represent a hydrogen atom, a halogen atom, a carboxyl group, an amino group, a hydroxy group, a $C_1$-$C_4$ alkyl group, or a halogen-substituted $C_1$-$C_4$ alkyl group. In one or more embodiments, $R^{25}$, $R^{26}$, $R^{29}$, $R^{30}$, $R^{31}$, and $R^{32}$ each independently represent a hydrogen atom, a halogen atom, a methyl group, an ethyl group, a propyl group, an isopropyl group, a halogenated methyl group, a halogenated ethyl group, or a halogenated propyl group, preferably one, two, three, four, or five of $R^{25}$, $R^{26}$, $R^{29}$, $R^{30}$, $R^{31}$, and $R^{32}$ represent hydrogen atoms, and the others of $R^{25}$, $R^{26}$, $R^{29}$, $R^{30}$, $R^{31}$, and $R^{32}$ represent hydrogen atoms, halogen atoms, methyl groups, ethyl groups, propyl groups, isopropyl groups, halogenated methyl groups, halogenated ethyl groups, or halogenated propyl groups, or all of $R^{25}$, $R^{26}$, $R^{29}$, $R^{30}$, $R^{31}$, and $R^{32}$ represent hydrogen atoms.

In one or more embodiments, examples of the compound represented by Formula (VIII) include the compounds below,

[Chemical Formula 22]

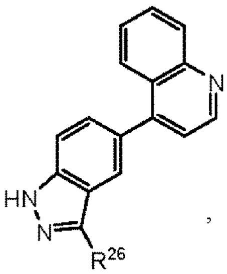 , 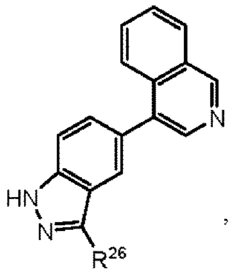 , 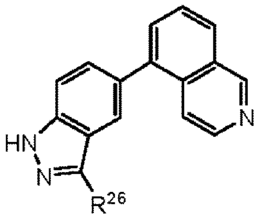 , 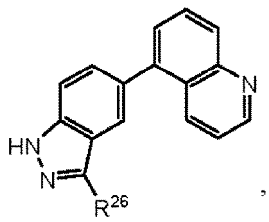 ,

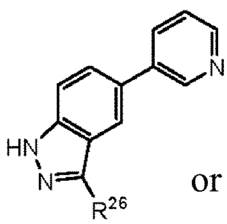 or 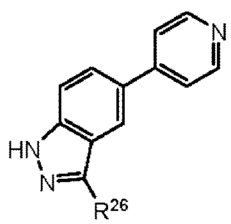

where $R^{26}$ represent a hydrogen atom, a halogen atom, a carboxyl group, an amino group, a hydroxy group, a $C_1$-$C_4$ alkyl group, or a halogen-substituted $C_1$-$C_4$ alkyl group. $R^{26}$ represents, in one or more embodiments, a hydrogen atom, a halogen atom, a methyl group, an ethyl group, a propyl group, an isopropyl group, a halogenated methyl group, a halogenated ethyl group, or a halogenated propyl group, and preferably a hydrogen atom or a methyl group.

In one or more embodiments, examples of the compound represented by Formula (VIII) include the compounds below.

[Chemical Formula 23]

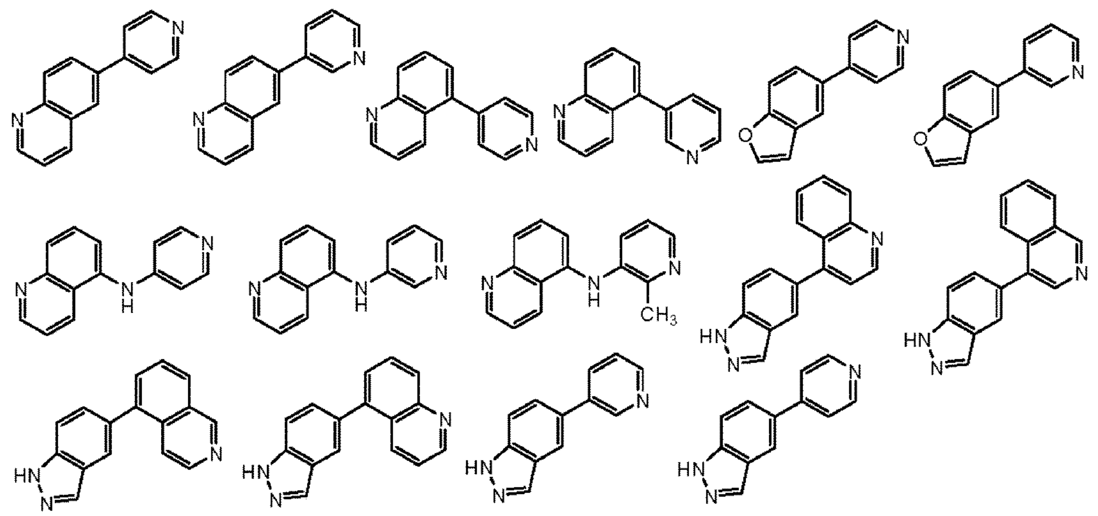

A "pharmaceutically acceptable salt" in the present disclosure refers to a salt that can be used as a pharmaceutical agent, and in one or more embodiments, examples thereof include basic salts or acidic salts.

In one or more embodiments, examples of the "basic salts" include alkali metal salts such as sodium salts, potassium salts, and lithium salts; alkaline earth salts such as magnesium salts and calcium salts; organic base salts such as N-methylmorpholine salts, triethylamine salts, tributylamine salts, diisopropylethylamine salts, dicyclohexylamine salts, N-methylpiperidine salts, pyridine salts, 4-pyrrolidinopyridine salts, and picoline salts, and amino acid salts such as glycine salts, lysine salts, arginine salts, ornithine salts, glutamate, and aspartate, and alkali metal salts are preferable.

In one or more embodiments, examples of "acidic salts" include inorganic acid salts including hydrogen halide salts such as hydrofluoric acid salts, hydrochloric acid salts, hydrobromic acid salts, and hydroiodic acid salts, nitrates, perchlorates, sulfates, and phosphates; organic acid salts including lower-alkane-sulfonates such as methanesulfonates, trifluoromethanesulfonates, and ethanesulfonates, arylsulfonates such as benzenesulfonates and p-toluenesulfonates, acetates, malates, fumarates, succinates, citrates, ascorbates, tartrates, oxalates, and maleates; and amino acid salts such as glycine salts, lysine salts, arginine salts, ornithine salts, glutamates, and aspartates, and hydrogen halides (in particular, hydrochloric acid salts) are preferable.

A pharmaceutically acceptable salt of a compound according to the present disclosure may include a hydrate. Also, in the present disclosure, a "salt of a compound" may include a solvate that can be formed as a result of a compound absorbing a certain type of solvent.

Various isomers such as geometric isomers (e.g., cis and trans isomers), tautomers, rotamers, or optical isomers (enantiomers) (e.g., d isomers and l isomers), and diastereomers of the compound according to the present disclosure, pharmaceutically acceptable salts thereof, or solvates thereof may be present depending on types and combinations of substituents. The compound according to the present disclosure encompasses all of the isomers, stereoisomers, and mixtures of these isomers and stereoisomers in any ratio unless specifically limited. These isomers in a mixture can be separated from each other using a known separation means.

The compound according to the present disclosure also includes a labeled compound, that is, a compound whose one or more atoms are substituted by an isotope (e.g., $^{2}H$, $^{3}H$, $^{13}C$, $^{14}C$, $^{35}S$, or the like).

Compounds that are to be converted through reactions with enzymes, gastric acid, or the like under physiological conditions in a living body, into compounds represented by Formulae (II) to (VIII), which are active ingredients of the pharmaceutical composition according to the present invention, that is, the compounds that undergo enzymatic oxidation, reduction, hydrolysis, or the like to be converted into compounds represented by Formulae (II) to (VIII), or compounds that undergo hydrolysis or the like due to gastric acid or the like to be converted into compounds represented by Formulae (II) to (VIII) are included in the present disclosure as "medicinally acceptable prodrug compounds". In one or more embodiments, a "prodrug" refers to compounds having a group, which can be converted into an amino group, a hydroxy group, a carboxyl group, or the like of the compound through hydrolysis or under physiological conditions, and examples of the group that forms such a prodrug include groups described in Prog. Med., vol. 5, p. 2157 to 2161, 1985, and the like.

When compounds represented by Formulae (II) to (VIII) have an amino group, examples of a prodrug include compounds whose amino group is acylated, alkylated, or phosphorylated (e.g., compounds whose amino group is eicosanoylated, alanylated, pentylaminocarbonylated, (5-methyl-2-oxo-1,3-dioxolen-4-yl) methoxylcarbonylated, tetrahydrofuranylated, pyrrolidyl methylated, or pivaloyloxymethylated, tert-butylated). When compounds represented by

Formulae (II) to (VIII) have a hydroxy group, examples of a prodrug include compounds whose hydroxy group is acylated, alkylated, phosphorylated, or borated (e.g., compounds whose hydroxy group is acetylated, palmitoylated, propanoylated, pivaloylated, succinylated, fumarylated, alanylated, or dimethylaminomethylcarbonylated). Also, when compounds represented by Formulae (II) to (VIII) have a carboxy group, examples of a prodrug include compounds whose carboxy group is esterified or amidated (e.g., compounds whose carboxy group is ethyl esterified, phenyl esterified, carboxymethyl esterified, dimethylaminomethyl esterified, pivaloyloxymethyl esterified, ethoxylcarbonyloxyethyl esterified, or methylamidated).

The present disclosure may relate to one or more embodiments below;

[A1] A method for enhancing an innate immune response of a subject or a cell,

the subject or the cell being a subject or a cell having an innate immune response that a gene to be subjected to splicing participates in,

the method including administering a compound that affects alternative splicing to the subject or the cell, or bringing a compound that affects alternative splicing into contact with the subject or the cell.

[A2] The method according to [A1], in which the subject or the cell is a subject or a cell having an innate immune response attenuated by alternative splicing of a gene for the innate immune response.

[A3] The method according to [A1] or [A2], in which the compound is a compound having an ability to inhibit or activate a protein kinase CLK (CDC-like kinase).

[A4] The method according to [A1] to [A3], in which the subject or the cell has a mutation or polymorphism (may include an SNP) that causes alternative splicing that attenuates the innate immune response in a gene on an innate immune response pathway.

[A5] The method according to any one of [A1] to [A4], in which the innate immune response is an innate immune response realized by a pathway selected from the group consisting of TP53, TLR3, TLR7, RARRES3, IFNA1, IFNA2, IFNA4, IFNA5, IFNA6, IFNA7, IFNA8, IFNA10, IFNA13, IFNA14, IFNA16, IFNA17, IFNA21, IFNK, IFNB1, IL6, TICAM1, TICAM2, MAYS, STAT1, STAT2, EIF2AK2, IRF3, TBK1, CDKN1A, CDKN2A, RNASEL, IFNAR1, IFNAR2, OAS1, OAS2, OAS3, OASL, RB1, ISG15, ISG20, IFIT1, IFIT2, IFIT3, and IFIT5, or a biologically active fragment, analog, or variant thereof.

[A6] The method according to any one of [A1] to [A5], in which the subject or the cell has the A-allele of SNP rs10774671.

[A7] The method according to any one of [A1] to [A6], in which the innate immune response is an innate immune response to infection with a virus, for example, an RNA virus, for example, a coronavirus, for example, SARS-CoV, MARS-CoV, or SARS-CoV-2.

[A8] The method according to any one of [A3] to [A7], in which the compound having the ability to inhibit or activate a protein kinase CLK (CDC-like kinase) has an ability to inhibit at least one kinase belonging to the CLK family, has activity of inhibiting the phosphorylation activity of at least one of the kinases belonging to the CLK family, or has an ability to inhibit at least one selected from the group consisting of CLK1, CLK2, CLK3, and CLK4.

[A9] The method according to any one of [A3] to [A8], in which the compound having the ability to inhibit or activate a protein kinase CLK (CDC-like kinase) is a compound represented by Formulae (II) to (VIII) below, a prodrug thereof, or a pharmaceutically acceptable salt thereof,

[Chemical Formula 24]

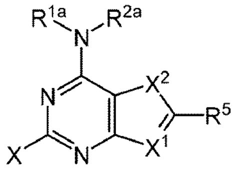 (II) 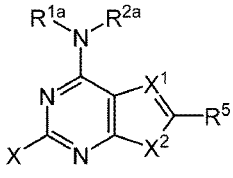 (II') 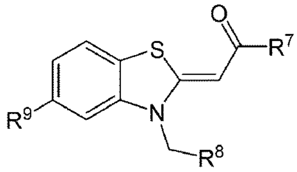 (III)

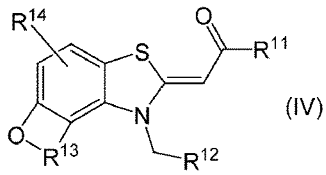 (IV) 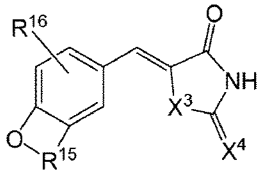 (V)

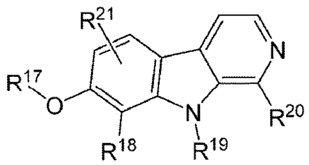 (VI) 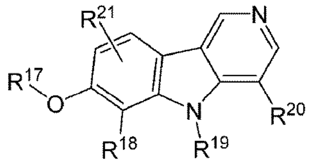 (VII) 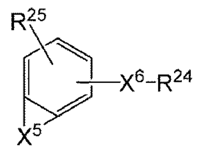 (VIII)

where, in Formulae (II) and (II'),

$R^{1a}$ and $R^{2a}$ each independently represent a hydrogen atom, a substituted or unsubstituted $C_1$-$C_6$ alkyl group, a substituted or unsubstituted benzyl group, a substituted or unsubstituted heteroarylmethyl group, a substituted or unsubstituted heteroarylethyl group, a substituted or unsubstituted aryloxy group, a substituted or unsubstituted heteroaryloxy group, a substituted or unsubstituted alkoxyamidoalkyl group, a substituted or unsubstituted aryl group, or a substituted or unsubstituted heteroaryl group, or alternatively, $R^{1a}$ and $R^{2a}$ bind to each other to form a ring together with N, and the ring is a substituted or unsubstituted monocyclic heterocyclic ring, or a substituted or unsubstituted bicyclic heterocyclic ring,

$R^5$ represents a hydrogen atom, a halogen atom, or a substituted or unsubstituted $C_1$-$C_6$ alkoxy group;

$X^1$ represents N or CH;

$X^2$ represents -N($R^3$)-, S, or O;

$R^3$ represents a hydrogen atom, a $C_1$-$C_6$ alkyl group, a benzyl group, a heteroarylmethyl group, a substituted or unsubstituted aryl group, a substituted or unsubstituted heteroaryl group, or $CH_2OC(O)R^4$-:

$R^4$ represents a $C_1$-$C_6$ alkyl group, a benzyl group, a heteroarylmethyl group, a substituted or unsubstituted aryl group, or a substituted or unsubstituted heteroaryl group; and

X represents a hydrogen atom, a halogen atom, an amino group, an $R^{1a-}$ and $R^{2a}$-substituted amino group, an azido group, a cyano group, a nitro group, a hydroxy group, a $C_1$-$C_6$ alkyloxy group, a substituted or unsubstituted aryloxy group, a substituted or unsubstituted heteroaryloxy group, a mercapto group, a $C_1$-$C_6$ alkylthio group, a substituted or unsubstituted arylthio group, a substituted or unsubstituted heteroarylthio group, a benzyl group, a heteroarylmethyl group, a substituted or unsubstituted aryl group, or a substituted or unsubstituted heteroaryl group;

in Formula (III),

$R^7$ and $R^8$ each independently represent a hydrogen atom, a halogen-substituted or unsubstituted $C_1$-$C_{10}$ alkyl group, or a $C_2$-$C_6$ alkenyl group;

$R^9$ represents a hydrogen atom, a halogen atom, or a halogen-substituted or unsubstituted $C_1$-$C_{10}$ alkyl group, -$OR^{10}$, -$NHR^{10}$, or -$N(R^{10})_2$; and

$R^{10}$ represents a hydrogen atom or a $C_1$-$C_{10}$ alkyl group;

in Formula (IV),

$R^{11}$ and $R^{12}$ each independently represent a hydrogen atom or a $C_1$-$C_6$ alkyl group;

$R^{13}$ represents

[Chemical Formula 25]

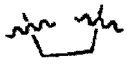, 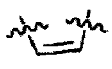, or 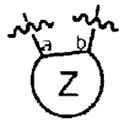

where Z forms, together with atoms marked with a and b, a ring selected from the group consisting of one benzene ring, one heteroaromatic ring, an aromatic ring fused with one or more benzene rings, a heteroaromatic ring fused with one or more heteroaromatic rings, a mixed fused polycyclic ring in which one or more benzene rings and one or more heteroaromatic rings are fused, and cycloaliphatic groups, and the ring may include one or more substituents, the substituents being a hydrogen atom, a halogen atom, or a $C_1$-$C_6$ alkyl group; and $R^{14}$ represents a hydrogen atom, a halogen atom, or a $C_1$-$C_6$ alkyl group; in Formula (V),
$X^3$ and $X^4$ each independently represent S or NH;
$R^{15}$ represents

[Chemical Formula 26]

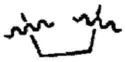, 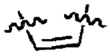, or 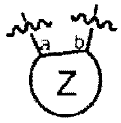

where Z forms, together with atoms marked with a and b, a ring selected from the group consisting of one benzene ring, one heteroaromatic ring, an aromatic ring fused with one or more benzene rings, a heteroaromatic ring fused with one or more heteroaromatic rings, a mixed fused polycyclic ring in which one or more benzene rings and one or more heteroaromatic rings are fused, and cycloaliphatic groups, and the ring may include one or more substituents, the substituents being a hydrogen atom, a halogen atom, or a $C_1$-$C_6$ alkyl group; and $R^{16}$ represents a hydrogen atom, a halogen atom, or a $C_1$-$C_6$ alkyl group; in Formulae (VI) and (VII),
$R^{17}$ and $R^{19}$ each independently represent a hydrogen atom, a $C_1$-$C_6$ alkyl group, a benzyl group, a heteroarylmethyl group, a substituted or unsubstituted aryl group, or a substituted or unsubstituted heteroaryl group;
$R^{18}$ represents $-R^{22}$, $-C{\equiv}C-R^{22}$, $-CH=CH-R^{22}$, or $-O-(CH_2)n-R^{22}$, n is 1 to 6, $R^{22}$ represents a hydrogen atom, a hydroxy group, a $C_1$-$C_8$ alkyl group, $-Si(R^{23})_3$, a substituted or unsubstituted phenyl group, a monocyclic heteroaromatic group, or a cycloaliphatic group, or alternatively, $R^{17}$ and $R^{18}$ bind to each other to form a ring, and $-R^{17}-R^{18}-$ is substituted by $-(CH_2)m-CH_2-$, $-CH=CH-$, $-(CH_2)_m-O-$, or a halogen atom, m is 1 to 6, $R^{23}$ represents a hydrogen atom, a $C_1$-$C_6$ alkyl group, a trihalomethyl group, or a hydroxy group, and three $R^{23}$ of $-Si(R^{23})_3$ may be different from each other; and
$R^{20}$ and $R^{21}$ represent a hydrogen atom or a $C_1$-$C_6$ alkyl group;

in Formula (VIII),

$X^5$ represents

[Chemical Formula 27]

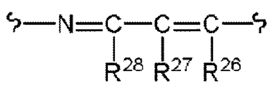, 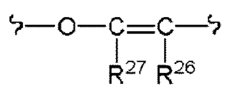, or 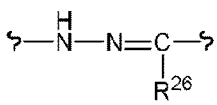

where $R^{26}$, $R^{27}$, and $R^{28}$ each independently represent a hydrogen atom, a halogen atom, a carboxyl group, an amino group, a hydroxy group, a $C_1$-$C_4$ alkyl group, or a halogen-substituted $C_1$-$C_4$ alkyl group;
$X^6$ represents -(atomic bonding) or -NH-;
$R^{24}$ represents

[Chemical Formula 28]

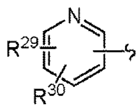, 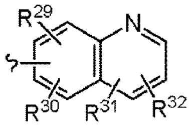, 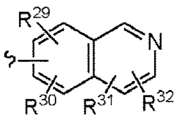, 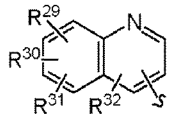, or 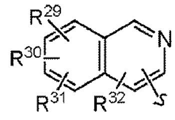

where $R^{29}$, $R^{30}$, $R^{31}$, and $R^{32}$ each independently represent a hydrogen atom, a halogen atom, a carboxyl group, an amino group, a hydroxy group, a $C_1$-$C_4$ alkyl group, or a halogen-substituted $C_1$-$C_4$ alkyl group; and $R^{25}$ represents a hydrogen atom, a halogen atom, a carboxyl group, an amino group, a hydroxy group, or a halogen-substituted or unsubstituted $C_1$-$C_4$ alkyl group.

[B 1] A composition for enhancing an innate immune response of a subject or a cell,

the subject or the cell being a subject or a cell having an innate immune response that a gene to be subjected to splicing participates in,
the composition containing a compound that affects alternative splicing as an active ingredient.

[B2] The composition according to [B 1], in which the subject or the cell is a subject or a cell having an innate immune response attenuated by alternative splicing of a gene for the innate immune response.
[B3] The composition according to [B 1] or [B2], in which the compound is a compound having an ability to inhibit or activate a protein kinase CLK (CDC-like kinase).
[B4] The composition according to [B 1] to [B3], in which the subject or the cell has a mutation or polymorphism (may include an SNP) that causes alternative splicing that attenuates the innate immune response in a gene on an innate immune response pathway.
[B5] The composition according to any one of [B1] to [B4], in which the innate immune response is an innate immune response realized by a pathway selected from the group consisting of TP53, TLR3, TLR7, RARRES3, IFNA1, IFNA2, IFNA4, IFNA5, IFNA6, IFNA7, IFNA8, IFNA10, IFNA13, IFNA14, IFNA16, IFNA17, IFNA21, IFNK, IFNB1, IL6, TICAM1, TICAM2, MAYS, STAT1, STAT2, EIF2AK2, IRF3, TBK1, CDKN1A, CDKN2A, RNASEL, IFNAR1, IFNAR2, OAS1, OAS2, OAS3, OASL, RB1, ISG15, ISG20, IFIT1, IFIT2, IFIT3, and IFIT5, or a biologically active fragment, analog, or variant thereof.
[B6] The composition according to any one of [B1] to [B5], in which the subject or the cell has the A-allele of SNP rs10774671.
[B7] The composition according to any one of [B1] to [B6], in which the innate immune response is an innate immune response to infection with a virus, for example, an RNA virus, for example, a coronavirus, for example, SARS-CoV, MARS-CoV, or SARS-CoV-2.
[B8] The composition according to any one of [B3] to [B7], in which the compound having the ability to inhibit or activate a protein kinase CLK (CDC-like kinase) has an ability to inhibit at least one kinase belonging to the CLK family, has activity of inhibiting the phosphorylation activity of at least one of the kinases belonging to the CLK family, or has an ability to inhibit at least one selected from the group consisting of CLK1, CLK2, CLK3, and CLK4.
[B9] The composition according to any one of [B3] to [B8], in which the compound having the ability to inhibit or activate a protein kinase CLK (CDC-like kinase) is a compound represented by Formulae (II) to (VIII) below, a prodrug thereof, or a pharmaceutically acceptable salt thereof,

[Chemical Formula 29]

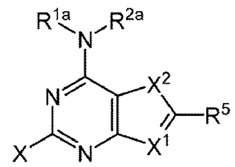 (II)

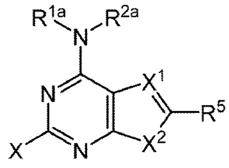 (II')

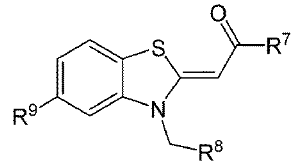 (III)

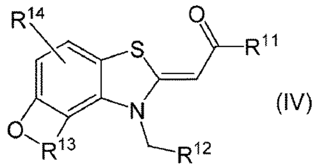 (IV)

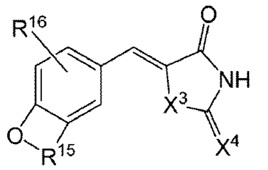 (V)

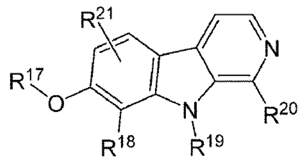 (VI)

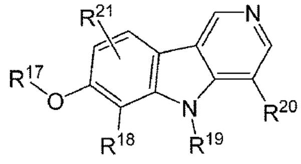 (VII)

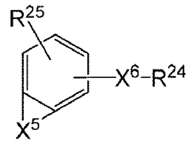 (VIII)

where, in Formulae (II) and (II'),

$R^{1a}$ and $R^{2a}$ each independently represent a hydrogen atom, a substituted or unsubstituted $C_1$-$C_6$ alkyl group, a substituted or unsubstituted benzyl group, a substituted or unsubstituted heteroarylmethyl group, a substituted or unsubstituted heteroarylethyl group, a substituted or unsubstituted aryloxy group, a substituted or unsubstituted heteroaryloxy group, a substituted or unsubstituted alkoxyamidoalkyl group, a substituted or unsubstituted aryl group, or a substituted or unsubstituted heteroaryl group, or alternatively, $R^{1a}$ and $R^{2a}$ bind to each other to form a ring together with N, and the ring is a substituted or unsubstituted monocyclic heterocyclic ring, or a substituted or unsubstituted bicyclic heterocyclic ring,

$R^5$ represents a hydrogen atom, a halogen atom, or a substituted or unsubstituted $C_1$-$C_6$ alkoxy group;

$X^1$ represents N or CH;

$X^2$ represents -N($R^3$)-, S, or O;

$R^3$ represents a hydrogen atom, a $C_1$-$C_6$ alkyl group, a benzyl group, a heteroarylmethyl group, a substituted or unsubstituted aryl group, a substituted or unsubstituted heteroaryl group, or $CH_2OC(O)R^4$-;

$R^4$ represents a $C_1$-$C_6$ alkyl group, a benzyl group, a heteroarylmethyl group, a substituted or unsubstituted aryl group, or a substituted or unsubstituted heteroaryl group; and

X represents a hydrogen atom, a halogen atom, an amino group, an $R^{1a}$- and $R^{2a}$-substituted amino group, an azido group, a cyano group, a nitro group, a hydroxy group, a $C_1$-$C_6$ alkyloxy group, a substituted or unsubstituted aryloxy group, a substituted or unsubstituted heteroaryloxy group, a mercapto group, a $C_1$-$C_6$ alkylthio group, a substituted or unsubstituted arylthio group, a substituted or unsubstituted heteroarylthio group, a benzyl group, a heteroarylmethyl group, a substituted or unsubstituted aryl group, or a substituted or unsubstituted heteroaryl group;

in Formula (III),

$R^7$ and $R^8$ each independently represent a hydrogen atom, a halogen-substituted or unsubstituted $C_1$-$C_{10}$ alkyl group, or a $C_2$-$C_6$ alkenyl group;

$R^9$ represents a hydrogen atom, a halogen atom, or a halogen-substituted or unsubstituted $C_1$-$C_{10}$ alkyl group, -$OR^{10}$, -$NHR^{10}$, or -N($R^{10}$)$_2$; and

$R^{10}$ represents a hydrogen atom or a $C_1$-$C_{10}$ alkyl group;

in Formula (IV),

$R^{11}$ and $R^{12}$ each independently represent a hydrogen atom or a $C_1$-$C_6$ alkyl group;

$R^{13}$ represents

[Chemical Formula 30]

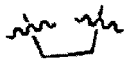, 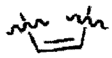, or 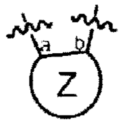

where Z forms, together with atoms marked with a and b, a ring selected from the group consisting of one benzene ring, one heteroaromatic ring, an aromatic ring fused with one or more benzene rings, a heteroaromatic ring fused with one or more heteroaromatic rings, a mixed fused polycyclic ring in which one or more benzene rings and one or more heteroaromatic rings are fused, and cycloaliphatic groups, and the ring may include one or more substituents, the substituents being a hydrogen atom, a halogen atom, or a $C_1$-$C_6$ alkyl group; and $R^{14}$ represents a hydrogen atom, a halogen atom, or a $C_1$-$C_6$ alkyl group;

in Formula (V),

$X^3$ and $X^4$ each independently represent S or NH;
$R^{15}$ represents

[Chemical Formula 31]

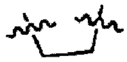, 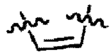, or 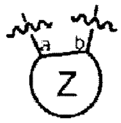

where Z forms, together with atoms marked with a and b, a ring selected from the group consisting of one benzene ring, one heteroaromatic ring, an aromatic ring fused with one or more benzene rings, a heteroaromatic ring fused with one or more heteroaromatic rings, a mixed fused polycyclic ring in which one or more benzene rings and one or more heteroaromatic rings are fused, and cycloaliphatic groups, and the ring may include one or more substituents, the substituents being a hydrogen atom, a halogen atom, or a $C_1$-$C_6$ alkyl group; and $R^{16}$ represents a hydrogen atom, a halogen atom, or a $C_1$-$C_6$ alkyl group; in Formulae (VI) and (VII),
$R^{17}$ and $R^{19}$ each independently represent a hydrogen atom, a $C_1$-$C_6$ alkyl group, a benzyl group, a heteroarylmethyl group, a substituted or unsubstituted aryl group, or a substituted or unsubstituted heteroaryl group;
$R^{18}$ represents $-R^{22}$, $-C \equiv C-R^{22}$, $-CH=CH-R^{22}$, or $-O-(CH_2)n-R^{22}$, n is 1 to 6, $R^{22}$ represents a hydrogen atom, a hydroxy group, a $C_1$-$C_8$ alkyl group, $-Si(R^{23})_3$, a substituted or unsubstituted phenyl group, a monocyclic heteroaromatic group, or a cycloaliphatic group, or alternatively, $R^{17}$ and $R^{18}$ bind to each other to form a ring, and $-R^{17}-R^{18}-$ is substituted by $-(CH_2)m-CH_2-$, $-CH=CH-$, $-(CH_2)_m-O-$, or a halogen atom, m is 1 to 6, $R^{23}$ represents a hydrogen atom, a $C_1$-$C_6$ alkyl group, a trihalomethyl group, or a hydroxy group, and three $R^{23}$ of $-Si(R^{23})_3$ may be different from each other; and
$R^{20}$ and $R^{21}$ represent a hydrogen atom or a $C_1$-$C_6$ alkyl group;

in Formula (VIII),

$X^5$ represents

[Chemical Formula 32]

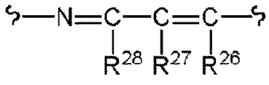, 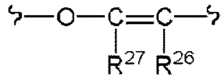, or 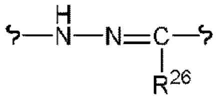

where $R^{26}$, $R^{27}$, and $R^{28}$ each independently represent a hydrogen atom, a halogen atom, a carboxyl group, an amino group, a hydroxy group, a $C_1$-$C_4$ alkyl group, or a halogen-substituted $C_1$-$C_4$ alkyl group;
$X^6$ represents -(atomic bonding) or -NH-;

$R^{24}$ represents

[Chemical Formula 33]

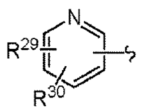, 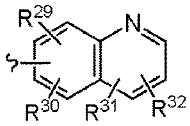, 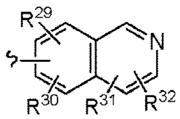, 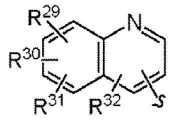, or 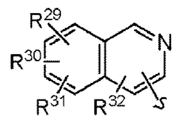

where $R^{29}$, $R^{30}$, $R^{31}$, and $R^{32}$ each independently represent a hydrogen atom, a halogen atom, a carboxyl group, an amino group, a hydroxy group, a $C_1$-$C_4$ alkyl group, or a halogen-substituted $C_1$-$C_4$ alkyl group; and $R^{25}$ represents a hydrogen atom, a halogen atom, a carboxyl group, an amino group, a hydroxy group, or a halogen-substituted or unsubstituted $C_1$-$C_4$ alkyl group.

[B10] The composition according to any one of [B 1] to [B9], in which the composition is a pharmaceutical composition.
[C 1] A method for preventing a viral infectious disease, alleviating a symptom, or treating a viral infectious disease, the method including:

(1) confirming whether at least one condition selected from the group consisting of

a condition in which a subject has an innate immune response that a gene to be subjected to splicing participates in,
a condition in which the subject has an innate immune response attenuated by alternative splicing of a gene for the innate immune response,
a condition in which the subject has a mutation or polymorphism (including an SNP) that causes alternative splicing that attenuates the innate immune response in a gene on an innate immune response pathway, and
a condition in which the subject has the A-allele of SNP rs10774671, is satisfied, and

(2) administering a compound that affects alternative splicing to the subject who satisfies the condition in the (1) above, or bringing a compound that affects alternative splicing into contact with the subject.

[C2] The method according to [C 1], in which the compound is a compound having an ability to inhibit or activate a protein kinase CLK (CDC-like kinase).
[C3] The method according to [C1] or [C2], in which the innate immune response is an innate immune response realized by a pathway selected from the group consisting of TP53, TLR3, TLR7, RARRES3, IFNA1, IFNA2, IFNA4, IFNA5, IFNA6, IFNA7, IFNA8, IFNA10, IFNA13, IFNA14, IFNA16, IFNA17, IFNA21, IFNK, IFNB1, IL6, TICAM1, TICAM2, MAYS, STAT1, STAT2, EIF2AK2, IRF3, TBK1, CDKN1A, CDKN2A, RNASEL, IFNAR1, IFNAR2, OAS1, OAS2, OAS3, OASL, RB1, ISG15, ISG20, IFIT1, IFIT2, IFIT3, and IFIT5, or a biologically active fragment, analog, or variant thereof.
[C4] The method according to any one of [C1] to [C3], in which the virus is an RNA virus, for example, a coronavirus, for example, SAR,S-CoV, MARS-CoV, or SARS-CoV-2.
[C5] The method according to any one of [C2] to [C4], in which the compound having the ability to inhibit or activate a protein kinase CLK (CDC-like kinase) has an ability to inhibit at least one kinase belonging to the CLK family, has activity of inhibiting the phosphorylation activity of at least one of the kinases belonging to the CLK family, or has an ability to inhibit at least one selected from the group consisting of CLK1, CLK2, CLK3, and CLK4.
[C6] The method according to any one of [C2] to [C5], in which the compound having the ability to inhibit or activate a protein kinase CLK (CDC-like kinase) is a compound represented by Formulae (II) to (VIII) below, a prodrug thereof, or a pharmaceutically acceptable salt thereof,

[Chemical Formula 34]

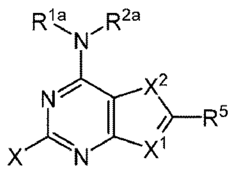 (II) 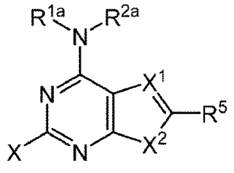 (II') 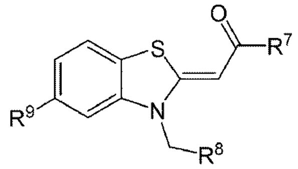 (III)

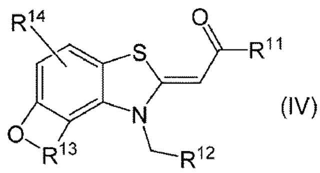 (IV) 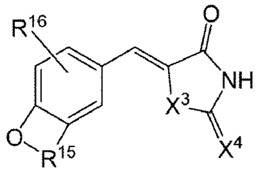 (V)

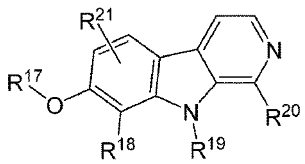 (VI) 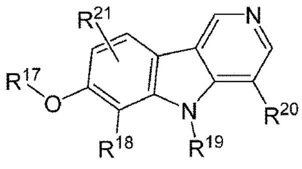 (VII) 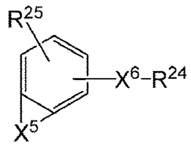 (VIII)

where, in Formulae (II) and (II'),

$R^{1a}$ and $R^{2a}$ each independently represent a hydrogen atom, a substituted or unsubstituted $C_1$-$C_6$ alkyl group, a substituted or unsubstituted benzyl group, a substituted or unsubstituted heteroarylmethyl group, a substituted or unsubstituted heteroarylethyl group, a substituted or unsubstituted aryloxy group, a substituted or unsubstituted heteroaryloxy group, a substituted or unsubstituted alkoxyamidoalkyl group, a substituted or unsubstituted aryl group, or a substituted or unsubstituted heteroaryl group, or alternatively, $R^{1a}$ and $R^{2a}$ bind to each other to form a ring together with N, and the ring is a substituted or unsubstituted monocyclic heterocyclic ring, or a substituted or unsubstituted bicyclic heterocyclic ring,
$R^5$ represents a hydrogen atom, a halogen atom, or a substituted or unsubstituted $C_1$-$C_6$ alkoxy group;
$X^1$ represents N or CH;
$X^2$ represents -N($R^3$)-, S, or O;
$R^3$ represents a hydrogen atom, a $C_1$-$C_6$ alkyl group, a benzyl group, a heteroarylmethyl group, a substituted or unsubstituted aryl group, a substituted or unsubstituted heteroaryl group, or $CH_2OC(O)R^4$-;
$R^4$ represents a $C_1$-$C_6$ alkyl group, a benzyl group, a heteroarylmethyl group, a substituted or unsubstituted aryl group, or a substituted or unsubstituted heteroaryl group; and
X represents a hydrogen atom, a halogen atom, an amino group, an $R^{1a-}$ and $R^{2a}$-substituted amino group, an azido group, a cyano group, a nitro group, a hydroxy group, a $C_1$-$C_6$ alkyloxy group, a substituted or unsubstituted aryloxy group, a substituted or unsubstituted heteroaryloxy group, a mercapto group, a $C_1$-$C_6$ alkylthio group, a substituted or unsubstituted arylthio group, a substituted or unsubstituted heteroarylthio group, a benzyl group, a heteroarylmethyl group, a substituted or unsubstituted aryl group, or a substituted or unsubstituted heteroaryl group;

in Formula (III),

$R^7$ and $R^8$ each independently represent a hydrogen atom, a halogen-substituted or unsubstituted $C_1$-$C_{10}$ alkyl group, or a $C_2$-$C_6$ alkenyl group;
$R^9$ represents a hydrogen atom, a halogen atom, or a halogen-substituted or unsubstituted $C_1$-$C_{10}$ alkyl group, -$OR^{10}$, -$NHR^{10}$, or -$N(R^{10})_2$; and
$R^{10}$ represents a hydrogen atom or a $C_1$-$C_{10}$ alkyl group;

in Formula (IV),

$R^{11}$ and $R^{12}$ each independently represent a hydrogen atom or a $C_1$-$C_6$ alkyl group;
$R^{13}$ represents

[Chemical Formula 35]

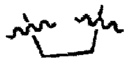, 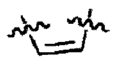, or 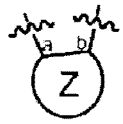

where Z forms, together with atoms marked with a and b, a ring selected from the group consisting of one benzene ring, one heteroaromatic ring, an aromatic ring fused with one or more benzene rings, a heteroaromatic ring fused with one or more heteroaromatic rings, a mixed fused polycyclic ring in which one or more benzene rings and one or more heteroaromatic rings are fused, and cycloaliphatic groups, and the ring may include one or more substituents, the substituents being a hydrogen atom, a halogen atom, or a $C_1$-$C_6$ alkyl group; and $R^{14}$ represents a hydrogen atom, a halogen atom, or a $C_1$-$C_6$ alkyl group;

in Formula (V),

$X^3$ and $X^4$ each independently represent S or NH;
$R^{15}$ represents

[Chemical Formula 36]

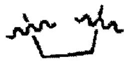, 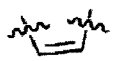, or 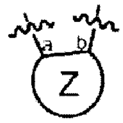

where Z forms, together with atoms marked with a and b, a ring selected from the group consisting of one benzene ring, one heteroaromatic ring, an aromatic ring fused with one or more benzene rings, a heteroaromatic ring fused with one or more heteroaromatic rings, a mixed fused polycyclic ring in which one or more benzene rings and one or more heteroaromatic rings are fused, and cycloaliphatic groups, and the ring may include one or more substituents, the substituents being a hydrogen atom, a halogen atom, or a $C_1$-$C_6$ alkyl group; and $R^{16}$ represents a hydrogen atom, a halogen atom, or a $C_1$-$C_6$ alkyl group;

in Formulae (VI) and (VII),

$R^{17}$ and $R^{19}$ each independently represent a hydrogen atom, a $C_1$-$C_6$ alkyl group, a benzyl group, a heteroarylmethyl group, a substituted or unsubstituted aryl group, or a substituted or unsubstituted heteroaryl group;
$R^{18}$ represents $-R^{22}$, $-C \equiv C-R^{22}$, $-CH=CH-R^{22}$, or $-O-(CH_2)n-R^{22}$, n is 1 to 6, $R^{22}$ represents a hydrogen atom, a hydroxy group, a $C_1$-$C_8$ alkyl group, $-Si(R^{23})_3$, a substituted or unsubstituted phenyl group, a monocyclic heteroaromatic group, or a cycloaliphatic group, or alternatively, $R^{17}$ and $R^{18}$ bind to each other to form a ring, and $-R^{17}-R^{18}-$ is substituted by $-(CH_2)m-CH_2-$, $-CH=CH-$, $-(CH_2)_m-O-$, or a halogen atom, m is 1 to 6, $R^{23}$ represents a hydrogen atom, a $C_1$-$C_6$ alkyl group, a trihalomethyl group, or a hydroxy group, and three $R^{23}$ of $-Si(R^{23})_3$ may be different from each other; and
$R^{20}$ and $R^{21}$ represent a hydrogen atom or a $C_1$-$C_6$ alkyl group;

in Formula (VIII),

$X^5$ represents

[Chemical Formula 37]

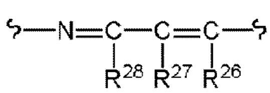, 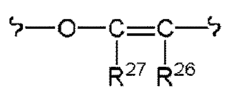, or 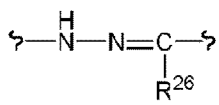

where $R^{26}$, $R^{27}$, and $R^{28}$ each independently represent a hydrogen atom, a halogen atom, a carboxyl group, an amino group, a hydroxy group, a $C_1$-$C_4$ alkyl group, or a halogen-substituted $C_1$-$C_4$ alkyl group;
$X^6$ represents -(atomic bonding) or -NH-;
$R^{24}$ represents

[Chemical Formula 38]

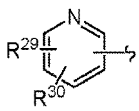,
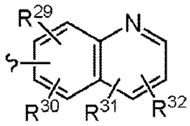,
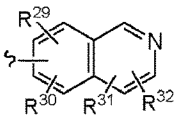,
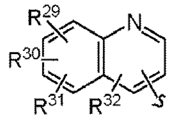, or
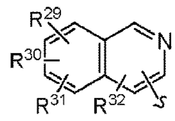

where $R^{29}$, $R^{30}$, $R^{31}$, and $R^{32}$ each independently represent a hydrogen atom, a halogen atom, a carboxyl group, an amino group, a hydroxy group, a $C_1$-$C_4$ alkyl group, or a halogen-substituted $C_1$-$C_4$ alkyl group; and $R^{25}$ represents a hydrogen atom, a halogen atom, a carboxyl group, an amino group, a hydroxy group, or a halogen-substituted or unsubstituted $C_1$-$C_4$ alkyl group.

[D1] Use of a compound that affects alternative splicing, for producing a composition that enhances an innate immune response of a subject or a cell having an innate immune response that a gene to be subjected to splicing participates in.
[D2] The use according to [D1], in which the subject or the cell is a subject or a cell having an innate immune response attenuated by alternative splicing of a gene for the innate immune response.
[D3] The use according to [D1] or [D2], in which the compound is a compound having an ability to inhibit or activate a protein kinase CLK (CDC-like kinase).
[D4] The use according to any one of [D1] to [D3], in which the subject or the cell has a mutation or polymorphism (including an SNP) that causes alternative splicing that attenuates the innate immune response in a gene on an innate immune response pathway.
[D5] The use according to any one of [D1] to [D4], in which the innate immune response is an innate immune response realized by a pathway selected from the group consisting of TP53, TLR3, TLR7, RARRES3, IFNA1, IFNA2, IFNA4, IFNA5, IFNA6, IFNA7, IFNA8, IFNA10, IFNA13, IFNA14, IFNA16, IFNA17, IFNA21, IFNK, IFNB1, IL6, TICAM1, TICAM2, MAYS, STAT1, STAT2, EIF2AK2, IRF3, TBK1, CDKN1A, CDKN2A, RNASEL, IFNAR1, IFNAR2, OAS1, OAS2, OAS3, OASL, RB1, ISG15, ISG20, IFIT1, IFIT2, IFIT3, and IFIT5, or a biologically active fragment, analog, or variant thereof.
[D6] The use according to any one of [D1] to [D5], in which the subject or the cell has the A-allele of SNP rs10774671.
[D7] The use according to any one of [D1] to [D6], in which the innate immune response is an innate immune response to infection with a virus, for example, an RNA virus, for example, a coronavirus, for example, SARS-CoV, MARS-CoV, or SARS-CoV-2.
[D8] The use according to any one of [D3] to [D7], in which the compound having the ability to inhibit or activate a protein kinase CLK (CDC-like kinase) has an ability to inhibit at least one kinase belonging to the CLK family, has activity of inhibiting the phosphorylation activity of at least one of the kinases belonging to the CLK family, or has an ability to inhibit at least one selected from the group consisting of CLK1, CLK2, CLK3, and CLK4.
[D9] The use according to any one of [D3] to [D8], in which the compound having the ability to inhibit or activate a protein kinase CLK (CDC-like kinase) is a compound represented by Formulae (II) to (VIII) below, a prodrug thereof, or a pharmaceutically acceptable salt thereof,

[Chemical Formula 39]

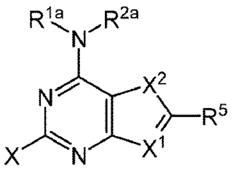 (II) 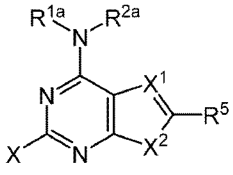 (II') 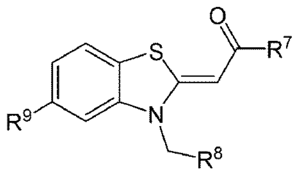 (III)

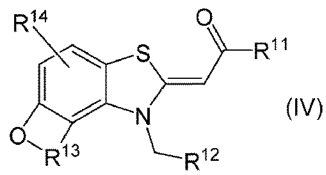 (IV) 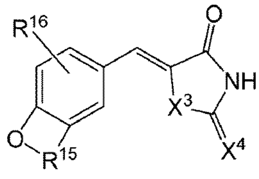 (V)

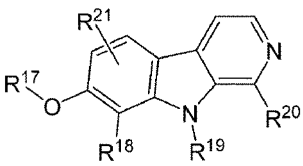 (VI) 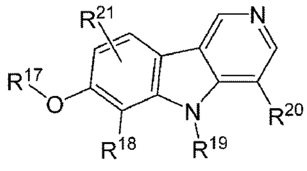 (VII) 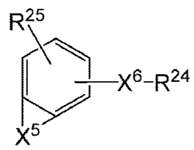 (VIII)

where, in Formulae (II) and (II'),

$R^{1a}$ and $R^{2a}$ each independently represent a hydrogen atom, a substituted or unsubstituted $C_1$-$C_6$ alkyl group, a substituted or unsubstituted benzyl group, a substituted or unsubstituted heteroarylmethyl group, a substituted or unsubstituted heteroarylethyl group, a substituted or unsubstituted aryloxy group, a substituted or unsubstituted heteroaryloxy group, a substituted or unsubstituted alkoxyamidoalkyl group, a substituted or unsubstituted aryl group, or a substituted or unsubstituted heteroaryl group, or alternatively, $R^{1a}$ and $R^{2a}$ bind to each other to form a ring together with N, and the ring is a substituted or unsubstituted monocyclic heterocyclic ring, or a substituted or unsubstituted bicyclic heterocyclic ring,
$R^5$ represents a hydrogen atom, a halogen atom, or a substituted or unsubstituted $C_1$-$C_6$ alkoxy group;
$X^1$ represents N or CH;
$X^2$ represents -N($R^3$)-, S, or O;
$R^3$ represents a hydrogen atom, a $C_1$-$C_6$ alkyl group, a benzyl group, a heteroarylmethyl group, a substituted or unsubstituted aryl group, a substituted or unsubstituted heteroaryl group, or $CH_2OC(O)R^4$-;
$R^4$ represents a $C_1$-$C_6$ alkyl group, a benzyl group, a heteroarylmethyl group, a substituted or unsubstituted aryl group, or a substituted or unsubstituted heteroaryl group; and
X represents a hydrogen atom, a halogen atom, an amino group, an $R^{1a-}$ and $R^{2a}$-substituted amino group, an azido group, a cyano group, a nitro group, a hydroxy group, a $C_1$-$C_6$ alkyloxy group, a substituted or unsubstituted aryloxy group, a substituted or unsubstituted heteroaryloxy group, a mercapto group, a $C_1$-$C_6$ alkylthio group, a substituted or unsubstituted arylthio group, a substituted or unsubstituted heteroarylthio group, a benzyl group, a heteroarylmethyl group, a substituted or unsubstituted aryl group, or a substituted or unsubstituted heteroaryl group;

in Formula (III),

$R^7$ and $R^8$ each independently represent a hydrogen atom, a halogen-substituted or unsubstituted $C_1$-$C_{10}$ alkyl group, or a $C_2$-$C_6$ alkenyl group;
$R^9$ represents a hydrogen atom, a halogen atom, or a halogen-substituted or unsubstituted $C_1$-$C_{10}$ alkyl group, -$OR^{10}$, -$NHR^{10}$, or -$N(R^{10})_2$; and
$R^{10}$ represents a hydrogen atom or a $C_1$-$C_{10}$ alkyl group;

in Formula (IV),

$R^{11}$ and $R^{12}$ each independently represent a hydrogen atom or a $C_1$-$C_6$ alkyl group;
$R^{13}$ represents

[Chemical Formula 40]

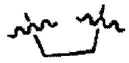 , 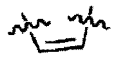 , or 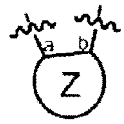

where Z forms, together with atoms marked with a and b, a ring selected from the group consisting of one benzene ring, one heteroaromatic ring, an aromatic ring fused with one or more benzene rings, a heteroaromatic ring fused with one or more heteroaromatic rings, a mixed fused polycyclic ring in which one or more benzene rings and one or more heteroaromatic rings are fused, and cycloaliphatic groups, and the ring may include one or more substituents, the substituents being a hydrogen atom, a halogen atom, or a $C_1$-$C_6$ alkyl group; and $R^{14}$ represents a hydrogen atom, a halogen atom, or a $C_1$-$C_6$ alkyl group; in Formula (V),
$X^3$ and $X^4$ each independently represent S or NH;
$R^{15}$ represents

[Chemical Formula 41]

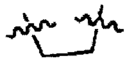 , 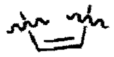 , or 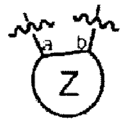

where Z forms, together with atoms marked with a and b, a ring selected from the group consisting of one benzene ring, one heteroaromatic ring, an aromatic ring fused with one or more benzene rings, a heteroaromatic ring fused with one or more heteroaromatic rings, a mixed fused polycyclic ring in which one or more benzene rings and one or more heteroaromatic rings are fused, and cycloaliphatic groups, and the ring may include one or more substituents, the substituents being a hydrogen atom, a halogen atom, or a $C_1$-$C_6$ alkyl group; and $R^{16}$ represents a hydrogen atom, a halogen atom, or a $C_1$-$C_6$ alkyl group; in Formulae (VI) and (VII),
$R^{17}$ and $R^{19}$ each independently represent a hydrogen atom, a $C_1$-$C_6$ alkyl group, a benzyl group, a heteroarylmethyl group, a substituted or unsubstituted aryl group, or a substituted or unsubstituted heteroaryl group;
$R^{18}$ represents $-R^{22}$, $-C{\equiv}C-R^{22}$, $-CH=CH-R^{22}$, or $-O-(CH_2)n-R^{22}$, n is 1 to 6, $R^{22}$ represents a hydrogen atom, a hydroxy group, a $C_1$-$C_8$ alkyl group, $-Si(R^{23})_3$, a substituted or unsubstituted phenyl group, a monocyclic heteroaromatic group, or a cycloaliphatic group, or alternatively, $R^{17}$ and $R^{18}$ bind to each other to form a ring, and $-R^{17}-R^{18}-$ is substituted by $-(CH_2)m-CH_2-$, $-CH=CH-$, $-(CH_2)_m-O-$, or a halogen atom, m is 1 to 6, $R^{23}$ represents a hydrogen atom, a $C_1$-$C_6$ alkyl group, a trihalomethyl group, or a hydroxy group, and three $R^{23}$ of $-Si(R^{23})_3$ may be different from each other; and
$R^{20}$ and $R^{21}$ represent a hydrogen atom or a $C_1$-$C_6$ alkyl group;

in Formula (VIII),

$X^5$ represents

[Chemical Formula 42]

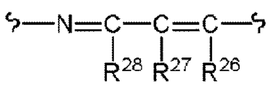 , 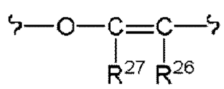 , or 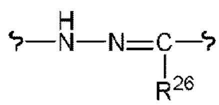

where $R^{26}$, $R^{27}$, and $R^{28}$ each independently represent a hydrogen atom, a halogen atom, a carboxyl group, an amino group, a hydroxy group, a $C_1$-$C_4$ alkyl group, or a halogen-substituted $C_1$-$C_4$ alkyl group;
$X^6$ represents -(atomic bonding) or -NH-;
$R^{24}$ represents

[Chemical Formula 43]

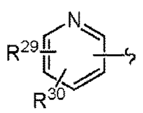, 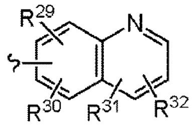, 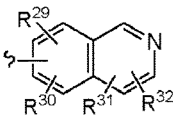, 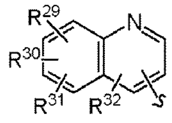, or 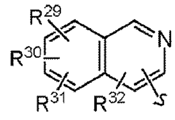

where $R^{29}$, $R^{30}$, $R^{31}$, and $R^{32}$ each independently represent a hydrogen atom, a halogen atom, a carboxyl group, an amino group, a hydroxy group, a $C_1$-$C_4$ alkyl group, or a halogen-substituted $C_1$-$C_4$ alkyl group; and $R^{25}$ represents a hydrogen atom, a halogen atom, a carboxyl group, an amino group, a hydroxy group, or a halogen-substituted or unsubstituted $C_1$-$C_4$ alkyl group.

[D10] The use according to any one of [D1] to [D9], in which the composition is a pharmaceutical composition.

Hereinafter, although the present disclosure will be described in more detail by way of examples, these are illustrative, and the present disclosure is not limited to these examples. Note that all of the references cited in the present disclosure is incorporated as a portion of the present disclosure.

Brief Description of Drawings

[FIG. 1] OAS1-3 loci have COVID-19 associated SNPs and respond to SARS-CoV-2. Positions and GWAS p-values for SNPs on OAS1, OAS3, and OAS2 gene loci are shown. (a) GWAS results for confirmed COVID-19 cases with very severe respiratory conditions, (b) GWAS results for hospitalized COVID-19 patients, and FIG. 1(c) shows GWAS results for all COVID-19 patients, reported by COVID-19 Host Genome Initiative groups (Reported by COVID-19 Host Genome Initiative groups). Red and blue horizontal lines show the p-value positions of 5E-8 and 1E-5, respectively. The position of rs10774671 SNP is shown with a green dotted line and red arrows.

[FIG. 2] CLK inhibitor compound 1 induces splice-switching in OAS1 rs10774671 A allele. a. Allele-dependent OAS1 alternative splicing. Pre-mRNAof OAS1, with the rs10774671 G-allele at -1 position of exon 6 splice acceptor (SA), dominantly produces the p46 variant, whereas the A-allele leads to alternative splicing to produce the p42, p48, p44a, and p44b variants with differences in the last exon (yellow boxes). b. The SRSF6 binding motif (UGCUUC) (red letters) is present in close vicinity of the U1 binding site of OAS1 donor site for p44A/p44B/p48 splicing. Blue dots indicate UlsnRNA pairing. c. Western blots of Ul-70k and phosphorylated SRSF6 (p-SRSF6) for RNA pull-down products of OAS1 exon 5 splice donor (SD) sequence in Calu-3 cells treated with 0.1% DMSO or 10 μM compound 1. Input indicates input samples; bait (-) indicates negative control products without the bait RNA oligo. ACTB served as the loading control. d. RT-PCR for an OAS1 alternative splicing profile in Daudi (G/G allele) and Calu-3 (A/A allele) cells treated with 0.1% DMSO or 10 μM compound 1 for 24 h. ACTB served as the loading control. e. RNA-Seq results indicated by a Sashimi plot for a region covering p42, p44a, and p46. f. Numbers of splice-junction reads for OAS1 splice variants. Dots indicate the number of read repeats; error bars represent ±SD. and n.s. when $p \geq 0.05$; * represents $p < 0.05$; ** represents $p< 0.01$ by Student's t-test.

[FIG. 3] Pre-treatment with the compound 1 confers Calu-3 cells with resistance against SARS-CoV-2 infection via activation of the RNase L and interferon pathways. a. A scheme for the pre-treatment of the compound 1 and viral titer assay. b Box plots showing logarithm translated viral titers. The values obtained by correcting batch effects are shown (see methods and Table 4). c. Bar-plot for cleaved RNA products and 28S rRNA measured with the BioAnalyzer system for the RNA samples from Calu-3 cells, before and after the viral infection for 24 h, with and without compound 1 treatments. d. RT-PCR results for IFNB1 mRNA. e. Bar-plot for quantified IFNB1/ACTB in the infected cells based on RT-PCR results. Error bars represent ±SD; ** represents $p < 0.01$; by Student's t-test.

[FIG. 4] Genotyping of rs10774671 SNP. FIG. 4a shows results of the SNP genotyping for Calu-3 cells and FIG. 4b shows results of the SNP genotyping for Daudi cells.

[FIG. 5] Pathway analyses for compound 1-affected genes. Pathway analyses for 98 differentially up-regulated genes (a) and 63 differentially alternatively spliced genes (b) in compound 1-treated Calu-3 cells. For the differentially alternatively spliced genes, the splicing events that promote productive forms were used (see the following for details). These analyses were performed with the Metascape web server (Tripathi et al.).

[FIG. 6] RNA degradation in SARS-CoV-2-infected cells. (a) A gel image obtained based on the results from the Bioanalyzer. (b) Line graphs for fluorescence units according to migration times. Solid lines show the mean values, and ribbons show the range of standard errors.

[FIG. 7] Allele frequency of the rs10774671 SNP. A world map shows the relationship between the location and allele frequency of the SNP. The position in the hg19 genome is shown. The map was obtained from the Geography of Genetic Variants (GGV) browser (Marcus and Novembre, 2017).

[FIG. 8] RT-PCR forOAS1 alternative splicing profiles in Calu-3 (A/A allele) cells treated for 18 hours with 0.1% DMSO, or 10 pM compound 1, 10 pM compound 2, or 10 pM compound 3. β actin (ACTB) was used as a loading control.

[FIG. 9] RT-PCR for OAS 1 alternative splicing profiles in Calu-3 (A/A allele) cells treated for 18 hours with 0.1% DMSO, 1 pM compound 1, 3 pM compound 1, or 10 pM compound 1, or 10 pM compound 4 or 30 pM compound 4.

Examples

[Synthesis of compound]

Compound 1: (Z)-2-amino-5-[(2,3-dihydrobenzofuran-5-yl)methylene]thiazol-4(5H)-one

[Chemical Formula 44]

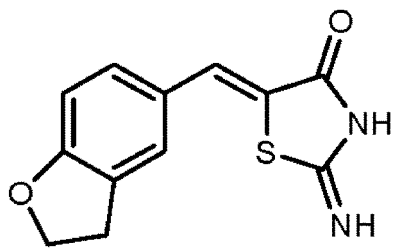

The compound 1 was synthesized according to the method disclosed in WO2014/021337. The CLK1 inhibitory activity (IC50 value), the CLK2 inhibitory activity (IC50 value), and the CLK3 inhibitory activity (IC50 value) of the compound 1 were respectively 21.6 nM, 68.3 nM, and 116 nM.

Compound 2: 5-pyiridin-3-yl-1H-indazole

[Chemical Formula 45]

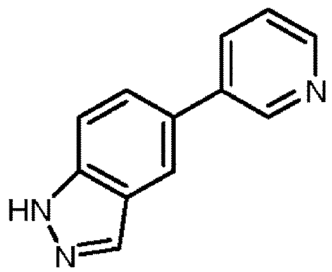

The compound 2 was synthesized according to the method disclosed in WO2018/043674. The CLK1 inhibitory activity, the CLK2 inhibitory activity, and the CLK3 inhibitory activity of the compound 2 at 1 pM were respectively 98.4%, 90.5%, and 21.6%.

Compound 3: 1-(1-ethylfuro[3,2-e][1,3]benzothiazol-2-ylidene)propan-2-one

[Chemical Formula 46]

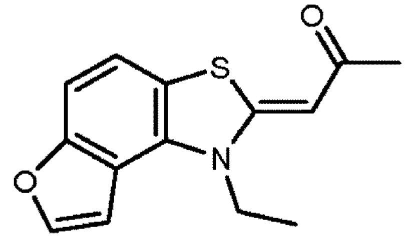

The compound 3 was synthesized according to the method disclosed in WO2015/083750. The CLK1 inhibitory activity, the CLK2 inhibitory activity, and the CLK3 inhibitory activity of the compound 3 at 1 pM were respectively 101.8%, 96.5%, and 32.9%.

Compound 4: 1-(3-ethyl-5-methoxy-1,3-benzothiazol-2-ylidene)propan-2-one

[Chemical Formula 47]

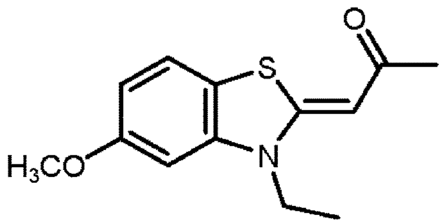

The compound 4 was synthesized according to the method disclosed in WO2010/010797. The compound 4 is known as a Cdc2-like kinase (CLK) inhibitor (CAS 300801-52-9). The CLK1 inhibitory activity (IC50 value), the CLK2 inhibitory activity (IC50 value), and the CLK3 inhibitory activity (IC50 value) of the compound 4 were respectively 26.2 nM, 309 nM, and >3,000 nM.

[Inhibition confirmation test for CLK]

Enzyme activity was measured with off-chip mobility shift assay using purified CLK protein. DYRKtide-F peptide (1 pM) was used as a substrate, and a composition of a reaction solution used was as follows (20 mM HEPES (pH7.5), 0.01% Triton X-100, 1 mM DTT, 1% DMSO, andATP (10 pM for CLK1, 150 pM for CLK2, and 75 pM for CLK3)). The enzyme reaction was performed at room temperature for 1 hour, and the reaction was terminated by adding QuickScout Screening Assist MSA (Carna Biosciences, Inc.). A substrate peptide and a phosphorylated peptide in the enzyme reaction solution were separated and quantified using the LabChip system (Perkin-Elmer, Waltham, MA, USA), and inhibition of CLK was evaluated by calculating a product ratio using the following formula, the substrate peptide peak height (S), and the phosphorylated peptide peak height (P).

$$(P/(P+S))$$

P: product-derived peak
S: substrate-derived peak

[Example 1]

[Materials and methods]

Analysis of GWAS data

GWAS results were obtained from the COVID-19 Host Genetics Initiative data. The following terms were used:

"A2_ALL_leave_23andme" for GWAS of confirmed COVID patients with very severe respiratory conditions vs. population, "B2_ALL_leave_23andme" for GWAS of hospitalized COVID patients vs. population, and "C2_ALL_leave_23andme" for GWAS of all COVID patients vs. population. Data version round 5 was used for all of the data obtained in this study (January 18, 2021).

The sample size of 5582, 12888, and 36590 individuals were chosen for evaluation of GWAS datasets of severe respiratory symptoms, hospitalization, and COVID-19 susceptibility, respectively, obtained from COVID-19 host genome initiative database (The COVID-19 Host Genetics Initiative, 2021). This sample number was determined based on the number of applicable data without exclusion, and a sample-size estimation was not conducted prior to the analysis.

[Cell lines]

Daudi cells, which are derived from Burkitt's lymphoma, were obtained from the cell bank of the National Institutes of Biomedical Innovation, Health and Nutrition (NIBIOHN, Osaka, Japan) and maintained in RPMI 1640 medium (Nacalai Tesque, Kyoto, Japan) supplemented with 20% fetal bovine serum, 100 U/mL penicillin, and 100 μg/mL streptomycin. Calu-3 cells, which are derived from lung adenocarcinoma, were obtained from American Type Culture Collection (ATCC, Manassas, VA, USA), and cultured in Dulbecco's modified Eagle's medium (DMEM) (Nacalai Tesque) supplemented with 10 % fetal bovine serum, 100 U/mL penicillin, and 100 μg/mL streptomycin. All cells were maintained in an incubator at 37°C with 5 % $CO_2$, and mycoplasma was confirmed to be negative in routine polymerase chain reaction tests. VeroE6/TMPRSS2 cells were obtained from JCRB Cell Bank, and cultured in Dulbecco's modified Eagle's medium (DMEM) (Sigma-Aldrich) supplemented with 10 % fetal bovine serum, 100 U/mL penicillin, and 100 μg/mL streptomycin. The cells were maintained in an incubator at 37°C with 5 % $CO_2$.

RNApull-down assay and western blot

Calu-3 whole cell lysates were prepared using a lysis buffer containing 10 mM Tris-HCl (pH 7.4), 150 mM NaCl, 1 mM ethylenediaminetetraacetic acid, 1% Triton X-100, 0.1% sodium dodecyl sulphate, 0.25% sodium deoxycholate, and 10% glycerol with protease inhibitors (Nacalai Tesque) and phosphatase inhibitors (Sigma-Aldrich, Munich, Germany), followed by sonication, treatment with DNase I (Promega, Madison, WI, USA) at 37°C for 5 min, and centrifugation (24,000×g at 4°C for 15 min). The supernatant was used as the soluble fraction for the RNA-pull down assay. For the RNA pull-down assay, 5'-biotin, 3'-dTdT-attached RNA, designed for the sequence adjacent to the OAS1 exon 5 splice donor (5'-CUGCUGGUGAGACCUCCUGCUCC-3' (SEQ ID NO: 1) (oAM685), was incubated with NeutrAvidin (trademark) beads (Thermo Fisher Scientific, Waltham, MA, USA) for 2 hours at 4°C (no bait RNA was used for the negative control), followed by wash with 1X TBS thrice. RNA-bound NeutrAvidin beads were then incubated with the Calu-3 cell lysate in the presence of 1% DMSO or 10 pM compound 1 for 16 hours with rotation at 4°C. This was followed by washing the resultant thrice with tris-buffered saline and elution with Laemmli buffer. Three technical replicates for RNA pull-down assay were independently conducted, three times from a stocked cell lysate. Eluted proteins were then analyzed by western blotting with anti-Ul-70k mouse monoclonal antibody (9C4.1) (05-1588, Merk Millipore, Burlington, MA, USA) at a dilution of 1:500 for the detection of U1-70k, anti-SR protein (1H4G7) mouse monoclonal antibody (33-9400, Thermo Fisher Scientific, Waltham, MA, USA) at a dilution of 1:200 for phosphorylated SRSF6, and anti-β-actin (ACTB) mouse monoclonal antibody (Ac-15) (sc-69879, Santa Cruz Biotechnology, Dallas, TX, USA) at a dilution of 1:4,000 for ACTB. Chemiluminescent signals were detected using a ChemiDoc MP Imaging System (Bio-Rad, Hercules, CA, USA).

Transcriptome analysis for compound 1-treated Calu-3 cells

RNAs were extracted using RNeasy Mini kit (QIAGEN, Hilden, Germany) from Calu-3 cells, treated with 10 μM compound 1 or 0.1% DMSO for 18 h, and applied for RNA-Seq analysis. Three technical replicates for RNA-Seq, where each experiment was independently conducted three times from a stocked total RNA. RNA-seq reads were mapped to the human genome sequences (GRCh38) using STAR (ver. 2.7.1a, https://github.com/alexdobin/STAR) with ENCODE options, using the Ensembl genome annotation (ver. 102). Raw reads were counted with bam files, and TPM values were calculated using RSEM v1.2.31 (https://github.com/deweylab/RSEM). Differentially expressed genes were identified using the method described above. Differential alternative splicing (DAS) events were analyzed with the method previously described (Sakuma et al., 2015) using the rMATS program (http://maseq-mats.sourceforge.net/rmats4.1.1/). DAS was defined by the following criteria: FDR < 0.01, read counts ≥ 15, and delta Percent Spliced-In (PSI) ≥ 0.05. The DAS events were compared with the gene annotation information, the event types were then classified into events on the exons constituting the productive mRNAs, events on additional exons/regions of the productive mRNAs, and others. For characterizing gene set and transcriptome profiles, the Metascape website (https://metascape.org/) and Gene Set Enrichment Analysis (GSEA, https://www.gsea-msigdb.org/gseal) were used. The same samples were also used for RT-PCR.

SARS-CoV-2 infection

Calu-3 cells were pre-treated with 10 pM compound 1 or 0.1% DMSO for 24 hours, infected with SARS-CoV-2 (SARS-CoV-2/Hu/DP/Kng/19-027) at a multiplicity of infection of 0.01, and maintained in the presence of the 10 pM compound 1 or 0.1% DMSO. RNA samples were collected from the cells 24 hours post-infection (pi), and the viral titers were determined by the 50% tissue culture infectious dose ($TCID_{50}$) using VeroE6/TMPRSS2 cells at 48 hours pi. Titer assay was conducted in six biological replicate experiments for independent cell cultures.

Analysis of RNA degradation

RNA samples were diluted to 200 ng/ pL. The quality of the diluted RNA samples was evaluated using the Agilent RNA 6000 Nano Kit and the Agilent 2100 Bioanalyzer. For the infected cells, three biological replicates with independent cell cultures were prepared for DMSO treatment and compound 1 treatment, respectively, with the method described above. Total RNA from transcriptome analysis represented uninfected control for this study. The gel-like image of the 2100 Bioanalyzer result was visualized using the 2100 Expert Software (ver. B.02.11, Agilent Technologies) in pseudo colors with default settings. For detailed analyses, the migration time and fluorescence unit data were extracted in CSV format to be aligned with the sample data. Then, the fluorescence unit values were normalized to an RNA concentration of 300 ng/μL. The data located between 43.3 s and 48.0 s of the migration time was selected as cleaved RNA products and the data located between 49.9 s and 51.4 s was selected as the 28S rRNA The sums of the fluorescence unit values were used for further analysis. For drawing the bar-plot, the values were scaled to the mean values of DMSO samples.

RT-PCR

Total RNA extracted from cultured cells was used for reverse transcription using the PrimeScript RTase (Takara Bio, Shiga, Japan) with random hexamers, and the products were then amplified with ExTaq DNA polymerase (Takara Bio) with target-specific primer sets. Primers used in RTPCR are listed in Table 2. Detection of RTPCR products was conducted using the ChemiDoc MP Imaging System (Bio-Rad), with subsequent analysis by Image Lab software (Bio-Rad). RT-PCR was conducted in three technical replicates for a total RNA sample.

[Table 2]

| (Table 2) Primer name orientation Target gene Target isoform Sequence (5' -to-3' ) | | | | | |
|---|---|---|---|---|---|
| oAM13 | Forward | ACTB | | CCAACCGCGAGAAGATGACC | SEQ ID NO: 2 |
| oAM14 | Reverse | ACTB | | AGCTTCTCCTTAATGTCACG | SEQ ID NO: 3 |
| oAM674 | Forward | OAS1 | OAS1 p42, OAS1 p44a, OAS1 p44b, OAS1 p46, OAS1 p48 | GGTTGGAGGCAGCTGGCAC | SEQ ID NO: 4 |
| oAM675 | Reverse | OAS1 | OAS1 p42 | CCAGCTATATGTCTCAAGCT | SEQ ID NO: 5 |
| oAM676 | Reverse | OAS1 | OAS1 p46, OAS1 p48 | CTGGCATTCAGAGGATGGTG | SEQ ID NO: 6 |
| oAM677 | Reverse | OAS1 | OAS1 p44b | CTGCCTCCCTAAGCAACCTG | SEQ ID NO: 7 |
| oAM678 | Reverse | OAS1 | OAS1 p44a | CCATTTCCACCACTTGTTAG | SEQ ID NO: 8 |
| oAM700 | Forward | IFNB1 | | CAGTGTCAGAAGCTCCTGTG | SEQ ID NO: 9 |
| oAM701 | Reverse | IFNB1 | | ATCTGATGATAGACATTAGC | SEQ ID NO: 10 |

Modelling of viral infection

The statistical simulation of SARS-CoV-2 infections was performed with the SIR (Susceptible, Infectious, or Recovered) model (Harko et al., 2014) using the "deSolve" package (Soetaert et al., 2010) in the R environment. The initial number of susceptible persons was assigned as 9999 and the initial number of infectious persons was assigned as 1. Beta ($\beta$), which is a parameter for infection rate per day, per person, was set as 0.5 or 0.28 (i.e., $0.5 \times 0.56$), and Gamma ($\gamma$), which is a parameter for the removal or the recovery rate per day, per person, was set as 0.1, according to a report on the spread of SARS-CoV-2 in April 2020, in the USA (Adam, 2020).

Data availability

The datasets generated during and/or analyzed during the current study are available from the corresponding author on reasonable request. The original RNA-seq data were deposited at the Gene Expression Omnibus (GEO) of National Center for Biotechnology Information (NCBI) with the accession ID GSE174398.

[Results]

OAS1 splice variant regulation by SRSF6

The rs10774671 A/G SNP is known to control the production of OAS1 splice variants. The G allele of rs10774671 (G-allele) creates the AG-dinucleotide that is essential for the recognition of the exon 6 splice acceptor site for p46 variant production. The Aallele of rs10774671 (A-allele) leads to the alternative splicing of OAS1 pre-mRNA to produce p42, p48, p44a, and p44b variants (Noguchi et al., 2013) (FIG. 2a). OAS1 protein senses the double-stranded RNA structure, including RNA duplication intermediates of SAR,S-CoV2 (Sadler and Williams, 2008; Schlee and Hartmann, 2016), to synthesize 2'-5'-oligoadenylates, which in turn trigger the activation of latent ribonuclease L (RNase L) for viral RNA degradation (Sadler and Williams, 2008; Schlee and Hartmann, 2016). Previous studies revealed that the catalytic activity of OAS1 varies with its splice variants; p46, which is the major isoform of OAS1 produced in the presence of the G-allele, presents optimal activity, while p42, which is the major isoform produced in the presence of the A-allele, shows poor activity (Carey et al., 2019; Di et al., 2020).

In A-allele individuals, disruption of the exon 6 splice acceptor site induces OAS1 alternative splicing, yielding p44a, p44b, and p48 through activation of acceptor sites downstream of the exon 5, and p42 as a consequence of the skipped recognition of exon 5 donor site (FIG. 2a). Assuming that the splice-shift from the inactive p42 to other variants may improve the immune-response that is weakened due to the presence of OAS1 A-allele, the mechanism by which p42 variant is dominantly produced was examined. In searching for splice motifs surrounding the OAS1 exon 5 donor sites with the ESEfinder tool (Cartegni et al., 2003), a binding motif for serine/arginine-rich splicing factor 6 (SRSF6) (5'-UGCUUC-3') immediately downstream to the exon 5 donor site was found (FIG. 2b). In view of this, it was inferred that SRSF6 interaction with this site could prevent UlsnRNP from binding to the exon 5 donor site. To test this hypothesis, a compound 1, which is a pan-CDC-like kinase (CLK) inhibitor, was used to dissociate SRSF6 from RNAby preventing the kinase activity of CLKs to phosphorylate RS domain at the carboxyl-terminal of SRSF6 (Shibata et al., 2020). As a result of an RNApull-down assay, it was found that the U1snRNP binding to exon 5 donor site was enhanced when Calu-3 cells were treated with the compound 1, as represented by the detection of the U1-70k major subunit in the pull-down products, whereas phosphorylated SRSF6 was not detected (OAS1SD pull-down products in FIG. 2c). These data indicate that SRSF6 plays a major role in the exon 5 donor site recognition, which is crucial for OAS1 alternative splicing.

The compound 1 induces splice shift in A-allele.

Next, the consequence of CLK inhibition on alternative splicing of OAS 1 mRNAhaving A-allele was investigated. Calu-3 cells, which were lung adenocarcinoma cells homozygous for OAS1 A-allele (FIG. 4a), were treated with 10 $\mu$M compound 1 or DMSO and analyzed for OAS1 splicing profiles, along with Daudi cells, which are lymphoma cells homozygous for the G-allele (FIG. 4b). Consistent with previous observations (Kjaer et al., 2014; Noguchi et al., 2013), it was found that the p46 variant was dominantly expressed from OAS1 G-allele, while the p42 variant was expressed from the A-allele (FIG. 2d). Intriguingly, CLK inhibition induced splice-switching for OAS1 with the A-allele, by suppressing p42 splicing while promoting p44a and p44b variant production. This observation is consistent with the model suggested by the RNA pull-down assay (FIG. 2c), where the exon 5 donor site is made available for UlsnRNP binding upon CLK inhibition (FIG. 2b). However, the p48 variant expression was under the detection limit in both A- and G-alleles (FIG. 2d). The splicing shift of OAS1 with A-allele upon CLK inhibition was also evident in RNA-Seq analysis of Calu-3 cells, in which the p42 variant was suppressed by 51%, and p44a and p44b were increased by 130% and 100%, respectively; the p44b variant was found to be a minor form of OAS1, accounting for 1% to 4% of OAS1 transcripts (FIGS. 2e and 2f). Additionally, through the RNA-Seq analysis of Calu-3 cells with or without compound 1 treatment, differential expression of 98 genes, and splice alterations yielding protein-coding variants for 63 genes were identified; however, none

of these events were associated with viral infection except for the OAS1 splice alterations (Table 3, and FIG. 5).

[Table 3-1]

(Table 3) Altered splicing events altering protein-coding isoforms in compound 1-treated Calu-3 cells.

| Gene Symbol | delta PSI | Transcript ID | Exon number | Event type | Direction of Alternative splicing | Exon type | Size of alternatively spliced region |
|---|---|---|---|---|---|---|---|
| WNK1 | 0.517 | ENST00000 315939 | E11 | 1.SE.Event_on_Productive_form | Inclution | CDS | 459 |
| RPGR | 0.448 | ENST00000 339363 | E14 | 1.SE.Event_on_Productive_form | Inclution | CDS | 948 |
| NAV2 | 0.437 | ENST00000 396087 | E28 | 1.SE.Event_on_Productive_form | Inclution | CDS | 9 |
| CENPE | 0.422 | ENST00000 265148 | E17 | 1.SE.Event_on_Productive_form | Inclution | CDS | 75 |
| PI4KB | 0.421 | ENST00000 368875 | E2 | 1.SE.Event_on_Productive_form | Inclution | CDS_wIniCodon | 202 |
| LIMCH1 | 0.42 | ENST00000 313860 | E9 | 1.SE.Event_on_Productive_form | Inclution | CDS | 36 |
| MYO6 | 0.405 | ENST00000 369981 | E34 | 1.SE.Event_on_Productive_form | Inclution | CDS | 27 |
| MAP3K7 | 0.404 | ENST00000 369329 | E12 | 1.SE.Event_on_Productive_form | Inclution | CDS | 81 |
| LRP8 | 0.392 | ENST00000 306052 | E5 | 1.SE.Event_on_Productive_form | Inclution | CDS | 387 |
| LRRC6 | 0.391 | ENST00000 620350 | E5 | 1.SE.Event_on_Productive_form | Inclution | CDS | 224 |
| HERC4 | 0.38 | ENST00000 395198 | E17 | 1.SE.Event_on_Productive_form | Inclution | CDS | 24 |
| PDE9A | 0.378 | ENST00000 291539 | E5 | 1.SE.Event_on_Productive_form | Inclution | CDS | 180 |
| TRIM2 | 0.372 | ENST00000 674976 | E2 | 1.SE.Event_on_Productive_form | Inclution | 5UTR | 797 |
| CLK4 | 0.37 | ENST00000 316308 | E4 | 1.SE.Event_on_Productive_form | Inclution | CDS | 91 |

(continued)

(Table 3) Altered splicing events altering protein-coding isoforms in compound 1-treated Calu-3 cells.

| Gene Symbol | delta PSI | Transcript ID | Exon number | Event type | Direction of Alternative splicing | Exon type | Size of alternatively spliced region |
|---|---|---|---|---|---|---|---|
| OBSL1 | 0.369 | ENST00000 404537 | E12 | 1.SE.Event_ on_Producti ve_form | Inclution | CDS | 276 |
| SMARCA1 | 0.367 | ENST00000 371122 | E13 | 1.SE.Event_ on_Producti ve_form | Inclution | CDS | 36 |
| MCTP1 | 0.367 | ENST00000 515393 | E8 | 1.SE.Event_ on_Producti ve_form | Inclution | CDS | 78 |
| NEK1 | 0.367 | ENST00000 439128 | E17 | 1.SE.Event_ on_Producti ve_form | Inclution | CDS | 132 |
| DMD | 0.365 | ENST00000 357033 | E71 | 1.SE.Event_ on_Producti ve_form | Inclution | CDS | 39 |
| SAMD9L | 0.364 | ENST00000 318238 | Partial_ Intron _onE3 | 1.RI.Intron_ on_Exon_of_ Productive_ form | Inclution | 5UTR | 378 |
| RTN4 | 0.361 | ENST00000 337526 | E3 | 1.SE.Event_ on_Producti ve_form | Inclution | CDS | 2400 |
| MON2 | 0.345 | ENST00000 393630 | E28 | 1.SE.Event_ on_Producti ve_form | Inclution | CDS | 18 |
| MYOM1 | 0.344 | ENST00000 356443 | E18 | 1.SE.Event_ on_Producti ve_form | Inclution | CDS | 288 |
| DNM1 | 0.342 | ENST00000 372923 | E14 | 1.SE.Event_ on_Producti ve_form | Inclution | CDS | 12 |
| SNX14 | 0.336 | ENST00000 314673 | E16 | 1.SE.Event_ on_Producti ve_form | Inclution | CDS | 27 |
| CLK1 | 0.331 | ENST00000 434813 | E4 | 1.SE.Event_ on_Producti ve_form | Inclution | CDS | 91 |
| AL163636.1 | 0.331 | ENST00000 553909 | E2 | 1.SE.Event_ on_Producti ve_form | Inclution | CDS_ wIniCodon | 104 |
| GK | 0.322 | ENST00000 427190 | E9 | 1.SE.Event_ on_Producti ve_form | Inclution | CDS | 18 |

(continued)

| (Table 3) Altered splicing events altering protein-coding isoforms in compound 1-treated Calu-3 cells. | | | | | | | |
|---|---|---|---|---|---|---|---|
| **Gene Symbol** | **delta PSI** | **Transcript ID** | **Exon number** | **Event type** | **Direction of Alternative splicing** | **Exon type** | **Size of alternatively spliced region** |
| MPP3 | 0.32 | ENST00000 398393 | E11 | 1.SE.Event_ on_Producti ve_form | Inclution | CDS | 21 |
| AL117339.4 | 0.318 | ENST00000 640275 | E2 | 1.SE.Event_ on_Producti ve_form | Inclution | 5UTR | 294 |
| PPM1K | 0.317 | ENST00000 608933 | E2 | 1.SE.Event_ on_Producti ve_form | Inclution | CDS_ wInicodon | 499 |

[Table 3-2]

| | | | | | | | |
|---|---|---|---|---|---|---|---|
| ZNF519 | 0.316 | ENST00000 590202 | E3 | 1.A3SS.Shortened_ 3SS from_ Productive_form | Inclution | CDS_ wTerCodon | 4636 |
| CDK5RAP2 | 0.313 | ENST00000 349780 | E32 | 1.SE.Event_on_ Producti ve_form | Inclution | CDS | 237 |
| CABYR | 0.309 | ENST00000 621648 | E4 | 1.A5SS.Shortened_ 5SS from_ Productive_form | Inclution | CDS_ wTerCodon | 957 |
| HERC3 | 0.309 | ENST00000 402738 | E17 | 1.SE.Event_on_ Producti ve_form | Inclution | CDS | 24 |
| KTN1 | 0.304 | ENST00000 459737 | E41 | 1.SE.Event_on_ Producti ve_form | Inclution | CDS | 84 |
| RBPMS | 0.301 | ENST00000 320203 | E8 | 1.SE.Event_on_ Producti ve_form | Inclution | 3UTR | 206 |
| METTL26 | 0.3 | ENST00000 301686 | E2 | 1.SE.Event_on_ Producti ve_form | Inclution | CDS | 163 |
| PNKP | -0.3 | ENST00000 322344 | Intron_ betwe en_ E14-E16 | 2.RI.Intron_of_ Productiv e_form | Exclution | CDS-CDS | 263 |
| EPB41 | -0.301 | ENST00000 343067 | E16-E17 | 2.SE.Additional_ Exon_to Productive_ form | Exclution | CDS-CDS | 450 |
| NGDN | -0.302 | ENST00000 408901 | Intron_ betwe en_ E4-E7 | 2.RI.Intron_of_ Productiv e_form | Exclution | CDS-CDS | 722 |
| AAMP | -0.307 | ENST00000 248450 | Intron_ betwe en_ E5-E7 | 2.RI.Intron_of_ Productiv e_form | Exclution | CDS-CDS | 498 |
| CYLD | -0.307 | ENST00000 427738 | E2-E3 | 2.SE.Additional_ Exon_to Productive_ form | Exclution | 5UTR- CDS_ wIniCodon | 137 |

(continued)

| | | | | | | | |
|---|---|---|---|---|---|---|---|
| KANSL3 | -0.309 | ENST00000 666923 | E3 | 2.A3SS.Extended_ 3SS_f rom_ Productive_form | Exclution | CDS | 61 |
| DNHD1 | -0.31 | ENST00000 254579 | Intron_ betwe en_ E42-E43 | 2.RI.Intron_of_ Productiv e_form | Exclution | CDS-CDS_ wTerCodon | ! 214 |
| SLC25A29 | -0.314 | ENST00000 359232 | E2-E3 | 2.SE.Additional_ Exon_to Productive_ form | Exclution | CDS-CDS | 72 |
| FAAP100 | -0.318 | ENST00000 327787 | E2 | 2.A5SS.Extended_ 5SS_f rom_ Productive_form | Exclution | CDS | 196 |
| PCGF3 | -0.318 | ENST00000 362003 | E3-E4 | 2.SE.Additional_ Exon_to Productive_ form | Exclution | 5UTR- CDS_ wIniCodon | 99 |
| SPATA20 | -0.319 | ENST00000 356488 | E1-E2 | 2.SE.Additional_ Exon_to Productive_ form | Exclution | CDS_ wIniCodon -CDS | 103 |
| TRMU | -0.333 | ENST00000 645190 | Intron_ betwe en_ E9-E11 | 2.RI.Intron_of_ Productiv e_form | Exclution | CDS-CDS_ wTerCodon | 1255 |
| AC107871. 1 | -0.333 | ENST00000 637054 | E2-E3 | 2.SE.Additional_ Exon_to Productive_ form | Exclution | CDS-CDS_ wTerCodon | 141 |
| LRRC75B | -0.338 | ENST00000 318753 | E1-E2 | 2.SE.Additional_ Exon_to Productive_ form | Exclution | CDS_ wIniCodon -CDS | 207 |
| CLK4 | -0.346 | ENST00000 316308 | Intron_ betwe en_ E3-E4 | 2.RI.Intron_of_ Productiv e_form | Exclution | CDS-CDS | 1123 |
| GALT | -0.374 | ENST00000 378842 | Intron_ betwe en_ E2-E6 | 2.RI.Intron_of_ Productiv e_form | Exclution | CDS-CDS | 858 |
| IMPDH2 | -0.376 | ENST00000 326739 | Intron_ betwe en_ E3-E5 | 2.RI.Intron_of_ Productiv e_form | Exclution | CDS-CDS | 516 |
| RPL3 | -0.381 | ENST00000 216146 | Intron_ betwe en_ E7-E9 | 2.RI.Intron_of_ Productiv e_form | Exclution | CDS-CDS | 798 |
| CLK4 | -0.401 | ENST00000 316308 | Intron_ betwe en_ E4-E5 | 2.RI.Intron_of_ Productiv e_form | Exclution | CDS-CDS | 397 |
| AL136531.2 | -0.418 | ENST00000 618770 | E2-E3 | 2.SE.Additional_ Exon_to Productive_ form | Exclution | - | 119 |
| DCUN1D2 | -0.438 | ENST00000 478244 | E4-E5 | 2.SE.Additional_ Exon_to Productive_ form | Exclution | CDS-CDS | 152 |

(continued)

| | | | | | | | |
|---|---|---|---|---|---|---|---|
| APOL1 | -0.448 | ENST00000 319136 | E3 | 2.A3SS.Extended_ 3SS_f rom_ Productive_form | Exclution | CDS | 39 |
| CFAP298 | -0.451 | ENST00000 290155 | Intron_ betwe en_ E5-E7 | 2.RI.Intron_of_ Productiv e_form | Exclution | CDS-CDS_ wTerCodon | 1189 |
| MSL3 | -0.485 | ENST00000 312196 | Intron_ betwe en_ E4-E6 | 2.RI.Intron_of_ Productiv e_form | Exclution | CDS-CDS | 1194 |
| HAT1 | -0.584 | ENST00000 264108 | Intron_ betwe en_ E6-E8 | 2.RI.Intron_of_ Productiv e_form | Exclution | CDS-CDS | 936 |
| RPL13A | -0.654 | ENST00000 391857 | Intron_ betwe en_ E2-E4 | 2.RI.Intron_of_ Productiv e_form | Exclution | CDS-CDS | 554 |
| DYNC1LI1 | -0.778 | ENST00000 273130 | Intron_ betwe en_ E8-E10 | 2.RI.Intron_of_ Productiv e_form | Exclution | CDS-CDS | 2659 |

SARS-CoV-2 resistance with splice shift.

The alternation of SARS-CoV-2 infection rate in Calu-3 cells, when the splicing isoform was changed from p42 to p44a by manipulating the splicing phenomenon with a small compound, was examined. To sufficiently change the balance of the splicing isoforms, cells were pre-treated with the compound 1 for 24 hours. Viral titer was assayed 2 days after cells were exposed to the virus (FIG. 3a). The viral titer decreased from $5.18 \times 10^7$ to $2.90 \times 10^7$ (fold change = 0.56) in the cells treated with the compound 1 (FIG. 3b, Table 4). There are no reports on the enzyme activity or antiviral activity of the p44a variant; however, based on this results, it can be presumed that the product encoded by p44a has stronger enzyme activity/antiviral ability than that encoded by the p42 form. Furthermore, RNA degradation products were measured to confirm whether this change was caused due to the switching of OAS1 splicing isoforms, and it was found that this change was caused by the switching of OAS 1 splicing isoforms. If enzyme activities of the product encoded by OAS 1 are elevated, it is expected that the activities of RNase L will be elevated. Activated RNase L can not only degrade the foreign RNA molecules, but also the endogenous ribosomal RNA within the host cells (Lin et al.). Thus, degradation of rRNA was investigated as a measure of RNase L activity. As a result, the appearance of a band that is expected to be a degradation product of rRNAin cells infected with SARS-CoV-2 was confirmed (FIG. 3c). It was also confirmed that the concentration of decomposition products, sized 2,000-3,000 nucleotides, increased slightly (fold change = 1.35), with a decrease in the molecular weight and concentration of the 28S rRNA peak (fold change = 0.56), upon the compound 1 treatment (FIG. 3c and FIG. 6). It is known that degraded dsRNA activates the transcription of the interferon genes through the OASL and RIG-I related pathways (Choi et al.). It was confirmed that the compound 1 treatment increases the transcription of interferon β1 (IFNB1) during viral infection (FIGS. 3d and 3e). This result was consistent with the previous reports showing IFNB1 transactivation during viral infections (Ford and Thanos, 2010; Schwanke et al., 2020).

[Table 4]

| (Table 4) Results of the virus titre assay (Raw and Batch-effect correcte d) | | | | |
|---|---|---|---|---|
| | **Treatment** | **No.** | **$log_{10}$ ($TCID_{50}$/ml)** | **Batch-effect corrected** |
| **1 st batch** | **Compund 1 0 μM** | 1 | 7.75 | - |
| | | 2 | 7.67 | - |
| | | 3 | 7.75 | - |
| | **Compund 1 10 μM** | 1 | 7.5 | - |
| | | 2 | 7.5 | - |
| | | 3 | 7.25 | - |
| **2nd batch** | **Compund 1 0 μM** | 1 | 6.75 | 7.6 |
| | | 2 | 7.25 | 7.92 |
| | | 3 | 6.5 | 7.44 |
| | **Compund 1 10 μM** | 1 | 6.75 | 7.6 |
| | | 2 | 6.5 | 7.44 |
| | | 3 | 6.45 | 7.41 |

These results indicate that compound 1 treatment enhances the infection-dependent RNase L pathway, and the type I interferon pathway induced by the degraded RNAs, via a switching of OAS1 splicing isoforms from p42 to p44a in Calu-3 cells (FIG. 3f).

[Discussion]

This study revealed that the difference in the balance of OAS1 splicing isoforms affects the infectivity of SARS-CoV-2 in cells via modulation of the innate immune response. This result strongly suggests that the OAS1 intervening sequence (IVS) 5-1 G> A SNP that alters the OAS1 splicing pattern is one of the important therapeutic targets for COVID-19. The applicants recently reported on the compound 1 as a potential therapeutic drug for cystic fibrosis caused by a point mutation in the CFTR gene (Shibata et al., 2020). In this study, it was found that the compound 1 treatment increased the yield of the p44a splice form (FIGS. 2d, 2e, and 2f) and made the Calu-3 cells more resistant to viral infection (FIG. 3b), suggesting that the compound 1 treatment could overcome A-allele-derived vulnerabilities to SARS-CoV-2 infections.

The G-allele of SNP rs10774671 produces the p46 splice variant, which possesses 56 amino acids at the C-terminus, compared to the 18 completely different amino acids present in the p42 splice variant produced by the A-allele. This changes the localization of the OAS1 protein and its interacting partners (Kjaer et al., 2014), which may be linked to the differential 2'-5'-oligoadenylate synthesis activities, RNase L activation, and interferon pathway activation. Several clinical trials that investigated the efficacy of an intervention of the interferon β pathway to affect the progression of COVID-19 have been conducted successfully (Hung et al., 2020; Monk et al., 2021; Shalhoub, 2020). On the other hand, inhibition of the interferon pathway is considered important to prevent cytokine storms in patients with severe COVID-19 (Nile et al., 2020). Since the OAS1 IVS5-1 SNP can alter the response intensity of the interferon β pathway, stratification of COVID-19 patients depending on this SNP may assist in choosing the best course of interferon treatment.

It is reported that protective SNPs at OAS 1/OAS3/OAS2 loci originated in the Neanderthals (Zeberg and Paabo, 2021; Zhou et al., 2021). Interestingly, the OAS1 IVS5-1 A-allele, which was confirmed to be associated with the aggravation of COVID-19 in this study, was not found in the ancient human genome, the sequenced Neanderthal genome, or the Denisovan genome (Mendez et al., 2013). This suggests that the G to A mutation at the OAS1 IVS5-1 position occurred at a relatively modern age in human history. The OAS1 IVS5-1 A-allele has expanded to become a major allele in the population, especially in the Asian region (FIG. 7) (Marcus and Novembre, 2017). This suggests the possibility that this variant has some positive effects on human survival, trading off the effect of weakening resistance against viruses.

The results obtained in this study indicate that the OAS 1 IVS5-1 SNP is involved in the exacerbation of COVID-19 by eliciting changes in the OAS 1 splicing isoform balance. Individuals with the A-allele may have a higher risk for SARS-CoV-2 infection and its aggravation than those with the G-allele. However, we successfully demonstrate a way to overcome these genetic risks by modulating the splicing phenomenon. Splicing modulation reduced the virus infection rate 0.56 times in cell-based assays (FIG. 3b). This theoretically implies that the peak number of SAR,S-CoV-2-infected individuals will be reduced by 42%, if it is possible to alter the OAS1 splicing patterns for all of the individuals carrying

the OAS1 IVS5-1 A-allele and boost the immunity of the entire population (simulation using the Susceptible, Infected and Removed (SIR) model, see the above for details). It is expected that this approach based on genome-based precision medicine with a splicing modulator will contribute to the management of the COVID-19 pandemic.

[Example 2]

RT-PCR regarding OAS1 alternative splicing profiles in Calu-3 (A/A allele) cells treated for 18 hours with 0.1% DMSO, or 10 pM compound 1, 10 pM compound 2, or 10 pM compound 3 was performed. When 18-hour treatment was completed, RNA was collected, and RT-PCR was performed using primers (Table 2) designed for each splicing form. β actin (ACTB) was used as an internal standard. Results thereof are shown in FIG. 8.

The compounds 2 treatment and the compound 3 treatment increased the yield of the p44a splice form (FIG. 8). The yields of the p44a splice form in the compounds 2 treatment and in the compound 3 treatment were higher than that in the compound 1 treatment, and the yield of the p44a splice form in the compound 3 treatment was higher than that in the compound 2 treatment. That is, similarly to the compound 1, the compounds 2 and 3, which were CLK inhibitors, induced splice-switching of the OAS1 rs10774671 A-allele. These results suggest that the treatment using the compounds 2 and 3 could overcome A-allele-derived vulnerabilities to SAR,S-CoV-2 infections.

[Example 3]

RT-PCR regarding OAS1 alternative splicing profiles in Calu-3 (A/A allele) cells treated for 18 hours with 0.1% DMSO, 1 pM compound 1, 3 pM compound 1, or 10 pM compound 1, or 10 pM compound 4 or 30 pM compound 4 was performed. When 18-hour treatment was completed, RNA was collected, and RT-PCR was performed using primers (Table 2) designed for each splicing form. Results thereof are shown in FIG. 9.

Similarly to the compound 1 treatment, the compound 4 treatment increased the yield of p44a splice form (FIG. 9). That is, similarly to the compound 1, the compound 4, which was a CLK inhibitor, induced splice-switching of the OAS 1 rs10774671 A-allele. This result suggests that the treatment using the compound 4 could overcome A-allele-derived vulnerabilities to SARS-CoV-2 infections.

References

- Adam D. 2020. A guide to R - the pandemic's misunderstood metric. Nature 583:346-348. doi:10.1038/d41586-020-02009-w
- Blanco-Melo D, Nilsson-Payant BE, Liu W, Uhl S, Hoagland D, Moller R, Jordan TX, Oishi K, Panis M, Sachs D, Wang TT, Schwartz RE, Lim JK, Albrecht RA, TenOever BR. 2020. Imbalanced Host Response to SARS-CoV-2 Drives Development of COVID-19. Cell 181:1036-1045.e9. doi:10.1016/j.cell.2020.04.026
- Carey CM, Govande AA, Cooper JM, Hartley MK, Kranzusch PJ, Elde NC. 2019. Recurrent Loss-of-Function Mutations Reveal Costs to OAS1 Antiviral Activity in Primates. Cell Host Microbe 25:336-343.e4. doi:10.1016/j.chom.2019.01.001
- Cartegni L, Wang J, Zhu Z, Zhang MΓa, Krainer AR. 2003. ESEfinder: A web resource to identify exonic splicing enhancers. Nucleic Acids Res 31:3568-71.doi:10.1093/nar/gkg616
- Choi UY, Kang J-S, Hwang YS, Kim Y-J. 2015. Oligoadenylate synthase-like (OASL) proteins: dual functions and associations with diseases. Exp Mol Med 47:e144. doi:10.1038/emm.2014.110
- Di H, Elbahesh H, Brinton MA. 2020. Characteristics of Human OAS1 Isoform Proteins. Viruses 12. doi:10.3390/v12020152
- Ford E, Thanos D. 2010. The transcriptional code of human IFN-beta gene expression. Biochim Biophys Acta 1799:328-36. doi:10.1016/j.bbagrm.2010.01.010
- Hamano E, Hijikata M, Itoyama S, Quy T, Phi NC, Long HT, Ha LD, Ban V Van, Matsushita I, Yanai H, Kirikae F, Kirikae T, Kuratsuji T, Sasazuki T, Keicho N. 2005. Polymorphisms of interferon-inducible genes OAS-1 and MxA associated with SARS in the Vietnamese population. Biochem Biophys Res Commun 329:1234-9. doi:10.1016/j.bbrc.2005.02.101
- Harko T, Lobo FSN, Mak MK. 2014. Exact analytical solutions of the Susceptible-Infected-Recovered (SIR) epidemic model and of the SIR model with equal death and birth rates. Appl Math Comput 236:184-194. doi:10.1016/j.amc.2014.03.030
- He J, Feng D, de Vlas SJ, Wang H, Fontanet A, Zhang P, Plancoulaine S, Tang F, Zhan L, Yang H, Wang T, Richardus JH, Habbema JDF, Cao W. 2006. Association of SARS susceptibility with single nucleic acid polymorphisms of OAS1 and MxA genes: a case-control study. BMC Infect Dis 6:106. doi:10.1186/1471-2334-6-106

- Hung IF-N, Lung K-C, Tso EY K, Liu R, Chung TW-H, Chu M-Y, Ng Y-Y, Lo J, Chan J, Tam AR, Shum H-P, Chan V, Wu AK-L, Sin K-M, Leung W-S, Law W-L, Lung DC, Sin S, Yeung P, Yip CC-Y, Zhang RR, Fung AY-F, Yan EY-W, Leung K-H, Ip JD, Chu AW-H, Chan Wan-Mui, Ng AC-K, Lee R, Fung K, Yeung A, Wu T-C, Chan JW-M, Yan W-W, Chan Wai-Ming, Chan JF-W, Lie AK-W, Tsang OTY, Cheng VC-C, Que T-L, Lau C-S, Chan K-H, To KK-W, Yuen K-Y 2020. Triple combination of interferon beta-lb, lopinavir-ritonavir, and ribavirin in the treatment of patients admitted to hospital with COVID-19: an open-label, randomised, phase 2 trial. Lancet (London, England) 395:1695-1704. doi:10.1016/S0140-6736(20)31042-4
- Ibsen MS, Gad HH, Andersen LL, Hornung V, Julkunen I, Sarkar SN, Hartmann R. 2015. Structural and functional analysis reveals that human OASL binds dsRNA to enhance RIG-I signaling. Nucleic Acids Res 43:5236-48. doi:10.1093/nar/gkv389
- Kjaer KH, Pahus J, Hansen MF, Poulsen JB, Christensen EI, Justesen J, Martensen PM. 2014. Mitochondrial localization of the OAS1 p46 isoform associated with a common single nucleotide polymorphism. BMC Cell Biol 15:1-14. doi:10.1186/1471-2121-15-33
- Lin R-J, Yu H-P, Chang B-L, Tang W-C, Liao C-L, Lin Y-L. 2009. Distinct antiviral roles for human 2',5'-oligoadenylate synthetase family members against dengue virus infection. J Immunol 183:8035-43. doi:10.4049/jimmunol.0902728
- Marcus JH, Novembre J. 2017. Visualizing the geography of genetic variants. Bioinformatics 33:594-595. doi:10.1093/bioinformatics/btw643
- Mendez FL, Watkins JC, Hammer MF. 2013. Neandertal origin of genetic variation at the cluster of OAS immunity genes. Mol Biol Evol 30:798-801.doi:10.1093/molbev/mst004
- Monk PD, Marsden RJ, Tear VJ, Brookes J, Batten TN, Mankowski M, Gabbay FJ, Davies DE, Holgate ST, Ho L-P, Clark T, Djukanovic R, Wilkinson TMA, Inhaled Interferon Beta COVID-19 Study Group. 2021. Safety and efficacy of inhaled nebulised interferon beta-la (SNG001) for treatment of SARS-CoV-2 infection: a randomised, double-blind, placebo-controlled, phase 2 trial. Lancet Respir Med 9:196-206. doi:10.1016/S2213-2600(20)30511-7
- Nile SH, Nile A, Qiu J, Li L, Jia X, Kai G. 2020. COVID-19: Pathogenesis, cytokine storm and therapeutic potential of interferons. Cytokine Growth Factor Rev 53:66-70. doi:10.1016/j.cytogfr.2020.05.002
- Noguchi S, Hamano E, Matsushita I, Hijikata M, Ito H, Nagase T, Keicho N. 2013. Differential effects of a common splice site polymorphism on the generation of OAS1 variants in human bronchial epithelial cells. Hum Immunol 74:395-401. doi: 10. 10 16/j.humimm.2012. 11.0 11
- Pairo-Castineira E, Clohisey S, Klaric L, Bretherick AD, Rawlik K, Pasko D, Walker S, Parkinson N, Fourman MH, Russell CD, Furniss J, Richmond A, Gountouna E, Wrobel N, Harrison D, Wang B, Wu Y, Meynert A, Griffiths F, Oosthuyzen W, Kousathanas A, Moutsianas L, Yang Z, Zhai R, Zheng C, Grimes G, Beale R, Millar J, Shih B, Keating S, Zechner M, Haley C, Porteous DJ, Hayward C, Yang J, Knight J, Summers C, Shankar-Hari M, Klenerman P, Turtle L, Ho A, Moore SC, Hinds C, Horby P, Nichol A, Maslove D, Ling L, McAuley D, Montgomery H, Walsh T, Pereira AC, RenieriA, GenOMICC Investigators, ISARIC4C Investigators, COVID-19 Human Genetics Initiative, 23andMe Investigators, BRACOVID Investigators, Gen-COVID Investigators, Shen X, Ponting CP, Fawkes A, Tenesa A, Caulfield M, Scott R, Rowan K, Murphy L, Openshaw PJM, Semple MG, Law A, Vitart V, Wilson JF, Baillie JK. 2021. Genetic mechanisms of critical illness in COVID-19. Nature 591:92-98.doi:10.1038/s41586-020-03065-y
- Sadler AJ, Williams BRG. 2008. Interferon-inducible antiviral effectors. Nat Rev Immunol 8:559-68. doi:10.1038/nri2314
- Sakuma M, Iida K, Hagiwara M. 2015. Deciphering targeting rules of splicing modulator compounds: case of TG003. BMC Mol Biol 16:16. doi:10.1186/s12867-015-0044-6
- Schlee M, Hartmann G. 2016. Discriminating self from non-self in nucleic acid sensing. Nat Rev Immunol 16:566-80. doi:10.1038/nri.2016.78
- Schwanke H, Stempel M, Brinkmann MM. 2020. Of Keeping and Tipping the Balance: Host Regulation and Viral Modulation of IRF3-Dependent IFNB1 Expression. Viruses 12. doi:10.3390/v12070733
- Shalhoub S. 2020. Interferon beta-lb for COVID-19. Lancet (London, England) 395:1670-1671. doi:10.1016/S0140-6736(20)31101-6
- Shibata S, Ajiro M, Hagiwara M. 2020. Mechanism-Based Personalized Medicine for Cystic Fibrosis by Suppressing Pseudo Exon Inclusion. Cell Chem Biol 27:1472-1482.e6. doi:10.1016/j.chembiol.2020.08.013

Soetaert K, Petzoldt T, Setzer RW. 2010. Solving Differential Equations in R : Package deSolve. J Stat Softw 33. doi:10.18637/jss.v033.i09
- The COVID-19 Host Genetics Initiative. 2021. Mapping the human genetic architecture of COVID-19 by worldwide meta-analysis. medRxiv 2021.03.10.21252820. doi:10.1101/2021.03.10.21252820
- Zeberg H, Paabo S. 2021. A genomic region associated with protection against severe COVID-19 is inherited from Neandertals. Proc Natl Acad Sci U S A 118.doi:10.1073/pnas.2026309118
- Zhou S, Butler-Laporte G, Nakanishi T, Morrison DR, Afilalo J, Afilalo M, Laurent L, Pietzner M, Kerrison N, Zhao K, Brunet-Ratnasingham E, Henry D, Kimchi N, Afrasiabi Z, Rezk N, Bouab M, Petitjean L, Guzman C, Xue X,

Tselios C, Vulesevic B, Adeleye O, Abdullah T, Almamlouk N, Chen Y, Chasse M, Durand M, Paterson C, Normark J, Frithiof R, Lipcsey M, Hultstrom M, Greenwood CMT, Zeberg H, Langenberg C, Thysell E, Pollak M, Mooser V, Forgetta V, Kaufmann DE, Richards JB. 2021. A Neanderthal OAS1 isoform protects individuals of European ancestry against COVID-19 susceptibility and severity. Nat Med. doi:10.1038/s41591-021-01281-1

## Claims

**1.** A method for enhancing an innate immune response of a cell,

wherein the cell is a cell having an innate immune response that a gene to be subjected to splicing participates in, wherein the method comprises bringing a compound that affects alternative splicing into contact with the cell.

**2.** The method according to claim 1, wherein the cell is a cell having an innate immune response attenuated by alternative splicing of a gene for the innate immune response.

**3.** The method according to claim 1 or 2, wherein the compound is a compound having an ability to inhibit or activate a protein kinase CLK (CDC-like kinase).

**4.** The method according to any one of claims 1 to 3, wherein the cell has a mutation or polymorphism (may include an SNP) that causes alternative splicing that attenuates the innate immune response in a gene participates in an innate immune response pathway.

**5.** The method according to any one of claims 1 to 4, wherein the innate immune response is an innate immune response realized by a pathway selected from the group consisting of TP53, TLR3, TLR7, RARRES3, IFNA1, IFNA2, IFNA4, IFNA5, IFNA6, IFNA7, IFNA8, IFNA10, IFNA13, IFNA14, IFNA16, IFNA17, IFNA21, IFNK, IFNB1, IL6, TICAM1, TICAM2, MAVS, STAT1, STAT2, EIF2AK2, IRF3, TBK1, CDKN1A, CDKN2A, RNASEL, IFNAR1, IFNAR2, OAS1, OAS2, OAS3, OASL, RB1, ISG15, ISG20, IFIT1, IFIT2, IFIT3, and IFIT5, or a biologically active fragment, analog, or variant thereof.

**6.** The method according to any one of claims 1 to 5, wherein the cell has the A-allele of SNP rs10774671.

**7.** The method according to any one of claims 1 to 6, wherein the innate immune response is an innate immune response to infection with a virus, for example, an RNA virus, for example, a coronavirus, for example, SARS-CoV, MARS-CoV, or SARS-CoV-2.

**8.** The method according to any one of claims 3 to 7, wherein the compound having the ability to inhibit or activate a protein kinase CLK (CDC-like kinase) has an ability to inhibit at least one kinase belonging to the CLK family, has activity of inhibiting the phosphorylation activity of at least one of the kinases belonging to the CLK family, or has an ability to inhibit at least one selected from the group consisting of CLK1, CLK2, CLK3, and CLK4.

**9.** The method according to any one of claims 3 to 8,

wherein the compound having the ability to inhibit or activate a protein kinase CLK (CDC-like kinase) is a compound represented by Formulae (II) to (VIII) below, a prodrug thereof, or a pharmaceutically acceptable salt thereof,

[Chemical Formula 1]

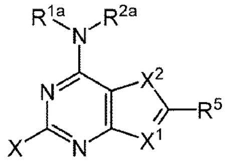 (II) 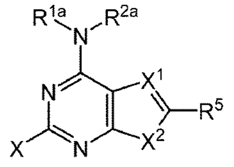 (II') 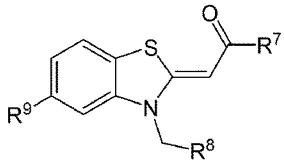 (III)

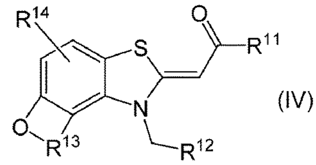 (IV) 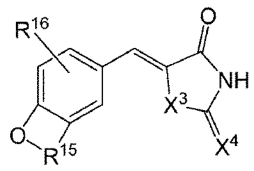 (V)

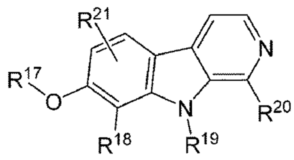 (VI) 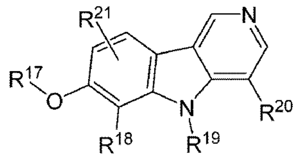 (VII) 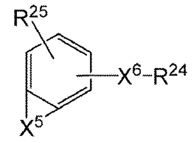 (VIII)

where, in Formulae (II) and (II'),

$R^{1a}$ and $R^{2a}$ each independently represent a hydrogen atom, a substituted or unsubstituted $C_1$-$C_6$ alkyl group, a substituted or unsubstituted benzyl group, a substituted or unsubstituted heteroarylmethyl group, a substituted or unsubstituted heteroarylethyl group, a substituted or unsubstituted aryloxy group, a substituted or unsubstituted heteroaryloxy group, a substituted or unsubstituted alkoxyamidoalkyl group, a substituted or unsubstituted aryl group, or a substituted or unsubstituted heteroaryl group, or alternatively, $R^{1a}$ and $R^{2a}$ bind to each other to form a ring together with N, and the ring is a substituted or unsubstituted monocyclic heterocyclic ring, or a substituted or unsubstituted bicyclic heterocyclic ring;

$R^5$ represents a hydrogen atom, a halogen atom, or a substituted or unsubstituted $C_1$-$C_6$ alkoxy group;

$X^1$ represents N or CH;

$X^2$ represents -N($R^3$)-, S, or O;

$R^3$ represents a hydrogen atom, a $C_1$-$C_6$ alkyl group, a benzyl group, a heteroarylmethyl group, a substituted or unsubstituted aryl group, a substituted or unsubstituted heteroaryl group, or $CH_2OC(O)R^4$-;

$R^4$ represents a $C_1$-$C_6$ alkyl group, a benzyl group, a heteroarylmethyl group, a substituted or unsubstituted aryl group, or a substituted or unsubstituted heteroaryl group; and

X represents a hydrogen atom, a halogen atom, an amino group, an $R^{1a}$- and $R^{2a}$-substituted amino group, an azido group, a cyano group, a nitro group, a hydroxy group, a $C_1$-$C_6$ alkyloxy group, a substituted or unsubstituted aryloxy group, a substituted or unsubstituted heteroaryloxy group, a mercapto group, a $C_1$-$C_6$ alkylthio group, a substituted or unsubstituted arylthio group, a substituted or unsubstituted heteroarylthio group, a benzyl group, a heteroarylmethyl group, a substituted or unsubstituted aryl group, or a substituted or unsubstituted heteroaryl group;

in Formula (III),

$R^7$ and $R^8$ each independently represent a hydrogen atom, a halogen-substituted or unsubstituted $C_1$-$C_{10}$ alkyl group, or a $C_2$-$C_6$ alkenyl group;

$R^9$ represents a hydrogen atom, a halogen atom, or a halogen-substituted or unsubstituted $C_1$-$C_{10}$ alkyl group, $-OR^{10}$, $-NHR^{10}$, or $-N(R^{10})_2$; and

$R^{10}$ represents a hydrogen atom or a $C_1$-$C_{10}$ alkyl group;

in Formula (IV),

$R^{11}$ and $R^{12}$ each independently represent a hydrogen atom or a $C_1$-$C_6$ alkyl group;

$R^{13}$ represents

[Chemical Formula 2]

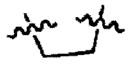 , 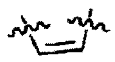 , or 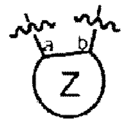

where Z forms, together with atoms marked with a and b, a ring selected from the group consisting of one benzene ring, one heteroaromatic ring, an aromatic ring fused with one or more benzene rings, a heteroaromatic ring fused with one or more heteroaromatic rings, a mixed fused polycyclic ring in which one or more benzene rings and one or more heteroaromatic rings are fused, and cycloaliphatic groups, and the ring may include one or more substituents, the substituents being a hydrogen atom, a halogen atom, or a $C_1$-$C_6$ alkyl group; and
$R^{14}$ represents a hydrogen atom, a halogen atom, or a $C_1$-$C_6$ alkyl group;

in Formula (V),

$X^3$ and $X^4$ each independently represent S or NH;
$R^{15}$ represents

[Chemical Formula 3]

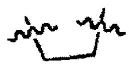 , 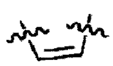 , or 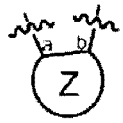

where Z forms, together with atoms marked with a and b, a ring selected from the group consisting of one benzene ring, one heteroaromatic ring, an aromatic ring fused with one or more benzene rings, a heteroaromatic ring fused with one or more heteroaromatic rings, a mixed fused polycyclic ring in which one or more benzene rings and one or more heteroaromatic rings are fused, and cycloaliphatic groups, and the ring may include one or more substituents, the substituents being a hydrogen atom, a halogen atom, or a $C_1$-$C_6$ alkyl group; and
$R^{16}$ represents a hydrogen atom, a halogen atom, or a $C_1$-$C_6$ alkyl group;

in Formulae (VI) and (VII),

$R^{17}$ and $R^{19}$ each independently represent a hydrogen atom, a $C_1$-$C_6$ alkyl group, a benzyl group, a heteroarylmethyl group, a substituted or unsubstituted aryl group, or a substituted or unsubstituted heteroaryl group;
$R^{18}$ represents $-R^{22}$, $-C{\equiv}C-R^{22}$, $-CH{=}CH-R^{22}$, or $-O-(CH_2)n-R^{22}$, n is 1 to 6, $R^{22}$ represents a hydrogen atom, a hydroxy group, a $C_1$-$C_8$ alkyl group, $-Si(R^{23})_3$, a substituted or unsubstituted phenyl group, a monocyclic heteroaromatic group, or a cycloaliphatic group, or alternatively, $R^{17}$ and $R^{18}$ bind to each other to form a ring, and $-R^{17}-R^{18}-$ is substituted by $-(CH_2)m-CH_2-$, $-CH{=}CH-$, $-(CH_2)m-O-$, or a halogen atom, m is 1 to 6, $R^{23}$ represents a hydrogen atom, a $C_1$-$C_6$ alkyl group, a trihalomethyl group, or a hydroxy group, and three $R^{23}$ of $-Si(R^{23})_3$ may be different from each other; and
$R^{20}$ and $R^{21}$ represent a hydrogen atom or a $C_1$-$C_6$ alkyl group;

in Formula (VIII),

$X^5$ represents

[Chemical Formula 4]

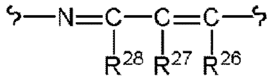 , 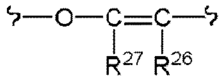 , or 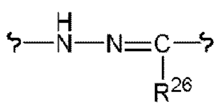

where $R^{26}$, $R^{27}$, and $R^{28}$ each independently represent a hydrogen atom, a halogen atom, a carboxyl group, an amino group, a hydroxy group, a $C_1$-$C_4$ alkyl group, or a halogen-substituted $C_1$-$C_4$ alkyl group;
$X^6$ represents -(atomic bonding) or -NH-;
$R^{24}$ represents

[Chemical Formula 5]

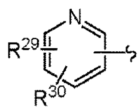
,
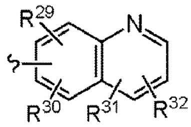
,
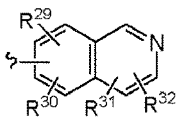
,
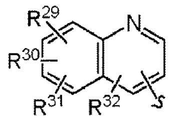
, or
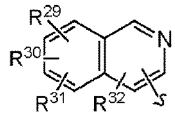

where $R^{29}$, $R^{30}$, $R^{31}$, and $R^{32}$ each independently represent a hydrogen atom, a halogen atom, a carboxyl group, an amino group, a hydroxy group, a $C_1$-$C_4$ alkyl group, or a halogen-substituted $C_1$-$C_4$ alkyl group; and $R^{25}$ represents a hydrogen atom, a halogen atom, a carboxyl group, an amino group, a hydroxy group, or a halogen-substituted or unsubstituted $C_1$-$C_4$ alkyl group.

**10.** A pharmaceutical composition for enhancing an innate immune response of a subject or a cell,

wherein the subject or the cell is a subject or a cell having an innate immune response that a gene to be subjected to splicing participates in,
wherein the pharmaceutical composition comprises a compound that affects alternative splicing as an active ingredient.

**11.** The pharmaceutical composition according to claim 10,
wherein the subject or the cell is a subject or a cell having an innate immune response attenuated by alternative splicing of a gene for the innate immune response.

**12.** The pharmaceutical composition according to claim 10 or 11,
wherein the compound is a compound having an ability to inhibit or activate a protein kinase CLK (CDC-like kinase).

**13.** The pharmaceutical composition according to any one of claims 10 to 12,
wherein the subject or the cell has a mutation or polymorphism (may include an SNP) that causes alternative splicing that attenuates the innate immune response in a gene involved in an innate immune response pathway.

**14.** The pharmaceutical composition according to any one of claims 10 to 13,
wherein the innate immune response is an innate immune response realized by a pathway selected from the group consisting of TP53, TLR3, TLR7, RARRES3, IFNA1, IFNA2, IFNA4, IFNA5, IFNA6, IFNA7, IFNA8, IFNA10, IFNA13, IFNA14, IFNA16, IFNA17, IFNA21, IFNK, IFNB1, IL6, TICAM1, TICAM2, MAVS, STAT1, STAT2, EIF2AK2, IRF3, TBK1, CDKN1A, CDKN2A, RNASEL, IFNAR1, IFNAR2, OAS1, OAS2, OAS3, OASL, RB1, ISG15, ISG20, IFIT1, IFIT2, IFIT3, and IFIT5, or a biologically active fragment, analog, or variant thereof.

**15.** The pharmaceutical composition according to any one of claims 10 to 14,
wherein the subject or the cell has the A-allele of SNP rs10774671.

**16.** The pharmaceutical composition according to any one of claims 10 to 15,
wherein the innate immune response is an innate immune response to infection with a virus, for example, an RNA virus, for example, a coronavirus, for example, SARS-CoV, MARS-CoV, or SARS-CoV-2.

**17.** The pharmaceutical composition according to any one of claims 12 to 16,
wherein the compound having the ability to inhibit or activate a protein kinase CLK (CDC-like kinase) has an ability to inhibit at least one kinase belonging to the CLK family, has activity of inhibiting the phosphorylation activity of at least one of the kinases belonging to the CLK family, or has an ability to inhibit at least one selected from the group consisting of CLK1, CLK2, CLK3, and CLK4.

**18.** The pharmaceutical composition according to any one of claims 12 to 17,

wherein the compound having the ability to inhibit or activate a protein kinase CLK (CDC-like kinase) is a compound represented by Formulae (II) to (VIII) below, a prodrug thereof, or a pharmaceutically acceptable salt thereof,

[Chemical Formula 6]

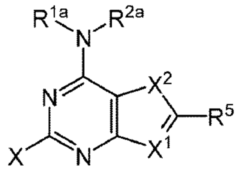 (II) 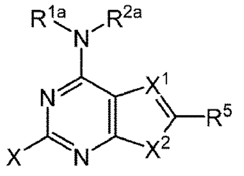 (II') 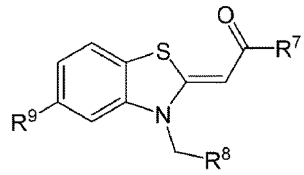 (III)

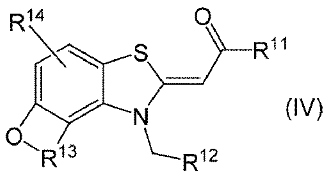 (IV) 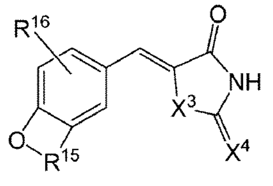 (V)

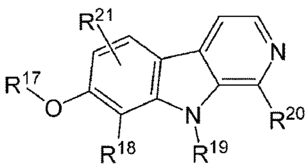 (VI) 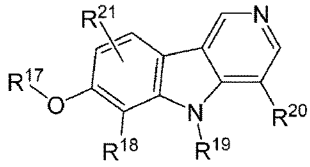 (VII) 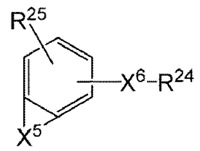 (VIII)

where, in Formulae (II) and (II'),

$R^{1a}$ and $R^{2a}$ each independently represent a hydrogen atom, a substituted or unsubstituted $C_1$-$C_6$ alkyl group, a substituted or unsubstituted benzyl group, a substituted or unsubstituted heteroarylmethyl group, a substituted or unsubstituted heteroarylethyl group, a substituted or unsubstituted aryloxy group, a substituted or unsubstituted heteroaryloxy group, a substituted or unsubstituted alkoxyamidoalkyl group, a substituted or unsubstituted aryl group, or a substituted or unsubstituted heteroaryl group, or alternatively, $R^{1a}$ and $R^{2a}$ bind to each other to form a ring together with N, and the ring is a substituted or unsubstituted monocyclic heterocyclic ring, or a substituted or unsubstituted bicyclic heterocyclic ring,

$R^5$ represents a hydrogen atom, a halogen atom, or a substituted or unsubstituted $C_1$-$C_6$ alkoxy group;

$X^1$ represents N or CH;

$X^2$ represents -N($R^3$)-, S, or O;

$R^3$ represents a hydrogen atom, a $C_1$-$C_6$ alkyl group, a benzyl group, a heteroarylmethyl group, a substituted or unsubstituted aryl group, a substituted or unsubstituted heteroaryl group, or $CH_2OC(O)R^4$-;

$R^4$ represents a $C_1$-$C_6$ alkyl group, a benzyl group, a heteroarylmethyl group, a substituted or unsubstituted aryl group, or a substituted or unsubstituted heteroaryl group; and

X represents a hydrogen atom, a halogen atom, an amino group, an $R^{1a}$- and $R^{2a}$-substituted amino group, an azido group, a cyano group, a nitro group, a hydroxy group, a $C_1$-$C_6$ alkyloxy group, a substituted or unsubstituted aryloxy group, a substituted or unsubstituted heteroaryloxy group, a mercapto group, a $C_1$-$C_6$ alkylthio group, a substituted or unsubstituted arylthio group, a substituted or unsubstituted heteroarylthio group, a benzyl group, a heteroarylmethyl group, a substituted or unsubstituted aryl group, or a substituted or unsubstituted heteroaryl group;

in Formula (III),

$R^7$ and $R^8$ each independently represent a hydrogen atom, a halogen-substituted or unsubstituted $C_1$-$C_{10}$ alkyl group, or a $C_2$-$C_6$ alkenyl group;

$R^9$ represents a hydrogen atom, a halogen atom, or a halogen-substituted or unsubstituted $C_1$-$C_{10}$ alkyl group, -$OR^{10}$, -$NHR^{10}$, or -$N(R^{10})_2$; and

$R^{10}$ represents a hydrogen atom or a $C_1$-$C_{10}$ alkyl group;

in Formula (IV),

$R^{11}$ and $R^{12}$ each independently represent a hydrogen atom or a $C_1$-$C_6$ alkyl group;
$R^{13}$ represents

[Chemical Formula 7]

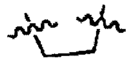, 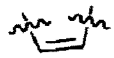, or 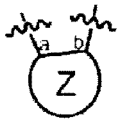

where Z forms, together with atoms marked with a and b, a ring selected from the group consisting of one benzene ring, one heteroaromatic ring, an aromatic ring fused with one or more benzene rings, a heteroaromatic ring fused with one or more heteroaromatic rings, a mixed fused polycyclic ring in which one or more benzene rings and one or more heteroaromatic rings are fused, and cycloaliphatic groups, and the ring may include one or more substituents, the substituents being a hydrogen atom, a halogen atom, or a $C_1$-$C_6$ alkyl group; and
$R^{14}$ represents a hydrogen atom, a halogen atom, or a $C_1$-$C_6$ alkyl group;

in Formula (V),

$X^3$ and $X^4$ each independently represent S or NH;
$R^{15}$ represents

[Chemical Formula 8]

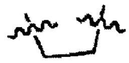, 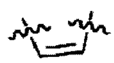, or 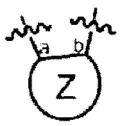

where Z forms, together with atoms marked with a and b, a ring selected from the group consisting of one benzene ring, one heteroaromatic ring, an aromatic ring fused with one or more benzene rings, a heteroaromatic ring fused with one or more heteroaromatic rings, a mixed fused polycyclic ring in which one or more benzene rings and one or more heteroaromatic rings are fused, and cycloaliphatic groups, and the ring may include one or more substituents, the substituents being a hydrogen atom, a halogen atom, or a $C_1$-$C_6$ alkyl group; and
$R^{16}$ represents a hydrogen atom, a halogen atom, or a $C_1$-$C_6$ alkyl group; in Formulae (VI) and (VII),
$R^{17}$ and $R^{19}$ each independently represent a hydrogen atom, a $C_1$-$C_6$ alkyl group, a benzyl group, a heteroarylmethyl group, a substituted or unsubstituted aryl group, or a substituted or unsubstituted heteroaryl group;
$R^{18}$ represents $-R^{22}$, $-C{\equiv}C-R^{22}$, $-CH=CH-R^{22}$, or $-O-(CH_2)n-R^{22}$, n is 1 to 6, $R^{22}$ represents a hydrogen atom, a hydroxy group, a $C_1$-$C_8$ alkyl group, $-Si(R^{23})_3$, a substituted or unsubstituted phenyl group, a monocyclic heteroaromatic group, or a cycloaliphatic group, or alternatively, $R^{17}$ and $R^{18}$ bind to each other to form a ring, and $-R^{17}-R^{18}-$ is substituted by $-(CH_2)m-CH_2-$, $-CH=CH-$, $-(CH_2)m-O-$, or a halogen atom, m is 1 to 6, $R^{23}$ represents a hydrogen atom, a $C_1$-$C_6$ alkyl group, a trihalomethyl group, or a hydroxy group, and three $R^{23}$ of $-Si(R^{23})_3$ may be different from each other; and
$R^{20}$ and $R^{21}$ represent a hydrogen atom or a $C_1$-$C_6$ alkyl group;

in Formula (VIII),

$X^5$ represents

[Chemical Formula 9]

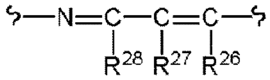, 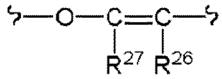, or 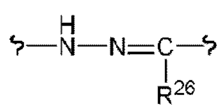

where $R^{26}$, $R^{27}$, and $R^{28}$ each independently represent a hydrogen atom, a halogen atom, a carboxyl group, an amino group, a hydroxy group, a $C_1$-$C_4$ alkyl group, or a halogen-substituted $C_1$-$C_4$ alkyl group;
$X^6$ represents -(atomic bonding) or -NH-;
$R^{24}$ represents

[Chemical Formula 10]

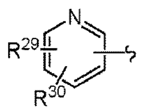,
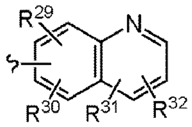,
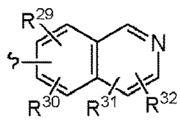,
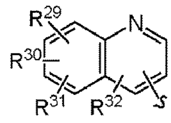, or
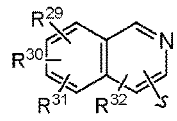

where $R^{29}$, $R^{30}$, $R^{31}$, and $R^{32}$ each independently represent a hydrogen atom, a halogen atom, a carboxyl group, an amino group, a hydroxy group, a $C_1$-$C_4$ alkyl group, or a halogen-substituted $C_1$-$C_4$ alkyl group; and $R^{25}$ represents a hydrogen atom, a halogen atom, a carboxyl group, an amino group, a hydroxy group, or a halogen-substituted or unsubstituted $C_1$-$C_4$ alkyl group.

**19.** A method for enhancing an innate immune response of a subject,

wherein the subject is a subject having an innate immune response that a gene to be subjected to splicing participates in,
wherein the method comprises administering a compound that affects alternative splicing to the subject.

**20.** A method for preventing, alleviating a symptom, or treating a viral infectious disease, the method comprising:

(1) confirming that a subject satisfies at least one condition selected from the group consisting of a) to d) below,

a) the subject has an innate immune response that a gene to be subjected to splicing participates in,
b) the subject has an innate immune response attenuated by alternative splicing of a gene for the innate immune response,
c) the subject has a mutation or polymorphism (including an SNP) that causes alternative splicing that attenuates the innate immune response in a gene participates in an innate immune response pathway, and
d) the subject has the A-allele of SNP rs10774671, and

(2) administering a compound that affects alternative splicing to the subject who satisfies the condition in the (1) above, or bringing a compound that affects alternative splicing into contact with the subject.

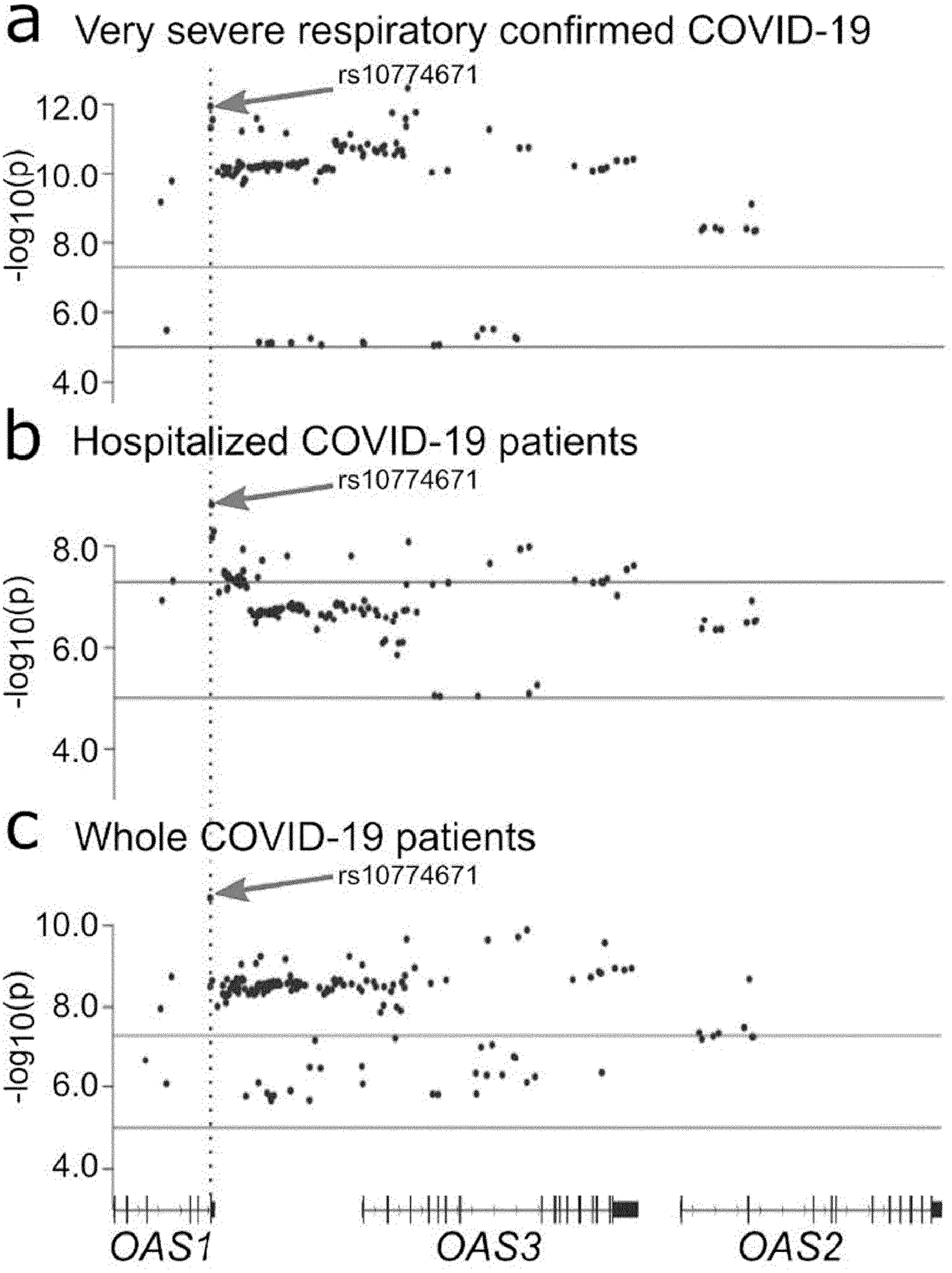
a Very severe respiratory confirmed COVID-19
rs10774671
12.0
10.0
8.0
6.0
4.0
-log10(p)
b Hospitalized COVID-19 patients
rs10774671
8.0
6.0
4.0
-log10(p)
c Whole COVID-19 patients
rs10774671
10.0
8.0
6.0
4.0
-log10(p)
OAS1
OAS3
OAS2

FIG. 1

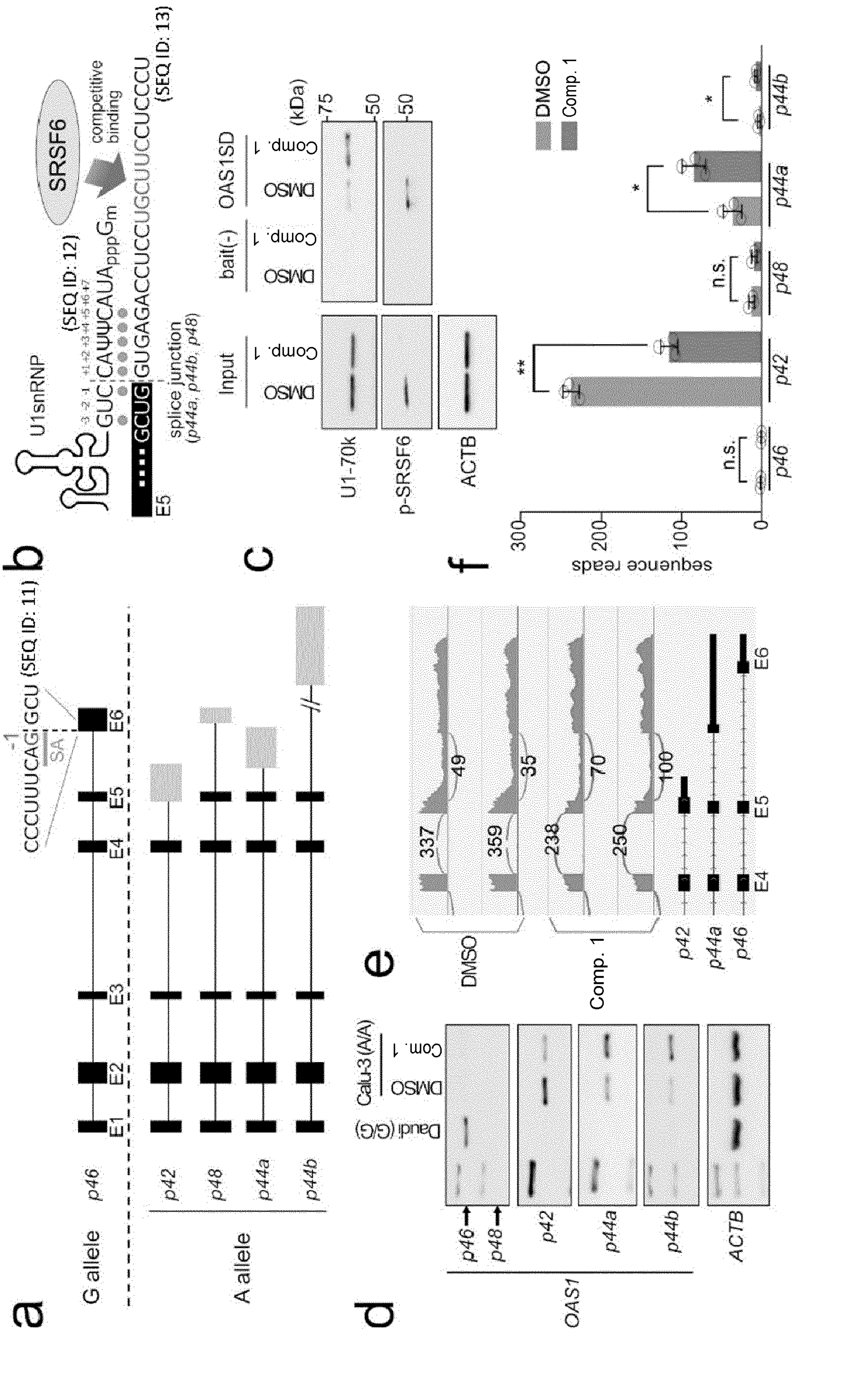
a
G allele
A allele
p46
p42
p48
p44a
p44b
E1
E2
E3
E4
E5
E6
CCCUUUCAG GCU (SEQ ID: 11)
-1
SA
b
U1snRNP
(SEQ ID: 12)
GUC CAΨΨCAUApppGm
GCUG GUGAGACCUCCUGCUUCCUCCCU
(SEQ ID: 13)
SRSF6
competitive binding
E5
splice junction
(p44a, p44b, p48)
c
Input
bait(-)
OAS1SD
DMSO
Comp. 1
(kDa)
75
50
50
U1-70k
p-SRSF6
ACTB
d
OAS1
Daudi (G/G)
Calu-3 (A/A)
DMSO
Com. 1
p46
p48
p42
p44a
p44b
ACTB
e
DMSO
Comp. 1
337
49
359
35
238
70
250
100
p42
p44a
p46
E4
E5
E6
f
sequence reads
300
200
100
0
DMSO
Comp. 1
n.s.
**
n.s.
*
*
p46
p42
p48
p44a
p44b
FIG. 2

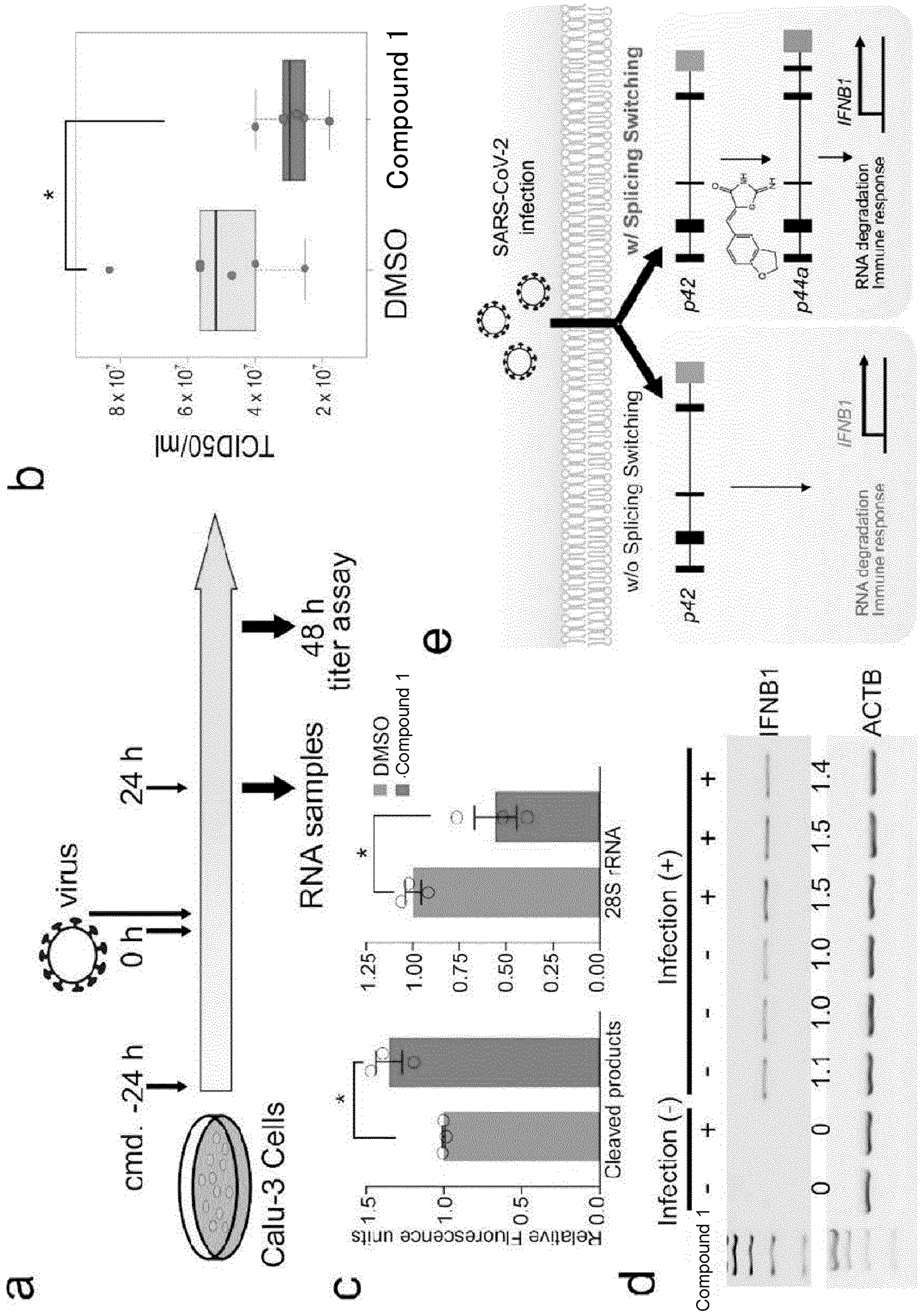
a
Calu-3 Cells
cmd. -24 h
virus
0 h
24 h
RNA samples
48 h
titer assay
b
TCID50/ml
8 x 10^7
6 x 10^7
4 x 10^7
2 x 10^7
*
DMSO
Compound 1
c
Relative Fluorescence units
Cleaved products
28S rRNA
DMSO
Compound 1
d
Compound 1
Infection (-)
Infection (+)
IFNB1
ACTB
0 0 1.1 1.0 1.0 1.5 1.5 1.4
e
SARS-CoV-2 infection
w/o Splicing Switching
w/ Splicing Switching
p42
p44a
IFNB1
RNA degradation
Immune response

FIG. 3

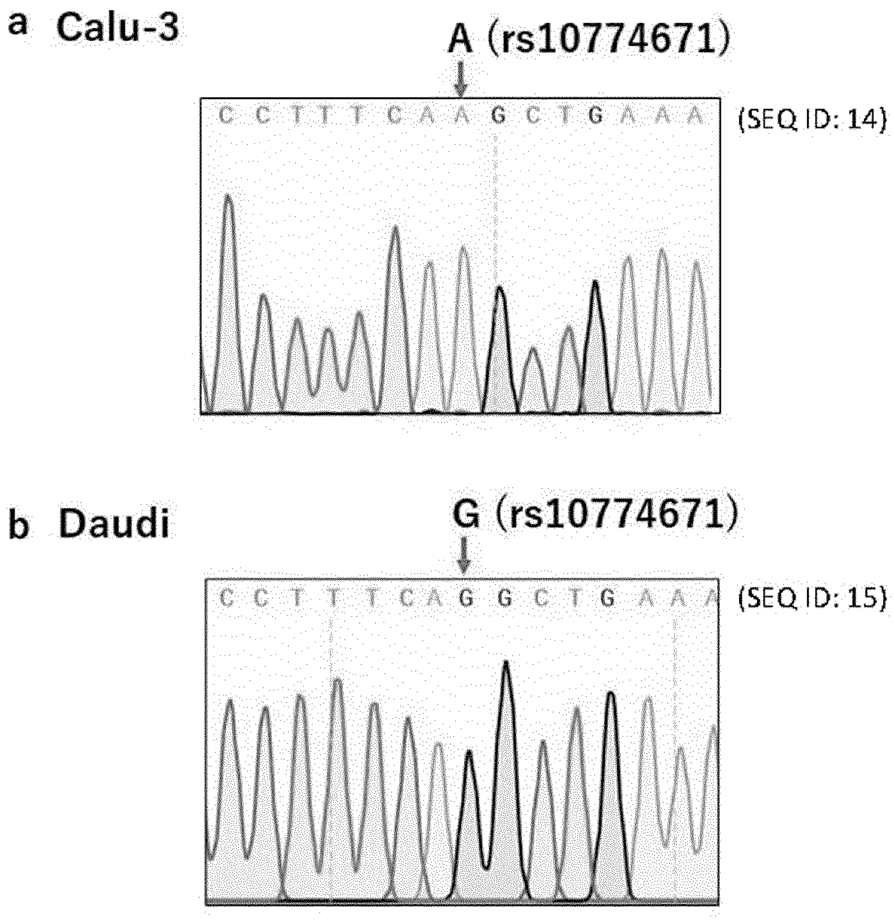
a Calu-3
A (rs10774671)
C C T T T C A A G C T G A A A
(SEQ ID: 14)
b Daudi
G (rs10774671)
C C T T T C A G G C T G A A A
(SEQ ID: 15)

FIG. 4

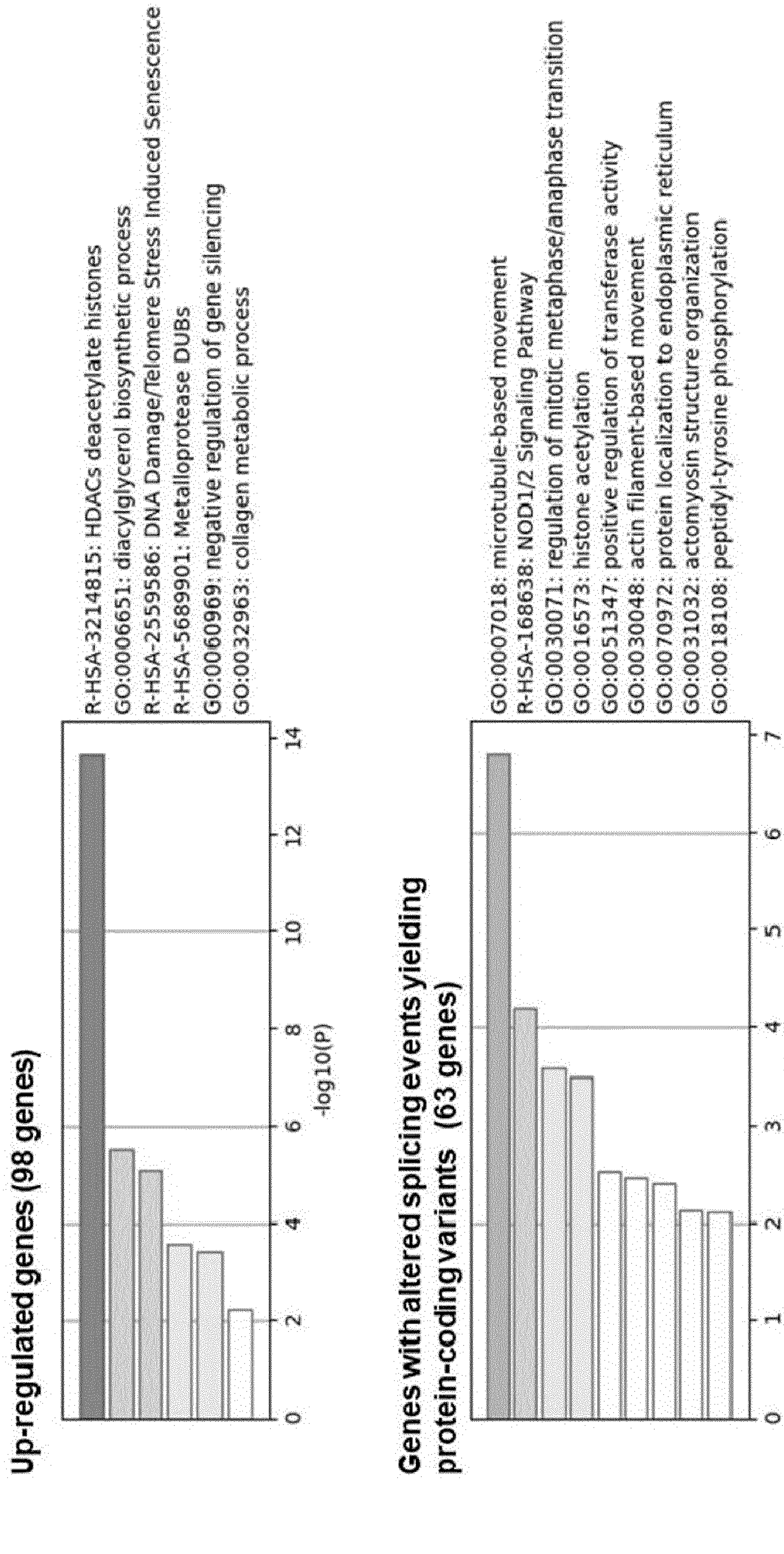
Up-regulated genes (98 genes)
R-HSA-3214815: HDACs deacetylate histones
GO:0006651: diacylglycerol biosynthetic process
R-HSA-2559586: DNA Damage/Telomere Stress Induced Senescence
R-HSA-5689901: Metalloprotease DUBs
GO:0060969: negative regulation of gene silencing
GO:0032963: collagen metabolic process
0
2
4
6
8
10
12
14
-log10(P)
Genes with altered splicing events yielding protein-coding variants (63 genes)
GO:0007018: microtubule-based movement
R-HSA-168638: NOD1/2 Signaling Pathway
GO:0030071: regulation of mitotic metaphase/anaphase transition
GO:0016573: histone acetylation
GO:0051347: positive regulation of transferase activity
GO:0030048: actin filament-based movement
GO:0070972: protein localization to endoplasmic reticulum
GO:0031032: actomyosin structure organization
GO:0018108: peptidyl-tyrosine phosphorylation
0
1
2
3
4
5
6
7
-log10(P)

FIG. 5

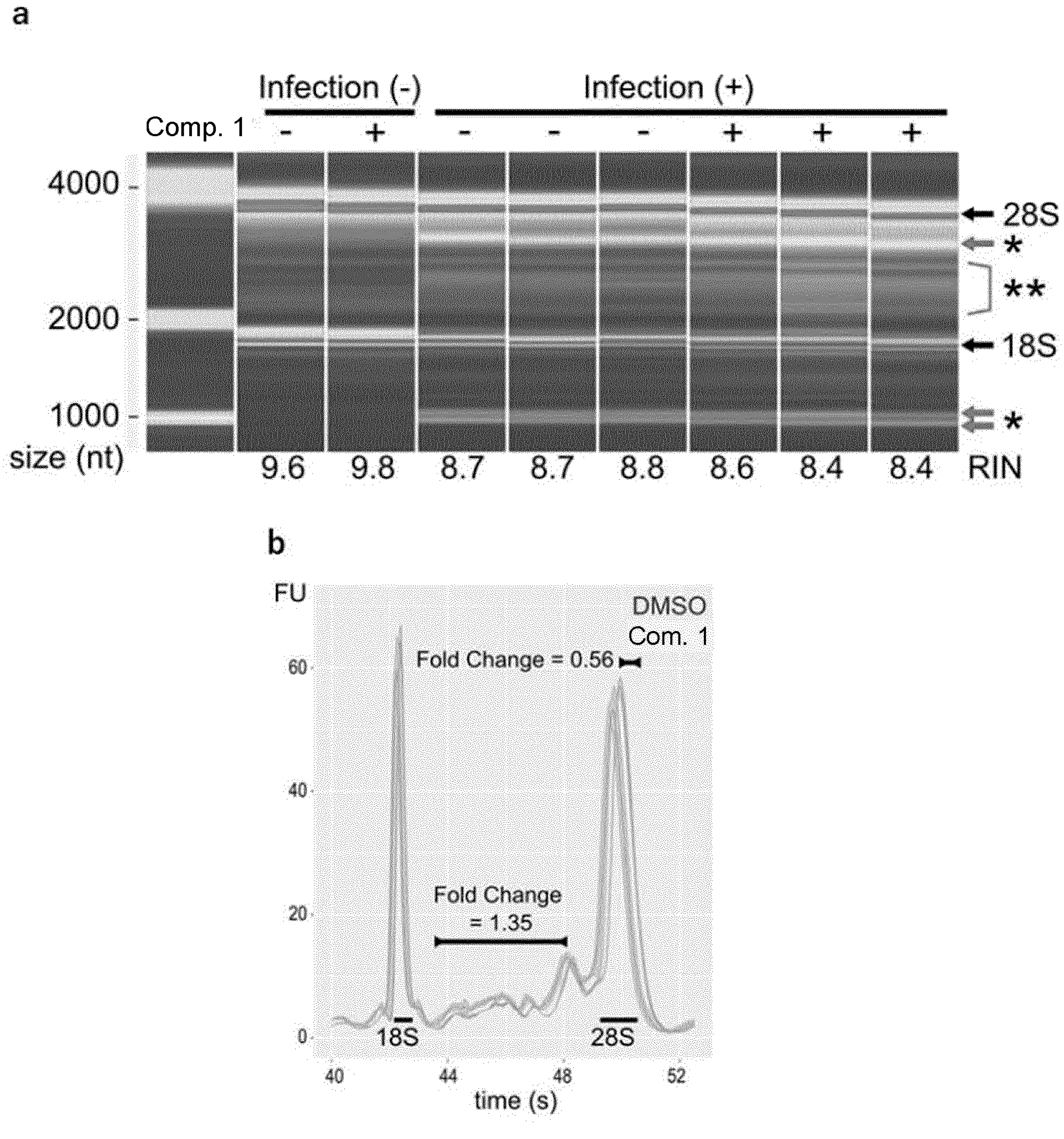
a
Infection (-)
Infection (+)
Comp. 1
-
+
-
-
-
+
+
+
4000
2000
1000
size (nt)
9.6
9.8
8.7
8.7
8.8
8.6
8.4
8.4
RIN
28S
*
**
18S
*
b
FU
DMSO
Com. 1
Fold Change = 0.56
Fold Change
= 1.35
60
40
20
0
18S
28S
40
44
48
52
time (s)

FIG. 6

chr12:113357193 G/A

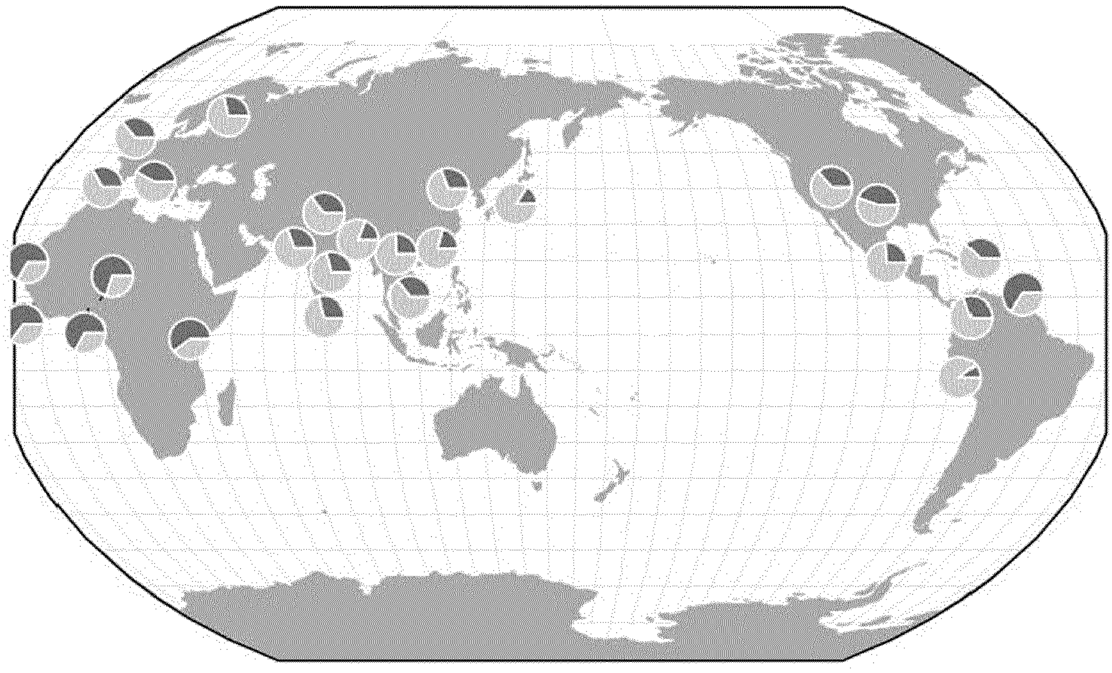

FIG. 7

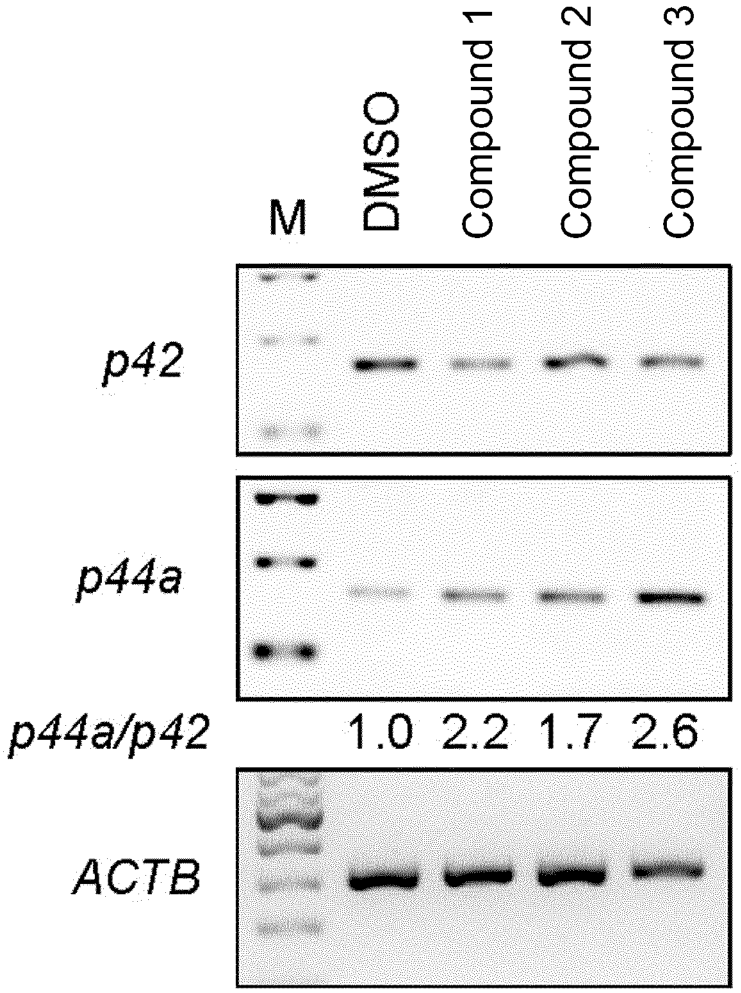
M
DMSO
Compound 1
Compound 2
Compound 3
p42
p44a
p44a/p42
1.0
2.2
1.7
2.6
ACTB

FIG. 8

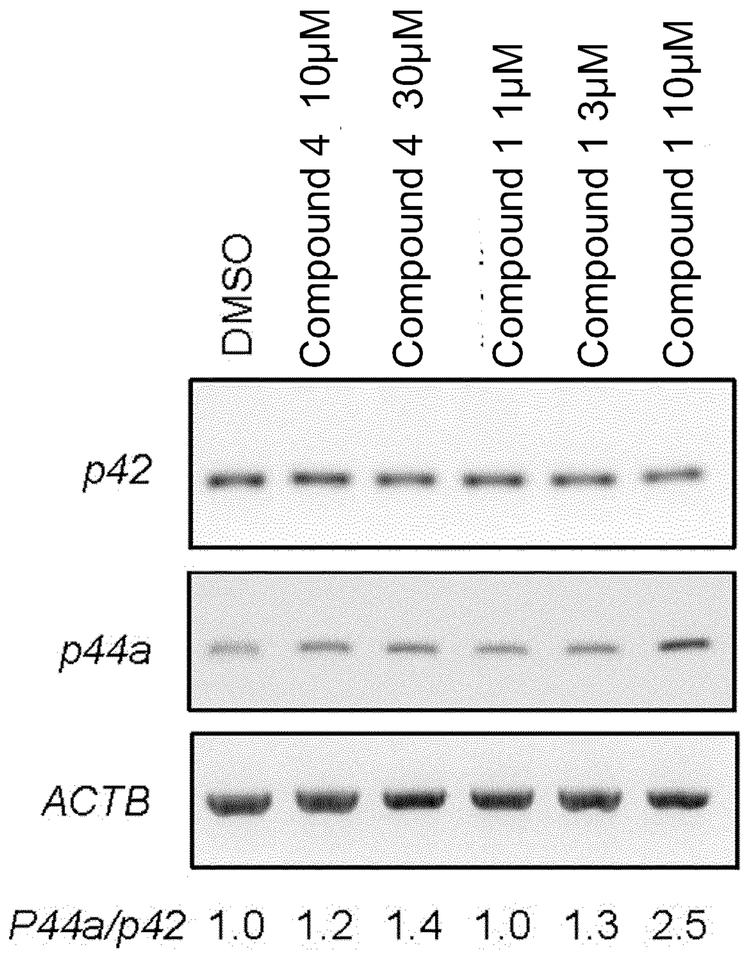
DMSO
Compound 4 10μM
Compound 4 30μM
Compound 1 1μM
Compound 1 3μM
Compound 1 10μM
p42
p44a
ACTB
P44a/p42 1.0 1.2 1.4 1.0 1.3 2.5

FIG. 9

**INTERNATIONAL SEARCH REPORT**

International application No.
**PCT/JP2022/022350**

**A. CLASSIFICATION OF SUBJECT MATTER**

***A61K 45/00***(2006.01)i; ***A61K 48/00***(2006.01)i; ***A61K 31/41***(2006.01)i; ***A61K 31/425***(2006.01)i; ***A61K 31/433***(2006.01)i; ***A61K 31/437***(2006.01)i
FI: A61K45/00; A61K48/00 ZNA; A61K31/425; A61K31/433; A61K31/437; A61K31/41

According to International Patent Classification (IPC) or to both national classification and IPC

**B. FIELDS SEARCHED**

Minimum documentation searched (classification system followed by classification symbols)

A61K45/00; A61K48/00; A61K31/41; A61K31/425; A61K31/433; A61K31/437

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| A | ARTARINI, A. et al. Regulation of influenza A virus mRNA splicing by CLK1. Antiviral Research. 2019, vol. 168, pp. 187-196<br>particularly, abstract, 3.2., 3.3., etc. | 1-20 |
| A | LI, C. et al. Anti-influenza effect and action mechanisms of the chemical constituent gallocatechin-7-gallate from Pithecellobium clypearia Benth. Acta Pharmacologica Sinica. 2018, vol. 39, no. 12, pp. 1913-1922<br>particularly, abstract, figures | 1-20 |
| A | DRAPPIER, M., MICHIELS, T. Inhibition of the OAS/RNase L pathway by viruses. Current Opinion in Virology. 2015, vol. 15, pp. 19-26<br>particularly, abstract, fig. 1, 2, table 1 | 1-20 |
| A | 網代将彦, 他, CLK-SRSF経路の阻害による偽エクソン型スプライシング疾患の治療効果, 第21回日本RNA学会年会要旨集, vol. 21, 2019, p. 47(O-45), (AJIRO, Masahiko et al.), non-official translation (Therapeutic effect of blocking CLK-SRSF pathway on pseudoexon splicing diseases. Abstracts of the 21st annual meeting of the RNA society of Japan.)<br>entire text | 1-20 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 July 2022** | **09 August 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

INTERNATIONAL SEARCH REPORT

International application No.
**PCT/JP2022/022350**

**C. DOCUMENTS CONSIDERED TO BE RELEVANT**

| Category* | Citation of document, with indication, where appropriate, of the relevant passages | Relevant to claim No. |
|---|---|---|
| A | WO 2014/021337 A1 (KYOTO UNIV.) 06 February 2014 (2014-02-06) particularly, compound 8 | 1-20 |
| A | WO 2018/043674 A1 (KYOTO UNIV.) 08 March 2018 (2018-03-08) particularly, claims, etc. | 1-20 |
| A | WO 2015/083750 A1 (KYOTO UNIV.) 11 June 2015 (2015-06-11) particularly, claims, example 3 | 1-20 |
| A | WO 2010/010797 A1 (KINOPHARMA, INC.) 28 January 2010 (2010-01-28) particularly, claims, etc. | 1-20 |
| A | WO 2012/001941 A1 (HAGIWARA, Masatoshi, MATSUO, Masafumi) 05 January 2012 (2012-01-05) particularly, claims, etc. | 1-20 |
| P, X | IIDA, K. et al. Switching of OAS1 splicing isoforms mitigates SARS-CoV-2 infection. bioRxiv. August 2021, DOI 10.1101/2021.08.23.457314 particularly, abstract, figures | 1-20 |

Form PCT/ISA/210 (second sheet) (January 2015)

**INTERNATIONAL SEARCH REPORT**

International application No.
**PCT/JP2022/022350**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

**INTERNATIONAL SEARCH REPORT**
**Information on patent family members**

International application No.
**PCT/JP2022/022350**

| Patent document cited in search report | Publication date (day/month/year) | Patent family member(s) | Publication date (day/month/year) |
|---|---|---|---|
| WO 2014/021337 A1 | 06 February 2014 | US 2015/0225421 A1<br>compound 8<br>EP 2881397 A1<br>CN 104520303 A | |
| WO 2018/043674 A1 | 08 March 2018 | US 2019/0247384 A1<br>claims<br>EP 3508223 A1 | |
| WO 2015/083750 A1 | 11 June 2015 | US 2016/0303089 A1<br>claims, example 3 | |
| WO 2010/010797 A1 | 28 January 2010 | KR 10-2010-0010894 A<br>claims | |
| WO 2012/001941 A1 | 05 January 2012 | US 2013/0102644 A1<br>claims<br>EP 2586462 A1<br>CN 103096928 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014021337 A
- WO 2018043674 A
- WO 2015083750 A
- WO 2010010797 A


### Non-patent literature cited in the description


- **PAIRO-CASTINEIRA et al.** *Nature,* 2021, vol. 591, 92-98
- *The COVID-19 Host Genetics Initiative,* 2021
- **BLANCO-MELO et al.** *Cell,* 2020, vol. 181, 1036-1045
- **HAMANO et al.** *Biochem Biophys Res Commun,* 2005, vol. 329, 1234-9
- **HE et al.** *BMC Infect Dis,* 2006, vol. 6, 106
- **LIN et al.** *J Immunol,* 2009, vol. 183, 8035-43
- *Prog. Med,* 1985, vol. 5, 2157-2161
- **ADAM D.** A guide to R - the pandemic's misunderstood metric. *Nature,* 2020, vol. 583, 346-348
- **BLANCO-MELO D ; NILSSON-PAYANT BE ; LIU W ; UHL S ; HOAGLAND D ; MOLLER R ; JORDAN TX ; OISHI K ; PANIS M ; SACHS D.** TenOever BR. 2020. Imbalanced Host Response to SARS-CoV-2 Drives Development of COVID-19. *Cell,* vol. 181, 1036-1045
- **CAREY CM ; GOVANDE AA ; COOPER JM ; HARTLEY MK ; KRANZUSCH PJ ; ELDE NC.** Recurrent Loss-of-Function Mutations Reveal Costs to OAS1 Antiviral Activity in Primates. *Cell Host Microbe,* 2019, vol. 25, 336-343
- **CARTEGNI L ; WANG J ; ZHU Z ; ZHANG MΓA ; KRAINER AR.** ESEfinder: A web resource to identify exonic splicing enhancers. *Nucleic Acids Res,* 2003, vol. 31, 3568-71
- **CHOI UY ; KANG J-S ; HWANG YS ; KIM Y-J.** Oligoadenylate synthase-like (OASL) proteins: dual functions and associations with diseases. *Exp Mol Med,* 2015, vol. 47, e144
- **DI H ; ELBAHESH H ; BRINTON MA.** Characteristics of Human OAS1 Isoform Proteins. *Viruses,* 2020, vol. 12
- **FORD E ; THANOS D.** The transcriptional code of human IFN-beta gene expression. *Biochim Biophys Acta,* 2010, vol. 1799, 328-36
- **HAMANO E ; HIJIKATA M ; ITOYAMA S ; QUY T ; PHI NC ; LONG HT ; HA LD ; BAN V VAN ; MATSUSHITA I ; YANAI H.** Polymorphisms of interferon-inducible genes OAS-1 and MxA associated with SARS in the Vietnamese population. *Biochem Biophys Res Commun,* 2005, vol. 329, 1234-9
- **HARKO T ; LOBO FSN ; MAK MK.** Exact analytical solutions of the Susceptible-Infected-Recovered (SIR) epidemic model and of the SIR model with equal death and birth rates. *Appl Math Comput,* 2014, vol. 236, 184-194
- **HE J ; FENG D ; DE VLAS SJ ; WANG H ; FONTANET A ; ZHANG P ; PLANCOULAINE S ; TANG F ; ZHAN L ; YANG H.** Association of SARS susceptibility with single nucleic acid polymorphisms of OAS1 and MxA genes: a case-control study. *BMC Infect Dis,* 2006, vol. 6, 106
- **HUNG IF-N ; LUNG K-C ; TSO EY K ; LIU R ; CHUNG TW-H ; CHU M-Y ; NG Y-Y ; LO J ; CHAN J ; TAM AR.** Triple combination of interferon beta-lb, lopinavir-ritonavir, and ribavirin in the treatment of patients admitted to hospital with COVID-19: an open-label, randomised, phase 2 trial. *Lancet (London, England),* 2020, vol. 395, 1695-1704
- **IBSEN MS ; GAD HH ; ANDERSEN LL ; HORNUNG V ; JULKUNEN I ; SARKAR SN ; HARTMANN R.** Structural and functional analysis reveals that human OASL binds dsRNA to enhance RIG-I signaling. *Nucleic Acids Res,* 2015, vol. 43, 5236-48
- **KJAER KH ; PAHUS J ; HANSEN MF ; POULSEN JB ; CHRISTENSEN EI ; JUSTESEN J ; MARTENSEN PM.** Mitochondrial localization of the OAS1 p46 isoform associated with a common single nucleotide polymorphism. *BMC Cell Biol,* 2014, vol. 15, 1-14
- **LIN R-J ; YU H-P ; CHANG B-L ; TANG W-C ; LIAO C-L ; LIN Y-L.** Distinct antiviral roles for human 2',5'-oligoadenylate synthetase family members against dengue virus infection. *J Immunol,* 2009, vol. 183, 8035-43
- **MARCUS JH ; NOVEMBRE J.** Visualizing the geography of genetic variants. *Bioinformatics,* 2017, vol. 33, 594-595
- **MENDEZ FL ; WATKINS JC ; HAMMER MF.** Neandertal origin of genetic variation at the cluster of OAS immunity genes. *Mol Biol Evol,* 2013, vol. 30, 798-801

- **MONK PD ; MARSDEN RJ ; TEAR VJ ; BROOKES J ; BATTEN TN ; MANKOWSKI M ; GABBAY FJ ; DAVIES DE ; HOLGATE ST ; HO L-P.** Interferon Beta COVID-19 Study Group. 2021. Safety and efficacy of inhaled nebulised interferon beta-la (SNG001) for treatment of SARS-CoV-2 infection: a randomised, double-blind, placebo-controlled, phase 2 trial. *Lancet Respir Med,* vol. 9, 196-206
- **NILE SH ; NILE A ; QIU J ; LI L ; JIA X ; KAI G.** COVID-19: Pathogenesis, cytokine storm and therapeutic potential of interferons. *Cytokine Growth Factor Rev,* 2020, vol. 53, 66-70
- **NOGUCHI S ; HAMANO E ; MATSUSHITA I ; HIJIKATA M ; ITO H ; NAGASE T ; KEICHO N.** Differential effects of a common splice site polymorphism on the generation of OAS1 variants in human bronchial epithelial cells. *Hum Immunol,* 2013, vol. 74, 395-401
- **PAIRO-CASTINEIRA E ; CLOHISEY S ; KLARIC L ; BRETHERICK AD ; RAWLIK K ; PASKO D ; WALKER S ; PARKINSON N ; FOURMAN MH ; RUSSELL CD.** GenOMICC Investigators, ISARIC4C Investigators. *COVID-19 Human Genetics Initiative, 23andMe Investigators, BRACOVID Investigators, Gen-COVID Investigators*
- **SHEN X ; PONTING CP ; FAWKES A ; TENESA A ; CAULFIELD M ; SCOTT R ; ROWAN K ; MURPHY L ; OPENSHAW PJM ; SEMPLE MG.** Genetic mechanisms of critical illness in COVID-19. *Nature,* 2021, vol. 591, 92-98
- **SADLER AJ ; WILLIAMS BRG.** Interferon-inducible antiviral effectors. *Nat Rev Immunol,* 2008, vol. 8, 559-68
- **SAKUMA M ; IIDA K ; HAGIWARA M.** Deciphering targeting rules of splicing modulator compounds: case of TG003. *BMC Mol Biol,* 2015, vol. 16, 16
- **SCHLEE M ; HARTMANN G.** Discriminating self from non-self in nucleic acid sensing. *Nat Rev Immunol,* 2016, vol. 16, 566-80
- **SCHWANKE H ; STEMPEL M ; BRINKMANN MM.** Of Keeping and Tipping the Balance: Host Regulation and Viral Modulation of IRF3-Dependent IFNB1 Expression. *Viruses,* 2020, vol. 12
- **SHALHOUB S.** Interferon beta-lb for COVID-19. *Lancet (London, England),* 2020, vol. 395, 1670-1671
- **SHIBATA S ; AJIRO M ; HAGIWARA M.** Mechanism-Based Personalized Medicine for Cystic Fibrosis by Suppressing Pseudo Exon Inclusion. *Cell Chem Biol,* 2020, vol. 27, 1472-1482.e6
- **SOETAERT K ; PETZOLDT T ; SETZER RW.** Solving Differential Equations in R : Package deSolve. *J Stat Softw,* 2010, vol. 33
- The COVID-19 Host Genetics Initiative. 2021. Mapping the human genetic architecture of COVID-19 by worldwide meta-analysis. *medRxiv,* 2021
- **ZEBERG H ; PAABO S.** A genomic region associated with protection against severe COVID-19 is inherited from Neandertals. *Proc Natl Acad Sci U S A,* 2021, vol. 118
- **ZHOU S ; BUTLER-LAPORTE G ; NAKANISHI T ; MORRISON DR ; AFILALO J ; AFILALO M ; LAURENT L ; PIETZNER M ; KERRISON N ; ZHAO K.** A Neanderthal OAS1 isoform protects individuals of European ancestry against COVID-19 susceptibility and severity. *Nat Med.,* 2021